# EUROPEAN PATENT SPECIFICATION

(11) **EP 2 245 151 B1**
(45) Date of publication and mention of the grant of the patent: **24.12.2014**
(21) Application number: 09711666.9
(22) Date of filing: 09.02.2009
(51) Int. Cl.: C12N 9/90, C12N 15/81

(54) **VECTORS AND YEAST STRAINS FOR PROTEIN PRODUCTION**
VEKTOREN UND HEFESTÄMME FÜR DIE PROTEINPRODUKTION
VECTEURS ET SOUCHES DE LEVURE POUR LA PRODUCTION DE PROTÉINES

(30) Priority: 20.02.2008 US 66409; 12.08.2008 US 188723
(43) Date of publication of application: 03.11.2010
(73) Proprietor: GlycoFi, Inc., Lebanon, NH 03766 (US)
(72) Inventor: CHOI, Byung-kwon, Lebanon, NH 03766 (US); BOBROWICZ, Piotr, Lebanon, NH 03766 (US); COOK, W. James, Lebanon, NH 03766 (US)
(74) Representative: Buchan, Gavin MacNicol
(86) International application number: PCT/US2009/033507
(87) International publication number: WO 2009/105357

(56) References cited:
- KIMURA T ET AL: "Functional differences between human and yeast protein disulfide isomerase family proteins" BIOCHEMICAL AND BIOPHYSICAL RESEARCH COMMUNICATIONS, ACADEMIC PRESS INC. ORLANDO, FL, US, vol. 320, no. 2, 23 July 2004 (2004-07-23), pages 359-365, XP004516637 ISSN: 0006-291X
- XIAO R ET AL: "Combinations of protein-disulfide isomerase domains show that there is little correlation between isomerase activity and wild-type growth." THE JOURNAL OF BIOLOGICAL CHEMISTRY 27 JUL 2001, vol. 276, no. 30, 27 July 2001 (2001-07-27), pages 27975-27980, XP002528283 ISSN: 0021-9258
- LABOISSIERE M C ET AL: "The essential function of protein-disulfide isomerase is to unscramble non-native disulfide bonds." THE JOURNAL OF BIOLOGICAL CHEMISTRY 24 NOV 1995, vol. 270, no. 47, 24 November 1995 (1995-11-24), pages 28006-28009, XP002528284 ISSN: 0021-9258
- GUENTHER R ET AL: "Functional replacement of the Saccharomyces cerevisiae Trg1/Pdi1 protein by members of the mammalian protein disulfide isomerase family" JOURNAL OF BIOLOGICAL CHEMISTRY, AMERICAN SOCIETY OF BIOLOCHEMICAL BIOLOGISTS, BIRMINGHAM, US, vol. 268, no. 11, 15 April 1993 (1993-04-15), pages 7728-7732, XP002223364 ISSN: 0021-9258 cited in the application
- SMITH JASON D ET AL: "Protein disulfide isomerase, but not binding protein, overexpression enhances secretion of a non-disulfide-bonded protein in yeast." BIOTECHNOLOGY AND BIOENGINEERING 5 FEB 2004, vol. 85, no. 3, 5 February 2004 (2004-02-05), pages 340-350, XP002528285 ISSN: 0006-3592
- ROBINSON A S ET AL: "PROTEIN DISULFIDE ISOMERASE OVEREXPRESSION INCREASES SECRETION OF FOREIGN PROTEINS IN SACCHAROMYCES CEREVISIAE" BIO/TECHNOLOGY, NATURE PUBLISHING CO. NEW YORK, US, vol. 12, 1 April 1994 (1994-04-01), pages 381-384, XP002070977 ISSN: 0733-222X
- MARTIN SCHRÃDER: "Engineering eukaryotic protein factories" BIOTECHNOLOGY LETTERS, KLUWER ACADEMIC PUBLISHERS, DO, vol. 30, no. 2, 21 September 2007 (2007-09-21), pages 187-196, XP019569973 ISSN: 1573-6776

### SEQUENCE LISTING

<110> CHOI, Byung-Kwon BOBROWICZ, Piotr COOK, W. James
<120> VECTORS AND YEAST STRAINS FOR PROTEIN
   PRODUCTION
<130> MRL-BIO-22395
<150> 61/0066,409
   <151> 2008-02-20
<150> 61/188,723 <151> 2008-08-12
<160> 77
<170> FastSEQ for Windows Version 4.0
<210> 1
   <211> 30
   <212> DNA
   <213> Artificial Sequence
<220>
   <223> PCR primer hPDI/UP1
<400> 1
   agcgctgacg cccccgagga ggaggaccac 30
<210> 2
   <211> 42
   <212> DNA
   <213> Artificial Sequence
<220>
   <223> PCR primer hPDI/LP-PacI
<400> 2
   ccttaattaa ttacagttca tcatgcacag ctttctgatc at 42
<210> 3
   <211> 47
   <212> DNA
   <213> Artificial Sequence
<220>
   <223> PCR primer PB248
<400> 3
   atgaattcag gccatatcgg ccattgttta ctgtgcgccc acagtag 47
<210> 4
   <211> 35
   <212> DNA
   <213> Artificial Sequence
<220>
   <223> PCR primer PB249
<400> 4
   atgtttaaac gtgaggatta ctggtgatga aagac 35
<210> 5
   <211> 34
   <212> DNA
   <213> Artificial Sequence
<220>
   <223> PCR primer PB250
<400> 5
   agactagtct atttggagac attgacggat ccac 34
<210> 6
   <211> 46
   <212> DNA
   <213> Artificial Sequence
<220>
   <223> PCR primer PB251
<400> 6
   atctcgagag gccatgcagg ccaaccacaa gatgaatcaa attttg 46
<210> 7
   <211> 34
   <212> DNA
   <213> Artificial Sequence
<220>
   <223> PCR primer PpPDI/UPi-1
<400> 7
   ggtgaggttg aggtcccaag tgactatcaa ggtc 34
<210> 8
   <211> 34
   <212> DNA
   <213> Artificial Sequence
<220>
   <223> PCR primer PpPDI/LPi-1
<400> 8
   gaccttgata gtcacttggg acctcaacct cacc 34
<210> 9
   <211> 34
   <212> DNA
   <213> Artificial Sequence
<220>
   <223> PCR primer PpPDI/UPi-2
<400> 9
   cgccaatgat gaggatgcct cttcaaaggt tgtg 34
<210> 10
   <211> 34
   <212> DNA
   <213> Artificial Sequence
<220>
   <223> PCR primer PpPDI/LPi-2
<400> 10
   cacaaccttt gaagaggcat cctcatcatt ggcg 34
<210> 11
   <211> 24
   <212> DNA
   <213> Artificial Sequence
<220>
   <223> PCR primer PpPDI-5'/UP
<400> 11
   ggcgattgca ttcgcgactg tatc 24
<210> 12
   <211> 23
   <212> DNA
   <213> Artificial Sequence
<220>
   <223> PCR primer hPDI-3'/LP
<400> 12
   cctagagagc ggtggccaag atg 23
<210> 13
   <211> 25
   <212> DNA
   <213> Artificial Sequence
<220>
   <223> PCR primer hPDI/UP
<400> 13
   gtggccacac cagggggcat ggaac 25
<210> 14
   <211> 23
   <212> DNA
   <213> Artificial Sequence
<220>
   <223> PCR primer hPDI-3'/LP
<400> 14
   cctagagagc ggtggccaag atg 23
<210> 15
   <211> 37
   <212> DNA
   <213> Artificial Sequence
<220>
   <223> PCR primer hGRP94/UP1
<400> 15
   agcgctgacg atgaagttga tgtggatggt acagtag 37
<210> 16
   <211> 38
   <212> DNA
   <213> Artificial Sequence
<220>
   <223> PCR primer hGRP94/LP1
<400> 16
   ggccggcctt acaattcatc atgttcagct gtagattc 38
<210> 17
   <211> 24
   <212> DNA
   <213> Artificial Sequence
<220>
   <223> PCR primer PMT1-KO1
<400> 17
   tgaacccatc tgtaaataga atgc 24
<210> 18
   <211> 45
   <212> DNA
   <213> Artificial Sequence
<220>
   <223> PCR primer PMT1-KO2
<400> 18
   gtgtcaccta aatcgtatgt gcccatttac tggaagctgc taacc 45
<210> 19
   <211> 45
   <212> DNA
   <213> Artificial Sequence
<220>
   <223> PCR primer PMT1-KO3
<400> 19
   ctccctatag tgagtcgtat tcatcattgt actttggtat attgg 45
<210> 20
   <211> 24
   <212> DNA
   <213> Artificial Sequence
<220>
   <223> PCR primer PMT1-KO4
<400> 20
   tatttgtacc tgcgtcctgt ttgc 24
<210> 21
   <211> 21
   <212> DNA
   <213> Artificial Sequence
<220>
   <223> PCR primer PR29
<400> 21
   cacatacgat ttaggtgaca c 21
<210> 22
   <211> 21
   <212> DNA
   <213> Artificial Sequence
<220>
   <223> PCR primer PR32
<400> 22
   aatacgactc actataggga g 21
<210> 23
   <211> 24
   <212> DNA
   <213> Artificial Sequence
<220>
   <223> PCR primer PMT4-KO1
<400> 23
   tgctctccgc gtgcaataga aact 24
<210> 24
   <211> 45
   <212> DNA
   <213> Artificial Sequence
<220>
   <223> PCR primer PMT4-KO2
<400> 24
   ctccctatag tgagtcgtat tcacagtgta ccatctttca tctcc 45
<210> 25
   <211> 45
   <212> DNA
   <213> Artificial Sequence
<220>
   <223> PCR primer PMT4-KO3
<400> 25
   gtgtcaccta aatcgtatgt gaacctaact ctaattcttc aaagc 45
<210> 26
   <211> 24
   <212> DNA
   <213> Artificial Sequence
<220>
   <223> PCR primer PMT4-KO4
<400> 26
   actagggtat ataattccca aggt 24
<210> 27
   <211> 57
   <212> DNA
   <213> Artificial Sequence
<220>
   <223> Saccharomyces cerevisiae mating factor pre-signal peptide
<400> 27
   atgagattcc catccatctt cactgctgtt ttgttcgctg cttcttctgc tttggct 57
<210> 28
   <211> 19
   <212> PRT
   <213> Artificial Sequence
<220>
   <223> Saccharomyces cerevisiae mating factor pre-signal peptide
<400> 28
   Met Arg Phe Pro Ser Ile Phe Thr Ala Val Leu Phe Ala Ala Ser Ser
    1               5                  10                  15
   Ala Leu Ala
<210> 29
   <211> 1353
   <212> DNA
   <213> Artificial Sequence
<220>
   <223> Anti-Her2 Heavy chain (VH + IgG1 constant region)
<400> 29
   gaggttcagt tggttgaatc tggaggagga ttggttcaac ctggtggttc tttgagattg 60
   tcctgtgctg cttccggttt caacatcaag gacacttaca tccactgggt tagacaagct 120
   ccaggaaagg gattggagtg ggttgctaga atctacccaa ctaacggtta cacaagatac 180
   gctgactccg ttaagggaag attcactatc tctgctgaca cttccaagaa cactgcttac 240
   ttgcagatga actccttgag agctgaggat actgctgttt actactgttc cagatggggt 300
   ggtgatggtt tctacgctat ggactactgg ggtcaaggaa ctttggttac tgtttcctcc 360
   gcttctacta agggaccatc tgttttccca ttggctccat cttctaagtc tacttccggt 420
   ggtactgctg ctttgggatg tttggttaaa gactacttcc cagagccagt tactgtttct 480
   tggaactccg gtgctttgac ttctggtgtt cacactttcc cagctgtttt gcaatcttcc 540
   ggtttgtact ctttgtcctc cgttgttact gttccatcct cttccttggg tactcagact 600
   tacatctgta acgttaacca caagccatcc aacactaagg ttgacaagaa ggttgagcca 660
   aagtcctgtg acaagactca tacttgtcca ccatgtccag ctccagaatt gttgggtggt 720
   ccttccgttt ttttgttccc accaaagcca aaggacactt tgatgatctc cagaactcca 780
   gaggttacat gtgttgttgt tgacgtttct cacgaggacc cagaggttaa gttcaactgg 840
   tacgttgacg gtgttgaagt tcacaacgct aagactaagc caagagagga gcagtacaac 900
   tccacttaca gagttgtttc cgttttgact gttttgcacc aggattggtt gaacggaaag 960
   gagtacaagt gtaaggtttc caacaaggct ttgccagctc caatcgaaaa gactatctcc 1020
   aaggctaagg gtcaaccaag agagccacag gtttacactt tgccaccatc cagagatgag 1080
   ttgactaaga accaggtttc cttgacttgt ttggttaagg gattctaccc atccgacatt 1140
   gctgttgaat gggagtctaa cggtcaacca gagaacaact acaagactac tccacctgtt 1200
   ttggactctg acggttcctt tttcttgtac tccaagttga ctgttgacaa gtccagatgg 1260
   caacagggta acgttttctc ctgttccgtt atgcatgagg ctttgcacaa ccactacact 1320
   caaaagtcct tgtctttgtc ccctggtaag taa                              1353
<210> 30
   <211> 450
   <212> PRT
   <213> Artificial Sequence
<220>
   <223> Anti-Her2 Heavy chain (VH + IgG1 constant region)
<400> 30
   Glu Val Gln Leu Val Glu Ser Gly Gly Gly Leu Val Gln Pro Gly Gly
    1               5                  10                  15
   Ser Leu Arg Leu Ser Cys Ala Ala Ser Gly Phe Asn Ile Lys Asp Thr
               20                  25                  30
   Tyr Ile His Trp Val Arg Gln Ala Pro Gly Lys Gly Leu Glu Trp Val
           35                  40                  45
   Ala Arg Ile Tyr Pro Thr Asn Gly Tyr Thr Arg Tyr Ala Asp Ser Val
       50                  55                  60
   Lys Gly Arg Phe Thr Ile Ser Ala Asp Thr Ser Lys Asn Thr Ala Tyr
   65                  70                  75                  80
   Leu Gln Met Asn Ser Leu Arg Ala Glu Asp Thr Ala Val Tyr Tyr Cys
                   85                  90                  95
   Ser Arg Trp Gly Gly Asp Gly Phe Tyr Ala Met Asp Tyr Trp Gly Gln
               100                 105                 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Lys Val Glu Pro Lys Ser Cys Asp
       210                 215                 220
   Lys Thr His Thr Cys Pro Pro Cys Pro Ala Pro Glu Leu Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
               260                 265                 270
   Asp Pro Glu Val Lys Phe Asn Trp Tyr Val Asp Gly Val Glu Val His
           275                 280                 285
   Asn Ala Lys Thr Lys Pro Arg Glu Glu Gln Tyr Asn Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Ala Leu Pro Ala Pro Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Ser Arg Asp Glu Leu Thr Lys Asn Gln Val Ser Leu
           355                 360                 365
   Thr Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Ser Asp Gly Ser Phe Phe Leu Tyr Ser Lys Leu Thr Val Asp
                   405                 410                 415
   Lys Ser Arg Trp Gln Gln Gly Asn Val Phe Ser Cys Ser Val Met His
               420                 425                 430
   Glu Ala Leu His Asn His Tyr Thr Gln Lys Ser Leu Ser Leu Ser Pro
           435                 440                 445
   Gly Lys
       450
<210> 31
   <211> 645
   <212> DNA
   <213> Artificial Sequence
<220>
   <223> Anti-Her2 light chain (VL + Kappa constant region)
<400> 31
   gacatccaaa tgactcaatc cccatcttct ttgtctgctt ccgttggtga cagagttact 60
   atcacttgta gagcttccca ggacgttaat actgctgttg cttggtatca acagaagcca 120
   ggaaaggctc caaagttgtt gatctactcc gcttccttct tgtactctgg tgttccatcc 180
   agattctctg gttccagatc cggtactgac ttcactttga ctatctcctc cttgcaacca 240
   gaagatttcg ctacttacta ctgtcagcag cactacacta ctccaccaac tttcggacag 300
   ggtactaagg ttgagatcaa gagaactgtt gctgctccat ccgttttcat tttcccacca 360
   tccgacgaac agttgaagtc tggtacagct tccgttgttt gtttgttgaa caacttctac 420
   ccaagagagg ctaaggttca gtggaaggtt gacaacgctt tgcaatccgg taactcccaa 480
   gaatccgtta ctgagcaaga ctctaaggac tccacttact ccttgtcctc cactttgact 540
   ttgtccaagg ctgattacga gaagcacaag gtttacgctt gtgaggttac acatcagggt 600
   ttgtcctccc cagttactaa gtccttcaac agaggagagt gttaa                645
<210> 32
   <211> 213
   <212> PRT
   <213> Artificial Sequence
<220>
   <223> Anti-Her2 light chain (VL + Kappa constant region)
<400> 32
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Asp Val Asn Thr Ala
               20                  25                  30
   Val Ala Trp Tyr Gln Gln Lys Pro Gly Lys Ala Pro Lys Leu Leu Ile
           35                  40                  45
   Tyr Ser Ala Ser Phe Leu Tyr Ser Gly Val Pro Ser Arg Phe Ser Gly
       50                  55                  60
   Ser Arg Ser Gly Thr Asp Phe Thr Leu Thr Ile Ser Ser Leu Gln Pro
   65                  70                  75                  80
   Glu Asp Phe Ala Thr Tyr Tyr Cys Gln Gln His Tyr Thr Thr Pro Pro
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Asn Asn Phe Tyr Pro Arg Glu Ala Lys
       130                 135                 140
   Val Gln Trp Lys Val Asp Asn Ala Leu Gln Ser Gly Asn Ser Gln Glu
   145                 150                 155                 160
   Ser Val Thr Glu Gln Asp Ser Lys Asp Ser Thr Tyr Ser Leu Ser Ser
                   165                 170                 175
   Thr Leu Thr Leu Ser Lys Ala Asp Tyr Glu Lys His Lys Val Tyr Ala
               180                 185                 190
   Cys Glu Val Thr His Gln Gly Leu Ser Ser Pro Val Thr Lys Ser Phe
           195                 200                 205
   Asn Arg Gly Glu Cys
       210
<210> 33
   <211> 60
   <212> DNA
   <213> Artificial Sequence
<220>
   <223> Alpha amylase signal peptide (from Aspergillus niger -amylase)
<400> 33
   atggttgctt ggtggtcctt gttcttgtac ggattgcaag ttgctgctcc agctttggct 60
<210> 34
   <211> 20
   <212> PRT
   <213> Artificial Sequence
<220>
   <223> Alpha amylase signal peptide (from Aspergillus niger -amylase)
<400> 34
   Met Val Ala Trp Trp Ser Leu Phe Leu Tyr Gly Leu Gln Val Ala Ala
    1               5                  10                  15
   Pro Ala Leu Ala
               20
<210> 35
   <211> 397
   <212> DNA
   <213> Artificial Sequence
<220>
   <223> Anti-CD20 Light chain Variable Region
<400> 35
   gagatcgttt tgacacagtc cccagctact ttgtctttgt ccccaggtga aagagctaca 60
   ttgtcctgta gagcttccca atctgtttcc tcctacttgg cttggtatca acaaaagcca 120
   ggacaggctc caagattgtt gatctacgac gcttccaata gagctactgg tatcccagct 180
   agattctctg gttctggttc cggtactgac ttcactttga ctatctcttc cttggaacca 240
   gaggacttcg ctgtttacta ctgtcagcag agatccaatt ggccattgac tttcggtggt 300
   ggtactaagg ttgagatcaa gcgtacggtt gctgctcctt ccgttttcat tttcccacca 360
   tccgacgaac aattgaagtc tggtacccaa ttcgccc                          397
<210> 36
   <211> 132
   <212> PRT
   <213> Artificial Sequence
<220>
   <223> Anti-CD20 Light chain Variable Region
<400> 36
   Glu Ile Val Leu Thr Gln Ser Pro Ala Thr Leu Ser Leu Ser Pro Gly
    1               5                  10                  15
   Glu Arg Ala Thr Leu Ser Cys Arg Ala Ser Gln Ser Val Ser Ser Tyr
               20                  25                  30
   Leu Ala Trp Tyr Gln Gln Lys Pro Gly Gln Ala Pro Arg Leu Leu Ile
           35                  40                  45
   Tyr Asp Ala Ser Asn Arg Ala Thr Gly Ile Pro Ala Arg Phe Ser Gly
       50                  55                  60
   Ser Gly Ser Gly Thr Asp Phe Thr Leu Thr Ile Ser Ser Leu Glu Pro
   65                  70                  75                  80
   Glu Asp Phe Ala Val Tyr Tyr Cys Gln Gln Arg Ser Asn Trp Pro Leu
                   85                  90                  95
   Thr Phe Gly Gly Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Gln Phe Ala
       130
<210> 37
   <211> 445
   <212> DNA
   <213> Artificial Sequence
<220>
   <223> Anti-CD20 Heavy chain Variable Region
<400> 37
   gctgttcagc tggttgaatc tggtggtgga ttggttcaac ctggtagatc cttgagattg 60
   tcctgtgctg cttccggttt tactttcggt gactacacta tgcactgggt tagacaagct 120
   ccaggaaagg gattggaatg ggtttccggt atttcttgga actccggttc cattggttac 180
   gctgattccg ttaagggaag attcactatc tccagagaca acgctaagaa ctccttgtac 240
   ttgcagatga actccttgag agctgaggat actgctttgt actactgtac taaggacaac 300
   caatacggtt ctggttccac ttacggattg ggagtttggg gacagggaac tttggttact 360
   gtctcgagtg cttctactaa gggaccatcc gtttttccat tggctccatc ctctaagtct 420
   acttccggtg gtacccaatt cgccc                                       445
<210> 38
   <211> 148
   <212> PRT
   <213> Artificial Sequence
<220>
   <223> Anti-CD20 Heavy chain Variable Region
<400> 38
   Ala Val Gln Leu Val Glu Ser Gly Gly Gly Leu Val Gln Pro Gly Arg
    1               5                  10                  15
   Ser Leu Arg Leu Ser Cys Ala Ala Ser Gly Phe Thr Phe Gly Asp Tyr
               20                  25                  30
   Thr Met His Trp Val Arg Gln Ala Pro Gly Lys Gly Leu Glu Trp Val
           35                  40                  45
   Ser Gly Ile Ser Trp Asn Ser Gly Ser Ile Gly Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Leu Tyr Tyr Cys
                   85                  90                  95
   Thr Lys Asp Asn Gln Tyr Gly Ser Gly Ser Thr Tyr Gly Leu Gly Val
               100                 105                 110
   Trp Gly Gln Gly Thr Leu Val Thr Val Ser Ser Ala Ser Thr Lys Gly
           115                 120                 125
   Pro Ser Val Phe Pro Leu Ala Pro Ser Ser Lys Ser Thr Ser Gly Gly
       130                 135                 140
   Thr Gln Phe Ala
   145
<210> 39
   <211> 1476
   <212> DNA
   <213> Artificial Sequence
<220>
   <223> human PDI without leader
<400> 39
   gacgcccccg aggaggagga ccacgtcttg gtgctgcgga aaagcaactt cgcggaggcg 60
   ctggcggccc acaagtaccc gccggtggag ttccatgccc cctggtgtgg ccactgcaag 120
   gctctggccc ctgagtatgc caaagccgct gggaagctga aggcagaagg ttccgagatc 180
   aggttggcca aggtggacgc cacggaggag tctgacctag cccagcagta cggcgtgcgc 240
   ggctatccca ccatcaagtt cttcaggaat ggagacacgg cttcccccaa ggaatataca 300
   gctggcagag aggctgatga catcgtgaac tggctgaaga agcgcacggg cccggctgcc 360
   accaccctgc ctgacggcgc agctgcagag tccttggtgg agtccagcga ggtggccgtc 420
   atcggcttct tcaaggacgt ggagtcggac tctgccaagc agtttttgca ggcagcagag 480
   gccatcgatg acataccatt tgggatcact tccaacagtg acgtgttctc caaataccag 540
   ctcgacaaag atggggttgt cctctttaag aagtttgatg aaggccggaa caactttgaa 600
   ggggaggtca ccaaggagaa cctgctggac tttatcaaac acaaccagct gccccttgtc 660
   atcgagttca ccgagcagac agccccgaag atttttggag gtgaaatcaa gactcacatc 720
   ctgctgttct tgcccaagag tgtgtctgac tatgacggca aactgagcaa cttcaaaaca 780
   gcagccgaga gcttcaaggg caagatcctg ttcatcttca tcgacagcga ccacaccgac 840
   aaccagcgca tcctcgagtt ctttggcctg aagaaggaag agtgcccggc cgtgcgcctc 900
   atcaccttgg aggaggagat gaccaagtac aagcccgaat cggaggagct gacggcagag 960
   aggatcacag agttctgcca ccgcttcctg gagggcaaaa tcaagcccca cctgatgagc 1020
   caggagctgc cggaggactg ggacaagcag cctgtcaagg tgcttgttgg gaagaacttt 1080
   gaagacgtgg cttttgatga gaaaaaaaac gtctttgtgg agttctatgc cccatggtgt 1140
   ggtcactgca aacagttggc tcccatttgg gataaactgg gagagacgta caaggaccat 1200
   gagaacatcg tcatcgccaa gatggactcg actgccaacg aggtggaggc cgtcaaagtg 1260
   cacggcttcc ccacactcgg gttctttcct gccagtgccg acaggacggt cattgattac 1320
   aacggggaac gcacgctgga tggttttaag aaattcctag agagcggtgg ccaagatggg 1380
   gcaggggatg ttgacgacct cgaggacctc gaagaagcag aggagccaga catggaggaa 1440
   gacgatgacc agaaagctgt gaaagatgaa ctgtaa                           1476
<210> 40
   <211> 491
   <212> PRT
   <213> Artificial Sequence
<220>
   <223> human PDI without leader
<400> 40
   Asp Ala Pro Glu Glu Glu Asp His Val Leu Val Leu Arg Lys Ser Asn
    1               5                  10                  15
   Phe Ala Glu Ala Leu Ala Ala His Lys Tyr Pro Pro Val Glu Phe His
               20                  25                  30
   Ala Pro Trp Cys Gly His Cys Lys Ala Leu Ala Pro Glu Tyr Ala Lys
               35                  40                  45
   Ala Ala Gly Lys Leu Lys Ala Glu Gly Ser Glu Ile Arg Leu Ala Lys
           50                  55                  60
   Val Asp Ala Thr Glu Glu Ser Asp Leu Ala Gln Gln Tyr Gly Val Arg
   65                  70                  75                  80
   Gly Tyr Pro Thr Ile Lys Phe Phe Arg Asn Gly Asp Thr Ala Ser Pro
                   85                  90                  95
   Lys Glu Tyr Thr Ala Gly Arg Glu Ala Asp Asp Ile Val Asn Trp Leu
               100                 105                 110
   Lys Lys Arg Thr Gly Pro Ala Ala Thr Thr Leu Pro Asp Gly Ala Ala
           115                 120                 125
   Ala Glu Ser Leu Val Glu Ser Ser Glu Val Ala Val Ile Gly Phe Phe
       130                 135                 140
   Lys Asp Val Glu Ser Asp Ser Ala Lys Gln Phe Leu Gln Ala Ala Glu
   145                 150                 155                 160
   Ala Ile Asp Asp Ile Pro Phe Gly Ile Thr Ser Asn Ser Asp Val Phe
                   165                 170                 175
   Ser Lys Tyr Gln Leu Asp Lys Asp Gly Val Val Leu Phe Lys Lys Phe
               180                 185                 190
   Asp Glu Gly Arg Asn Asn Phe Glu Gly Glu Val Thr Lys Glu Asn Leu
           195                 200                 205
   Leu Asp Phe Ile Lys His Asn Gln Leu Pro Leu Val Ile Glu Phe Thr
       210                 215                 220
   Glu Gln Thr Ala Pro Lys Ile Phe Gly Gly Glu Ile Lys Thr His Ile
   225                 230                 235                 240
   Leu Leu Phe Leu Pro Lys Ser Val Ser Asp Tyr Asp Gly Lys Leu Ser
                   245                 250                 255
   Asn Phe Lys Thr Ala Ala Glu Ser Phe Lys Gly Lys Ile Leu Phe Ile
               260                 265                 270
   Phe Ile Asp Ser Asp His Thr Asp Asn Gln Arg Ile Leu Glu Phe Phe
           275                 280                 285
   Gly Leu Lys Lys Glu Glu Cys Pro Ala Val Arg Leu Ile Thr Leu Glu
       290                 295                 300
   Glu Glu Met Thr Lys Tyr Lys Pro Glu Ser Glu Glu Leu Thr Ala Glu
   305                 310                 315                 320
   Arg Ile Thr Glu Phe Cys His Arg Phe Leu Glu Gly Lys Ile Lys Pro
                   325                 330                 335
   His Leu Met Ser Gln Glu Leu Pro Glu Asp Trp Asp Lys Gln Pro Val
               340                 345                 350
   Lys Val Leu Val Gly Lys Asn Phe Glu Asp Val Ala Phe Asp Glu Lys
           355                 360                 365
   Lys Asn Val Phe Val Glu Phe Tyr Ala Pro Trp Cys Gly His Cys Lys
       370                 375                 380
   Gln Leu Ala Pro Ile Trp Asp Lys Leu Gly Glu Thr Tyr Lys Asp His
   385                 390                 395                 400
   Glu Asn Ile Val Ile Ala Lys Met Asp Ser Thr Ala Asn Glu Val Glu
                   405                 410                 415
   Ala Val Lys Val His Gly Phe Pro Thr Leu Gly Phe Phe Pro Ala Ser
               420                 425                 430
   Ala Asp Arg Thr Val Ile Asp Tyr Asn Gly Glu Arg Thr Leu Asp Gly
           435                 440                 445
   Phe Lys Lys Phe Leu Glu Ser Gly Gly Gln Asp Gly Ala Gly Asp Val
       450                 455                 460
   Asp Asp Leu Glu Asp Leu Glu Glu Ala Glu Glu Pro Asp Met Glu Glu
   465                 470                 475                 480
   Asp Asp Asp Gln Lys Ala Val His Asp Glu Leu
                   485                 490
<210> 41
   <211> 1554
   <212> DNA
   <213> Pichia pastoris
<220>
   <223> Pichia pastoris PDI1 Gene
<400> 41
   atgcaattca actggaatat taaaactgtg gcaagtattt tgtccgctct cacactagca 60
   caagcaagtg atcaggaggc tattgctcca gaggactctc atgtcgtcaa attgactgaa 120
   gccacttttg agtctttcat caccagtaat cctcacgttt tggcagagtt ttttgcccct 180
   tggtgtggtc actgtaagaa gttgggccct gaacttgttt ctgctgccga gatcttaaag 240
   gacaatgagc aggttaagat tgctcaaatt gattgtacgg aggagaagga attatgtcaa 300
   ggctacgaaa ttaaagggta tcctactttg aaggtgttcc atggtgaggt tgaggtccca 360
   agtgactatc aaggtcaaag acagagccaa agcattgtca gctatatgct aaagcagagt 420
   ttaccccctg tcagtgaaat caatgcaacc aaagatttag acgacacaat cgccgaggca 480
   aaagagcccg tgattgtgca agtactaccg gaagatgcat ccaacttgga atctaacacc 540
   acattttacg gagttgccgg tactctcaga gagaaattca cttttgtctc cactaagtct 600
   actgattatg ccaaaaaata cactagcgac tcgactcctg cctatttgct tgtcagacct 660
   ggcgaggaac ctagtgttta ctctggtgag gagttagatg agactcattt ggtgcactgg 720
   attgatattg agtccaaacc tctatttgga gacattgacg gatccacctt caaatcatat 780
   gctgaagcta acatcccttt agcctactat ttctatgaga acgaagaaca acgtgctgct 840
   gctgccgata ttattaaacc ttttgctaaa gagcaacgtg gcaaaattaa ctttgttggc 900
   ttagatgccg ttaaattcgg taagcatgcc aagaacttaa acatggatga agagaaactc 960
   cctctatttg tcattcatga tttggtgagc aacaagaagt ttggagttcc tcaagaccaa 1020
   gaattgacga acaaagatgt gaccgagctg attgagaaat tcatcgcagg agaggcagaa 1080
   ccaattgtga aatcagagcc aattccagaa attcaagaag agaaagtctt caagctagtc 1140
   ggaaaggccc acgatgaagt tgtcttcgat gaatctaaag atgttctagt caagtactac 1200
   gccccttggt gtggtcactg taagagaatg gctcctgctt atgaggaatt ggctactctt 1260
   tacgccaatg atgaggatgc ctcttcaaag gttgtgattg caaaacttga tcacactttg 1320
   aacgatgtcg acaacgttga tattcaaggt tatcctactt tgatccttta tccagctggt 1380
   gataaatcca atcctcaact gtatgatgga tctcgtgacc tagaatcatt ggctgagttt 1440
   gtaaaggaga gaggaaccca caaagtggat gccctagcac tcagaccagt cgaggaagaa 1500
   aaggaagctg aagaagaagc tgaaagtgag gcagacgctc acgacgagct ttaa       1554
<210> 42
   <211> 1554
   <212> PRT
   <213> Pichia pastoris
<400> 42
   Ala Thr Gly Cys Ala Ala Thr Thr Cys Ala Ala Cys Thr Gly Gly Ala
    1               5                  10                  15
   Ala Thr Ala Thr Thr Ala Ala Ala Ala Cys Thr Gly Thr Gly Gly Cys
               20                  25                  30
   Ala Ala Gly Thr Ala Thr Thr Thr Thr Gly Thr Cys Cys Gly Cys Thr
           35                  40                  45
   Cys Thr Cys Ala Cys Ala Cys Thr Ala Gly Cys Ala Cys Ala Ala Gly
       50                  55                  60
   Cys Ala Ala Gly Thr Gly Ala Thr Cys Ala Gly Gly Ala Gly Gly Cys
   65                  70                  75                  80
   Thr Ala Thr Thr Gly Cys Thr Cys Cys Ala Gly Ala Gly Gly Ala Cys
                   85                  90                  95
   Thr Cys Thr Cys Ala Thr Gly Thr Cys Gly Thr Cys Ala Ala Ala Thr
               100                 105                 110
   Thr Gly Ala Cys Thr Gly Ala Ala Gly Cys Cys Ala Cys Thr Thr Thr
           115                 120                 125
   Thr Gly Ala Gly Thr Cys Thr Thr Thr Cys Ala Thr Cys Ala Cys Cys
       130                 135                 140
   Ala Gly Thr Ala Ala Thr Cys Cys Thr Cys Ala Cys Gly Thr Thr Thr
   145                 150                 155                 160
   Thr Gly Gly Cys Ala Gly Ala Gly Thr Thr Thr Thr Thr Thr Gly Cys
   165 170 175
   Cys Cys Cys Thr Thr Gly Gly Thr Gly Thr Gly Gly Thr Cys Ala Cys
   180 185 190
   Thr Gly Thr Ala Ala Gly Ala Ala Gly Thr Thr Gly Gly Gly Cys Cys
   195 200 205
   Cys Thr Gly Ala Ala Cys Thr Thr Gly Thr Thr Thr Cys Thr Gly Cys
   210 215 220
   Thr Gly Cys Cys Gly Ala Gly Ala Thr Cys Thr Thr Ala Ala Ala Gly
   225 230 235 240
   Gly Ala Cys Ala Ala Thr Gly Ala Gly Cys Ala Gly Gly Thr Thr Ala
   245 250 255
   Ala Gly Ala Thr Thr Gly Cys Thr Cys Ala Ala Ala Thr Thr Gly Ala
   260 265 270
   Thr Thr Gly Thr Ala Cys Gly Gly Ala Gly Gly Ala Gly Ala Ala Gly
   275 280 285
   Gly Ala Ala Thr Thr Ala Thr Gly Thr Cys Ala Ala Gly Gly Cys Thr
   290 295 300
   Ala Cys Gly Ala Ala Ala Thr Thr Ala Ala Ala Gly Gly Gly Thr Ala
   305 310 315 320
   Thr Cys Cys Thr Ala Cys Thr Thr Thr Gly Ala Ala Gly Gly Thr Gly
   325 330 335
   Thr Thr Cys Cys Ala Thr Gly Gly Thr Gly Ala Gly Gly Thr Thr Gly
   340 345 350
   Ala Gly Gly Thr Cys Cys Cys Ala Ala Gly Thr Gly Ala Cys Thr Ala
   355 360 365
   Thr Cys Ala Ala Gly Gly Thr Cys Ala Ala Ala Gly Ala Cys Ala Gly
   370 375 380
   Ala Gly Cys Cys Ala Ala Ala Gly Cys Ala Thr Thr Gly Thr Cys Ala
   385 390 395 400
   Gly Cys Thr Ala Thr Ala Thr Gly Cys Thr Ala Ala Ala Gly Cys Ala
   405 410 415
   Gly Ala Gly Thr Thr Thr Ala Cys Cys Cys Cys Cys Thr Gly Thr Cys
   420 425 430
   Ala Gly Thr Gly Ala Ala Ala Thr Cys Ala Ala Thr Gly Cys Ala Ala
   435 440 445
   Cys Cys Ala Ala Ala Gly Ala Thr Thr Thr Ala Gly Ala Cys Gly Ala
   450 455 460
   Cys Ala Cys Ala Ala Thr Cys Gly Cys Cys Gly Ala Gly Gly Cys Ala
   465 470 475 480
   Ala Ala Ala Gly Ala Gly Cys Cys Cys Gly Thr Gly Ala Thr Thr Gly
   485 490 495
   Thr Gly Cys Ala Ala Gly Thr Ala Cys Thr Ala Cys Cys Gly Gly Ala
   500 505 510
   Ala Gly Ala Thr Gly Cys Ala Thr Cys Cys Ala Ala Cys Thr Thr Gly
   515 520 525
   Gly Ala Ala Thr Cys Thr Ala Ala Cys Ala Cys Cys Ala Cys Ala Thr
   530 535 540
   Thr Thr Thr Ala Cys Gly Gly Ala Gly Thr Thr Gly Cys Cys Gly Gly
   545 550 555 560
   Thr Ala Cys Thr Cys Thr Cys Ala Gly Ala Gly Ala Gly Ala Ala Ala
   565 570 575
   Thr Thr Cys Ala Cys Thr Thr Thr Thr Gly Thr Cys Thr Cys Cys Ala
   580 585 590
   Cys Thr Ala Ala Gly Thr Cys Thr Ala Cys Thr Gly Ala Thr Thr Ala
   595 600 605
   Thr Gly Cys Cys Ala Ala Ala Ala Ala Ala Thr Ala Cys Ala Cys Thr
   610 615 620
   Ala Gly Cys Gly Ala Cys Thr Cys Gly Ala Cys Thr Cys Cys Thr Gly
   625 630 635 640
   Cys Cys Thr Ala Thr Thr Thr Gly Cys Thr Thr Gly Thr Cys Ala Gly
   645 650 655
   Ala Cys Cys Thr Gly Gly Cys Gly Ala Gly Gly Ala Ala Cys Cys Thr
   660 665 670
   Ala Gly Thr Gly Thr Thr Thr Ala Cys Thr Cys Thr Gly Gly Thr Gly
   675 680 685
   Ala Gly Gly Ala Gly Thr Thr Ala Gly Ala Thr Gly Ala Gly Ala Cys
   690 695 700
   Thr Cys Ala Thr Thr Thr Gly Gly Thr Gly Cys Ala Cys Thr Gly Gly
   705 710 715 720
   Ala Thr Thr Gly Ala Thr Ala Thr Thr Gly Ala Gly Thr Cys Cys Ala
   725 730 735
   Ala Ala Cys Cys Thr Cys Thr Ala Thr Thr Thr Gly Gly Ala Gly Ala
   740 745 750
   Cys Ala Thr Thr Gly Ala Cys Gly Gly Ala Thr Cys Cys Ala Cys Cys
   755 760 765
   Thr Thr Cys Ala Ala Ala Thr Cys Ala Thr Ala Thr Gly Cys Thr Gly
   770 775 780
   Ala Ala Gly Cys Thr Ala Ala Cys Ala Thr Cys Cys Cys Thr Thr Thr
   785 790 795 800
   Ala Gly Cys Cys Thr Ala Cys Thr Ala Thr Thr Thr Cys Thr Ala Thr
   805 810 815
   Gly Ala Gly Ala Ala Cys Gly Ala Ala Gly Ala Ala Cys Ala Ala Cys
   820 825 830
   Gly Thr Gly Cys Thr Gly Cys Thr Gly Cys Thr Gly Cys Cys Gly Ala
   835 840 845
   Thr Ala Thr Thr Ala Thr Thr Ala Ala Ala Cys Cys Thr Thr Thr Thr
   850 855 860
   Gly Cys Thr Ala Ala Ala Gly Ala Gly Cys Ala Ala Cys Gly Thr Gly
   865 870 875 880
   Gly Cys Ala Ala Ala Ala Thr Thr Ala Ala Cys Thr Thr Thr Gly Thr
   885 890 895
   Thr Gly Gly Cys Thr Thr Ala Gly Ala Thr Gly Cys Cys Gly Thr Thr
   900 905 910
   Ala Ala Ala Thr Thr Cys Gly Gly Thr Ala Ala Gly Cys Ala Thr Gly
   915 920 925
   Cys Cys Ala Ala Gly Ala Ala Cys Thr Thr Ala Ala Ala Cys Ala Thr
   930 935 940
   Gly Gly Ala Thr Gly Ala Ala Gly Ala Gly Ala Ala Ala Cys Thr Cys
   945 950 955 960
   Cys Cys Thr Cys Thr Ala Thr Thr Thr Gly Thr Cys Ala Thr Thr Cys
   965 970 975
   Ala Thr Gly Ala Thr Thr Thr Gly Gly Thr Gly Ala Gly Cys Ala Ala
   980 985 990
   Cys Ala Ala Gly Ala Ala Gly Thr Thr Thr Gly Gly Ala Gly Thr Thr
   995 1000 1005
   Cys Cys Thr Cys Ala Ala Gly Ala Cys Cys Ala Ala Gly Ala Ala Thr
   1010 1015 1020
   Thr Gly Ala Cys Gly Ala Ala Cys Ala Ala Ala Gly Ala Thr Gly Thr
   1025 1030 1035 1040
   Gly Ala Cys Cys Gly Ala Gly Cys Thr Gly Ala Thr Thr Gly Ala Gly
   1045 1050 1055
   Ala Ala Ala Thr Thr Cys Ala Thr Cys Gly Cys Ala Gly Gly Ala Gly
   1060 1065 1070
   Ala Gly Gly Cys Ala Gly Ala Ala Cys Cys Ala Ala Thr Thr Gly Thr
   1075 1080 1085
   Gly Ala Ala Ala Thr Cys Ala Gly Ala Gly Cys Cys Ala Ala Thr Thr
   1090 1095 1100
   Cys Cys Ala Gly Ala Ala Ala Thr Thr Cys Ala Ala Gly Ala Ala Gly
   1105 1110 1115 1120
   Ala Gly Ala Ala Ala Gly Thr Cys Thr Thr Cys Ala Ala Gly Cys Thr
   1125 1130 1135
   Ala Gly Thr Cys Gly Gly Ala Ala Ala Gly Gly Cys Cys Cys Ala Cys
   1140 1145 1150
   Gly Ala Thr Gly Ala Ala Gly Thr Thr Gly Thr Cys Thr Thr Cys Gly
   1155 1160 1165
   Ala Thr Gly Ala Ala Thr Cys Thr Ala Ala Ala Gly Ala Thr Gly Thr
   1170 1175 1180
   Thr Cys Thr Ala Gly Thr Cys Ala Ala Gly Thr Ala Cys Thr Ala Cys
   1185 1190 1195 1200
   Gly Cys Cys Cys Cys Thr Thr Gly Gly Thr Gly Thr Gly Gly Thr Cys
   1205 1210 1215
   Ala Cys Thr Gly Thr Ala Ala Gly Ala Gly Ala Ala Thr Gly Gly Cys
   1220 1225 1230
   Thr Cys Cys Thr Gly Cys Thr Thr Ala Thr Gly Ala Gly Gly Ala Ala
   1235 1240 1245
   Thr Thr Gly Gly Cys Thr Ala Cys Thr Cys Thr Thr Thr Ala Cys Gly
   1250 1255 1260
   Cys Cys Ala Ala Thr Gly Ala Thr Gly Ala Gly Gly Ala Thr Gly Cys
   1265 1270 1275 1280
   Cys Thr Cys Thr Thr Cys Ala Ala Ala Gly Gly Thr Thr Gly Thr Gly
   1285 1290 1295
   Ala Thr Thr Gly Cys Ala Ala Ala Ala Cys Thr Thr Gly Ala Thr Cys
   1300 1305 1310
   Ala Cys Ala Cys Thr Thr Thr Gly Ala Ala Cys Gly Ala Thr Gly Thr
   1315 1320 1325
   Cys Gly Ala Cys Ala Ala Cys Gly Thr Thr Gly Ala Thr Ala Thr Thr
   1330 1335 1340
   Cys Ala Ala Gly Gly Thr Thr Ala Thr Cys Cys Thr Ala Cys Thr Thr
   1345 1350 1355 1360
   Thr Gly Ala Thr Cys Cys Thr Thr Thr Ala Thr Cys Cys Ala Gly Cys
   1365 1370 1375
   Thr Gly Gly Thr Gly Ala Thr Ala Ala Ala Thr Cys Cys Ala Ala Thr
   1380 1385 1390
   Cys Cys Thr Cys Ala Ala Cys Thr Gly Thr Ala Thr Gly Ala Thr Gly
   1395 1400 1405
   Gly Ala Thr Cys Thr Cys Gly Thr Gly Ala Cys Cys Thr Ala Gly Ala
   1410 1415 1420
   Ala Thr Cys Ala Thr Thr Gly Gly Cys Thr Gly Ala Gly Thr Thr Thr
   1425 1430 1435 1440
   Gly Thr Ala Ala Ala Gly Gly Ala Gly Ala Gly Ala Gly Gly Ala Ala
   1445 1450 1455
   Cys Cys Cys Ala Cys Ala Ala Ala Gly Thr Gly Gly Ala Thr Gly Cys
   1460 1465 1470
   Cys Cys Thr Ala Gly Cys Ala Cys Thr Cys Ala Gly Ala Cys Cys Ala
   1475 1480 1485
   Gly Thr Cys Gly Ala Gly Gly Ala Ala Gly Ala Ala Ala Ala Gly Gly
   1490 1495 1500
   Ala Ala Gly Cys Thr Gly Ala Ala Gly Ala Ala Gly Ala Ala Gly Cys
   1505 1510 1515 1520
   Thr Gly Ala Ala Ala Gly Thr Gly Ala Gly Gly Cys Ala Gly Ala Cys
   1525 1530 1535
   Gly Cys Thr Cys Ala Cys Gly Ala Cys Gly Ala Gly Cys Thr Thr Thr
   1540 1545 1550
   Ala Ala
<210> 43
   <211> 1337
   <212> DNA
   <213> Artificial Sequence
<220>
   <223> human ERO1alpha without leader
<400> 43
   gaagaacaac caccagagac tgctgctcag agatgcttct gtcaggtttc cggttacttg 60
   gacgactgta cttgtgacgt tgagactatc gacagattca acaactacag attgttccca 120
   agattgcaga agttgttgga gtccgactac ttcagatact acaaggttaa cttgaagaga 180
   ccatgtccat tctggaacga catttcccag tgtggtagaa gagactgtgc tgttaagcca 240
   tgtcaatccg acgaagttcc agacggtatt aagtccgctt cctacaagta ctctgaagag 300
   gctaacaact tgatcgaaga gtgtgagcaa gctgaaagat tgggtgctgt tgacgaatct 360
   ttgtccgaga gactcagaag gctgttttgc agtggactaa gcacgatgat tcctccgaca 420
   acttctgtga agctgacgac attcaatctc cagaggctga gtacgttgac ttgttgttga 480
   acccagagag atacactggt tacaagggtc cagacgcttg gaagatttgg aacgttatct 540
   acgaagagaa ctgtttcaag ccacagacta tcaagagacc attgaaccca ttggcttccg 600
   gacagggaac ttctgaagag aacactttct actcttggtt ggagggtttg tgtgttgaga 660
   agagagcttt ctacagattg atctccggat tgcacgcttc tatcaacgtt cacttgtccg 720
   ctagatactt gttgcaagag acttggttgg aaaagaagtg gggtcacaac attactgagt 780
   tccagcagag attcgacggt attttgactg aaggtgaagg tccaagaaga ttgaagaact 840
   tgtacttttt gtacttgatc gagttgagag ctttgtccaa ggttttgcca ttcttcgaga 900
   gaccagactt ccaattgttc actggtaaca agatccagga cgaagagaac aagatgttgt 960
   tgttggagat tttgcacgag atcaagtcct ttccattgca cttcgacgag aactcatttt 1020
   tcgctggtga caagaaagaa gctcacaagt tgaaagagga cttcagattg cacttcagaa 1080
   atatctccag aatcatggac tgtgttggtt gtttcaagtg tagattgtgg ggtaagttgc 1140
   agactcaagg attgggtact gctttgaaga ttttgttctc cgagaagttg atcgctaaca 1200
   tgcctgaatc tggtccatct tacgagttcc acttgactag acaagagatc gtttccttgt 1260
   tcaacgcttt cggtagaatc tccacttccg ttaaagagtt ggagaacttc agaaacttgt 1320
   tgcagaacat ccactaa                                                1337
<210> 44
   <211> 445
   <212> PRT
   <213> Artificial Sequence
<220>
   <223> human ERO1alpha without leader
<400> 44
   Glu Glu Gln Pro Pro Glu Thr Ala Ala Gln Arg Cys Phe Cys Gln Val
    1               5                  10                  15
   Ser Gly Tyr Leu Asp Asp Cys Thr Cys Asp Val Glu Thr Ile Asp Arg
               20                  25                  30
   Phe Asn Asn Tyr Arg Leu Phe Pro Arg Leu Gln Lys Leu Leu Glu Ser
           35                  40                  45
   Asp Tyr Phe Arg Tyr Tyr Lys Val Asn Leu Lys Arg Pro Cys Pro Phe
       50                  55                  60
   Trp Asn Asp Ile Ser Gln Cys Gly Arg Arg Asp Cys Ala Val Lys Pro
   65                  70                  75                  80
   Cys Gln Ser Asp Glu Val Pro Asp Gly Ile Lys Ser Ala Ser Tyr Lys
                   85                  90                  95
   Tyr Ser Glu Glu Ala Asn Asn Leu Ile Glu Glu Cys Glu Gln Ala Glu
               100                 105                 110
   Arg Leu Gly Ala Val Asp Glu Ser Leu Ser Glu Glu Thr Gln Lys Ala
           115                 120                 125
   Val Leu Gln Trp Thr Lys His Asp Asp Ser Ser Asp Asn Phe Cys Glu
       130                 135                 140
   Ala Asp Asp Ile Gln Ser Pro Glu Ala Glu Tyr Val Asp Leu Leu Leu
   145                 150                 155                 160
   Asn Pro Glu Arg Tyr Thr Gly Tyr Lys Gly Pro Asp Ala Trp Lys Ile
                   165                 170                 175
   Trp Asn Val Ile Tyr Glu Glu Asn Cys Phe Lys Pro Gln Thr Ile Lys
               180                 185                 190
   Arg Pro Leu Asn Pro Leu Ala Ser Gly Gln Gly Thr Ser Glu Glu Asn
           195                 200                 205
   Thr Phe Tyr Ser Trp Leu Glu Gly Leu Cys Val Glu Lys Arg Ala Phe
       210                 215                 220
   Tyr Arg Leu Ile Ser Gly Leu His Ala Ser Ile Asn Val His Leu Ser
   225                 230                 235                 240
   Ala Arg Tyr Leu Leu Gln Glu Thr Trp Leu Glu Lys Lys Trp Gly His
                   245                 250                 255
   Asn Ile Thr Glu Phe Gln Gln Arg Phe Asp Gly Ile Leu Thr Glu Gly
               260                 265                 270
   Glu Gly Pro Arg Arg Leu Lys Asn Leu Tyr Phe Leu Tyr Leu Ile Glu
           275                 280                 285
   Leu Arg Ala Leu Ser Lys Val Leu Pro Phe Phe Glu Arg Pro Asp Phe
       290                 295                 300
   Gln Leu Phe Thr Gly Asn Lys Ile Gln Asp Glu Glu Asn Lys Met Leu
   305                 310                 315                 320
   Leu Leu Glu Ile Leu His Glu Ile Lys Ser Phe Pro Leu His Phe Asp
                   325                 330                 335
   Glu Asn Ser Phe Phe Ala Gly Asp Lys Lys Glu Ala His Lys Leu Lys
               340                 345                 350
   Glu Asp Phe Arg Leu His Phe Arg Asn Ile Ser Arg Ile Met Asp Cys
           355                 360                 365
   Val Gly Cys Phe Lys Cys Arg Leu Trp Gly Lys Leu Gln Thr Gln Gly
       370                 375                 380
   Leu Gly Thr Ala Leu Lys Ile Leu Phe Ser Glu Lys Leu Ile Ala Asn
   385                 390                 395                 400
   Met Pro Glu Ser Gly Pro Ser Tyr Glu Phe His Leu Thr Arg Gln Glu
                   405                 410                 415
   Ile Val Ser Leu Phe Asn Ala Phe Gly Arg Ile Ser Thr Ser Val Lys
               420                 425                 430
   Glu Leu Glu Asn Phe Arg Asn Leu Leu Gln Asn Ile His
           435                 440                 445
<210> 45
   <211> 2349
   <212> DNA
   <213> Artificial Sequence
<220>
   <223> human GRP94 without leader
<400> 45
   gatgatgaag ttgacgttga cggtactgtt gaagaggact tgggaaagtc tagagagggt 60
   tccagaactg acgacgaagt tgttcagaga gaggaagagg ctattcagtt ggacggattg 120
   aacgcttccc aaatcagaga gttgagagag aagtccgaga agttcgcttt ccaagctgag 180
   gttaacagaa tgatgaaatt gattatcaac tccttgtaca agaacaaaga gattttcttg 240
   agagagttga tctctaacgc ttctgacgct ttggacaaga tcagattgat ctccttgact 300
   gacgaaaacg ctttgtccgg taacgaagag ttgactgtta agatcaagtg tgacaaagag 360
   aagaacttgt tgcacgttac tgacactggt gttggaatga ctagagaaga gttggttaag 420
   aacttgggta ctatcgctaa gtctggtact tccgagttct tgaacaagat gactgaggct 480
   caagaagatg gtcaatccac ttccgagttg attggtcagt tcggtgttgg tttctactcc 540
   gctttcttgg ttgctgacaa ggttatcgtt acttccaagc acaacaacga cactcaacac 600
   atttgggaat ccgattccaa cgagttctcc gttattgctg acccaagagg taacactttg 660
   ggtagaggta ctactatcac tttggttttg aaagaagagg cttccgacta cttggagttg 720
   gacactatca agaacttggt taagaagtac tcccagttca tcaacttccc aatctatgtt 780
   tggtcctcca agactgagac tgttgaggaa ccaatggaag aagaagaggc tgctaaagaa 840
   gagaaagagg aatctgacga cgaggctgct gttgaagaag aggaagaaga aaagaagcca 900
   aagactaaga aggttgaaaa gactgtttgg gactgggagc ttatgaacga catcaagcca 960
   atttggcaga gaccatccaa agaggttgag gaggacgagt acaaggcttt ctacaagtcc 1020
   ttctccaaag aatccgatga cccaatggct tacatccact tcactgctga gggtgaagtt 1080
   actttcaagt ccatcttgtt cgttccaact tctgctccaa gaggattgtt cgacgagtac 1140
   ggttctaaga agtccgacta catcaaactt tatgttagaa gagttttcat cactgacgac 1200
   ttccacgata tgatgccaaa gtacttgaac ttcgttaagg gtgttgttga ttccgatgac 1260
   ttgccattga acgtttccag agagactttg cagcagcaca agttgttgaa ggttatcaga 1320
   aagaaacttg ttagaaagac tttggacatg atcaagaaga tcgctgacga caagtacaac 1380
   gacactttct ggaaagagtt cggaactaac atcaagttgg gtgttattga ggaccactcc 1440
   aacagaacta gattggctaa gttgttgaga ttccagtcct ctcatcaccc aactgacatc 1500
   acttccttgg accagtacgt tgagagaatg aaagagaagc aggacaaaat ctacttcatg 1560
   gctggttcct ctagaaaaga ggctgaatcc tccccattcg ttgagagatt gttgaagaag 1620
   ggttacgagg ttatctactt gactgagcca gttgacgagt actgtatcca ggctttgcca 1680
   gagtttgacg gaaagagatt ccagaacgtt gctaaagagg gtgttaagtt cgacgaatcc 1740
   gaaaagacta aagaatccag agaggctgtt gagaaagagt tcgagccatt gttgaactgg 1800
   atgaaggaca aggctttgaa ggacaagatc gagaaggctg ttgtttccca gagattgact 1860
   gaatccccat gtgctttggt tgcttcccaa tacggatgga gtggtaacat ggaaagaatc 1920
   atgaaggctc aggcttacca aactggaaag gacatctcca ctaactacta cgcttcccag 1980
   aagaaaactt tcgagatcaa cccaagacac ccattgatca gagacatgtt gagaagaatc 2040
   aaagaggacg aggacgacaa gactgttttg gatttggctg ttgttttgtt cgagactgct 2100
   actttgagat ccggttactt gttgccagac actaaggctt acggtgacag aatcgagaga 2160
   atgttgagat tgtccttgaa cattgaccca gacgctaagg ttgaagaaga accagaagaa 2220
   gagccagagg aaactgctga agatactact gaggacactg aacaagacga ggacgaagag 2280
   atggatgttg gtactgacga agaggaagag acagcaaagg aatccactgc tgaacacgac 2340
   gagttgtaa                                                         2349
<210> 46
   <211> 782
   <212> PRT
   <213> Artificial Sequence
<220>
   <223> human GRP94 without leader
<400> 46
   Asp Asp Glu Val Asp Val Asp Gly Thr Val Glu Glu Asp Leu Gly Lys
    1               5                  10                  15
   Ser Arg Glu Gly Ser Arg Thr Asp Asp Glu Val Val Gln Arg Glu Glu
               20                  25                  30
   Glu Ala Ile Gln Leu Asp Gly Leu Asn Ala Ser Gln Ile Arg Glu Leu
           35                  40                  45
   Arg Glu Lys Ser Glu Lys Phe Ala Phe Gln Ala Glu Val Asn Arg Met
       50                  55                  60
   Met Lys Leu Ile Ile Asn Ser Leu Tyr Lys Asn Lys Glu Ile Phe Leu
   65                  70                  75                  80
   Arg Glu Leu Ile Ser Asn Ala Ser Asp Ala Leu Asp Lys Ile Arg Leu
                   85                  90                  95
   Ile Ser Leu Thr Asp Glu Asn Ala Leu Ser Gly Asn Glu Glu Leu Thr
               100                 105                 110
   Val Lys Ile Lys Cys Asp Lys Glu Lys Asn Leu Leu His Val Thr Asp
           115                 120                 125
   Thr Gly Val Gly Met Thr Arg Glu Glu Leu Val Lys Asn Leu Gly Thr
       130                 135                 140
   Ile Ala Lys Ser Gly Thr Ser Glu Phe Leu Asn Lys Met Thr Glu Ala
   145                 150                 155                 160
   Gln Glu Asp Gly Gln Ser Thr Ser Glu Leu Ile Gly Gln Phe Gly Val
                   165                 170                 175
   Gly Phe Tyr Ser Ala Phe Leu Val Ala Asp Lys Val Ile Val Thr Ser
               180                 185                 190
   Lys His Asn Asn Asp Thr Gln His Ile Trp Glu Ser Asp Ser Asn Glu
           195                 200                 205
   Phe Ser Val Ile Ala Asp Pro Arg Gly Asn Thr Leu Gly Arg Gly Thr
       210                 215                 220
   Thr Ile Thr Leu Val Leu Lys Glu Glu Ala Ser Asp Tyr Leu Glu Leu
   225                 230                 235                 240
   Asp Thr Ile Lys Asn Leu Val Lys Lys Tyr Ser Gln Phe Ile Asn Phe
                   245                 250                 255
   Pro Ile Tyr Val Trp Ser Ser Lys Thr Glu Thr Val Glu Glu Pro Met
               260                 265                 270
   Glu Glu Glu Glu Ala Ala Lys Glu Glu Lys Glu Glu Ser Asp Asp Glu
           275                 280                 285
   Ala Ala Val Glu Glu Glu Glu Glu Glu Lys Lys Pro Lys Thr Lys Lys
       290                 295                 300
   Val Glu Lys Thr Val Trp Asp Trp Glu Leu Met Asn Asp Ile Lys Pro
   305                 310                 315                 320
   Ile Trp Gln Arg Pro Ser Lys Glu Val Glu Glu Asp Glu Tyr Lys Ala
                   325                 330                 335
   Phe Tyr Lys Ser Phe Ser Lys Glu Ser Asp Asp Pro Met Ala Tyr Ile
               340                 345                 350
   His Phe Thr Ala Glu Gly Glu Val Thr Phe Lys Ser Ile Leu Phe Val
           355                 360                 365
   Pro Thr Ser Ala Pro Arg Gly Leu Phe Asp Glu Tyr Gly Ser Lys Lys
       370                 375                 380
   Ser Asp Tyr Ile Lys Leu Tyr Val Arg Arg Val Phe Ile Thr Asp Asp
   385                 390                 395                 400
   Phe His Asp Met Met Pro Lys Tyr Leu Asn Phe Val Lys Gly Val Val
                   405                 410                 415
   Asp Ser Asp Asp Leu Pro Leu Asn Val Ser Arg Glu Thr Leu Gln Gln
               420                 425                 430
   His Lys Leu Leu Lys Val Ile Arg Lys Lys Leu Val Arg Lys Thr Leu
           435                 440                 445
   Asp Met Ile Lys Lys Ile Ala Asp Asp Lys Tyr Asn Asp Thr Phe Trp
       450                 455                 460
   Lys Glu Phe Gly Thr Asn Ile Lys Leu Gly Val Ile Glu Asp His Ser
   465                 470                 475                 480
   Asn Arg Thr Arg Leu Ala Lys Leu Leu Arg Phe Gln Ser Ser His His
                   485                 490                 495
   Pro Thr Asp Ile Thr Ser Leu Asp Gln Tyr Val Glu Arg Met Lys Glu
               500                 505                 510
   Lys Gln Asp Lys Ile Tyr Phe Met Ala Gly Ser Ser Arg Lys Glu Ala
           515                 520                 525
   Glu Ser Ser Pro Phe Val Glu Arg Leu Leu Lys Lys Gly Tyr Glu Val
       530                 535                 540
   Ile Tyr Leu Thr Glu Pro Val Asp Glu Tyr Cys Ile Gln Ala Leu Pro
   545                 550                 555                 560
   Glu Phe Asp Gly Lys Arg Phe Gln Asn Val Ala Lys Glu Gly Val Lys
                   565                 570                 575
   Phe Asp Glu Ser Glu Lys Thr Lys Glu Ser Arg Glu Ala Val Glu Lys
               580                 585                 590
   Glu Phe Glu Pro Leu Leu Asn Trp Met Lys Asp Lys Ala Leu Lys Asp
           595                 600                 605
   Lys Ile Glu Lys Ala Val Val Ser Gln Arg Leu Thr Glu Ser Pro Cys
       610                 615                 620
   Ala Leu Val Ala Ser Gln Tyr Gly Trp Ser Gly Asn Met Glu Arg Ile
   625                 630                 635                 640
   Met Lys Ala Gln Ala Tyr Gln Thr Gly Lys Asp Ile Ser Thr Asn Tyr
                   645                 650                 655
   Tyr Ala Ser Gln Lys Lys Thr Phe Glu Ile Asn Pro Arg His Pro Leu
               660                 665                 670
   Ile Arg Asp Met Leu Arg Arg Ile Lys Glu Asp Glu Asp Asp Lys Thr
           675                 680                 685
   Val Leu Asp Leu Ala Val Val Leu Phe Glu Thr Ala Thr Leu Arg Ser
       690                 695                 700
   Gly Tyr Leu Leu Pro Asp Thr Lys Ala Tyr Gly Asp Arg Ile Glu Arg
   705                 710                 715                 720
   Met Leu Arg Leu Ser Leu Asn Ile Asp Pro Asp Ala Lys Val Glu Glu
                   725                 730                 735
   Glu Pro Glu Glu Glu Pro Glu Glu Thr Ala Glu Asp Thr Thr Glu Asp
               740                 745                 750
   Thr Glu Gln Asp Glu Asp Glu Glu Met Asp Val Gly Thr Asp Glu Glu
           755                 760                 765
   Glu Glu Thr Ala Lys Glu Ser Thr Ala Glu His Asp Glu Leu
       770                 775                 780
<210> 47
   <211> 8448
   <212> DNA
   <213> Pichia pastoris
<220>
   <221> CDS
   <222> (3016)...(5385)
   <223> Encodes PMT1
<400> 47
   actttttcaa ttcctcaggg tactccgttg gaattctgta cttagcagca tactgatctt 60
   tgaccaccca aggagcacca gatctttgcg atctagtcaa cgtcaacttg agaaaagttt 120
   tcacgtacca cttagtgaac gcattcctat cacgggaaac ttgattttcg ttcacggtta 180
   cttctccatc agagtttgag aggccaacgc gataagagca gtatccttca cgtacggtac 240
   catcaggtaa ggtgatggga gcaaaccgtg ccttttctct gatgatccct ttatatctgt 300
   tagatccagc acttttaaca ttcactagat ccccaggaaa aaattctttc ttgaagtgta 360
   aatacacgtc atcgactaat tgatctagtc tgggtatgag actgaattgc acatatctca 420
   aaattggttc tctgacaggt tctggaaact tattttcaac cgcttgcatt tcctcctgtt 480
   cgtacttgag ggcctcaaag aaatcaaacg agctgtttcc agtgatttca cacgcaaact 540
   tcttttggct ataatagtcc atcctatcaa ggtactcatc gtagttcaaa aaccactcgc 600
   cagtctgtgg aatgtaccaa atttccgtgt ctagatcatc aggaagctgt tgtggaggga 660
   caacttccac ctgctttctt ttgaagagaa ccatggtgtt tggggattag aagaaaacaa 720
   atatttgagc ggaacttgcg aaaaaacgcc cctagcgaat gcaagctaga catgtcagga 780
   agataaaatt gataccgcag aagcaggggt agttggggag ggcaatcaag tacgttcaca 840
   gagcatggct gcgttatcaa ctgactattt tatggcgtgg tttagaagag agagtatcaa 900
   ttaggcgtca actgggacca ttatgattag acgttgtagg tagatgcagg tgaaaaatgg 960
   acagacgtag gcaacaaaca caaactgtcg ggtaaccttt aacagtattc aattccaggt 1020
   gtttcaagac agccttagat actagcaagc ttccagggaa accctattac tcatgctccc 1080
   actgttggaa ctcacaacca agaggctaca tgtatgcgta tgcatacagg tactgctcag 1140
   tgataaattt atttcgcgag atcgtactcc agaaactttc atgtaagcct tcctacttcg 1200
   ctctgcccac tatgttagcc agaaaggtat tagctagaca atgtctggtg gtagccaggc 1260
   tttgtgcggg tagatttgcc tcctcattat gcgggtgcag ttgtagaggt ttgatgaggc 1320
   caccaaaatt taacagttcc aaatctcttt cgagatcgat gacctcatcg tccctgtttg 1380
   agtctccaaa ttgtccttcc tgtggtgtgg ttctccaaac agaacatcca gacaaagatg 1440
   ggtattgtct actgcccaaa ggtgaaagga aagttaaaaa ttatcaaaat gaactaaaag 1500
   aaaagctttt tttgaatgtg aaaagggaag aacttgccga cagactgggc catgaggtgg 1560
   actctgaatc actgattata cccaaggaaa tgtaccaaaa gccccgtacc ccgaaacgac 1620
   tggtttgtca gagatgcttc aaatcgcaaa actattcctt gatcgaccat tccattcgtg 1680
   aagaaaatcc cgaacacaag atcctggatg agatcccttc aaacgccaat atcgtccacg 1740
   ttttatctgc tgttgatttt cctcttggtc tcagcaagga actggtaaac agatttaaac 1800
   ccactcagat tacgtacgtt attacaaagt ctgacgtgtt cttccccgat aagctaggtc 1860
   tccaacggac gggagctgct tattttgaag acagcttggt aaagcttgtc ggtgcagatc 1920
   ctagaaaggt agtattggtc tcaggaaaaa gaaattgggg cctcaaacag ctgctatcca 1980
   ctttacccag aggtcccaat tactttctgg gaatgacgaa caccggaaaa tcaaccctaa 2040
   tacgatccat cgttggtaag gattactcaa agaagcagac agagaatggc ccgggtgtct 2100
   ctcaccttcc ttcattcaca agaaaaccca tgaagttcaa aatggacaac aacagtcttg 2160
   aactcgtaga tctccctgga tacactgctc caaatggagg tgtttacaag tatcttaagg 2220
   aagagaacta ccgagacatt ttgaacgtta aacagttaaa gccattgaca tccctcaagg 2280
   catacacaga aacgttgcct tcgaagccaa aactattcaa tggtgtgcga gtaatatgca 2340
   ttggtggttt agtgtacatt cggcccccaa agggtgtagt gctgaaacag tttagtctcg 2400
   tcaaccttcc atccttcatg tactcgtcgc taaaaaaggc caccagtgta atccaagcgc 2460
   ccccacaagc cttggtgaat tgcagcgtcg tcaaggagga cagtccagat gaactggtaa 2520
   gatatgtgat ccctccattt tatggtttaa ttgacctggt cattcaaggt gttggattta 2580
   tcaagcttct gcccactgga gctcggaaca ccagagaact gatagaaatt tttgccccaa 2640
   aagacatcca gctcatggtg cgtgattcca tcctcaaata cgtctacaag acccatgccg 2700
   aacacgactc aaccaataat ctcctgcata aaaagaacat aaaagccaga ggccaaacca 2760
   tactacgaag actacccaaa aagcctgtat tcacaaagct ttttcccgta ccagccaacg 2820
   taccgtctca tgaactgctc accatggtga cgggaaagga cgacctagcc gaggaagaca 2880
   aagaataccg ctacgatatc cagtatccca acagatactg ggatgaaacc atctgtaaat 2940
   agaatgctta tgtaatcaag cactttctga aattccttag agtttcgcgt gtctccccgt 3000
   caaaaatcgc gtctc atg tgc cag ata ttt ctc ccg caa aac gta aca cgt  3051
                    Met Cys Gln Ile Phe Leu Pro Gln Asn Val Thr Arg
                    1               5                   10
   tgt tct gtt tcc ctt ttg aca atg agt aaa aca agt cct caa gag gtg   3099
   Cys Ser Val Ser Leu Leu Thr Met Ser Lys Thr Ser Pro Gln Glu Val
           15                  20                  25
   cca gaa aac act act gag ctt aaa atc tca aaa gga gag ctc cgt cct   3147
   Pro Glu Asn Thr Thr Glu Leu Lys Ile Ser Lys Gly Glu Leu Arg Pro
       30                  35                  40
   ttt att gtg acc tct cca tct cct caa ttg agc aag tct cgt tct gtg   3195
   Phe Ile Val Thr Ser Pro Ser Pro Gln Leu Ser Lys Ser Arg Ser Val
   45                  50                  55                  60
   act tca acc aag gag aag ctg ata ttg gct agt ttg ttc ata ttt gca   3243
   Thr Ser Thr Lys Glu Lys Leu Ile Leu Ala Ser Leu Phe Ile Phe Ala
                   65                  70                  75
   atg gtc atc agg ttc cac aac gtc gcc cac cct gac agc gtt gtg ttt   3291
   Met Val Ile Arg Phe His Asn Val Ala His Pro Asp Ser Val Val Phe
               80                  85                  90
   gat gaa gtt cac ttt ggg ggg ttt gcc aga aag tac att ttg gga acc   3339
   Asp Glu Val His Phe Gly Gly Phe Ala Arg Lys Tyr Ile Leu Gly Thr
           95                  100                 105
   ttt ttc atg gat gtt cat ccg cca ttg gcc aag cta tta ttt gct ggt   3387
   Phe Phe Met Asp Val His Pro Pro Leu Ala Lys Leu Leu Phe Ala Gly
       110                 115                 120
   gtt ggc agt ctt ggt gga tac gat gga gag ttt gag ttc aag aaa att   3435
   Val Gly Ser Leu Gly Gly Tyr Asp Gly Glu Phe Glu Phe Lys Lys Ile
   125                 130                 135                 140
   ggt gac gaa ttc cca gag aat gtt cct tat gtg ctc atg aga tat ctt   3483
   Gly Asp Glu Phe Pro Glu Asn Val Pro Tyr Val Leu Met Arg Tyr Leu
                   145                 150                 155
   ccc tct ggt atg gga gtt gga aca tgt att atg ttg tat ttg act ctg   3531
   Pro Ser Gly Met Gly Val Gly Thr Cys Ile Met Leu Tyr Leu Thr Leu
               160                 165                 170
   aga gct tct ggt tgt caa cca ata gtc tgt gct ctg aca acc gct ctt   3579
   Arg Ala Ser Gly Cys Gln Pro Ile Val Cys Ala Leu Thr Thr Ala Leu
           175                 180                 185
   ttg atc att gag aat gct aat gtt aca atc tcc aga ttc att ttg ctg   3627
   Leu Ile Ile Glu Asn Ala Asn Val Thr Ile Ser Arg Phe Ile Leu Leu
       190                 195                 200
   gat tcg cca atg ctg ttt ttt att gct tca aca gtt tac tct ttc aag   3675
   Asp Ser Pro Met Leu Phe Phe Ile Ala Ser Thr Val Tyr Ser Phe Lys
   205                 210                 215                 220
   aaa ttt caa att cag gaa ccg ttt acc ttc caa tgg tac aag acc ctt   3723
   Lys Phe Gln Ile Gln Glu Pro Phe Thr Phe Gln Trp Tyr Lys Thr Leu
                   225                 230                 235
   att gct act ggt gtt tct tta ggg tta gca gct tcc agt aaa tgg gtt   3771
   Ile Ala Thr Gly Val Ser Leu Gly Leu Ala Ala Ser Ser Lys Trp Val
               240                 245                 250
   ggt ttg ttc acc gtt gcc tgg att gga ttg ata aca att tgg gac tta   3819
   Gly Leu Phe Thr Val Ala Trp Ile Gly Leu Ile Thr Ile Trp Asp Leu
           255                 260                 265
   tgg ttc atc att ggt gat ttg act gtt tct gta aag aaa att ttc ggc   3867
   Trp Phe Ile Ile Gly Asp Leu Thr Val Ser Val Lys Lys Ile Phe Gly
       270                 275                 280
   cat ttt atc acc aga gct gta gct ttc tta gtc gtc ccc act ctg atc   3915
   His Phe Ile Thr Arg Ala Val Ala Phe Leu Val Val Pro Thr Leu Ile
   285                 290                 295                 300
   tac ctc act ttc ttt gcc atc cat ttg caa gtc tta acc aag gaa ggt   3963
   Tyr Leu Thr Phe Phe Ala Ile His Leu Gln Val Leu Thr Lys Glu Gly
                   305                 310                 315
   gat ggt ggt gct ttc atg tct tcc gtc ttc aga tcg acc tta gaa ggt   4011
   Asp Gly Gly Ala Phe Met Ser Ser Val Phe Arg Ser Thr Leu Glu Gly
               320                 325                 330
   aat gct gtt cca aaa cag tcg ctg gcc aac gtt ggt ttg ggc tct tta   4059
   Asn Ala Val Pro Lys Gln Ser Leu Ala Asn Val Gly Leu Gly Ser Leu
           335                 340                 345
   gtc act atc cgt cat ttg aac acc aga ggt ggt tac tta cac tct cac   4107
   Val Thr Ile Arg His Leu Asn Thr Arg Gly Gly Tyr Leu His Ser His
       350                 355                 360
   aat cat ctt tac gag ggt ggt tct ggt caa cag cag gtc acc ttg tac   4155
   Asn His Leu Tyr Glu Gly Gly Ser Gly Gln Gln Gln Val Thr Leu Tyr
   365                 370                 375                 380
   cca cac att gat tct aat aat caa tgg att gta cag gat tac aac gcg   4203
   Pro His Ile Asp Ser Asn Asn Gln Trp Ile Val Gln Asp Tyr Asn Ala
                   385                 390                 395
   act gag gag cca act gaa ttt gtt cca ttg aaa gac ggt gtc aaa atc   4251
   Thr Glu Glu Pro Thr Glu Phe Val Pro Leu Lys Asp Gly Val Lys Ile
               400                 405                 410
   aga tta aac cac aaa ttg act tcc cga aga ttg cac tct cat aac ctc   4299
   Arg Leu Asn His Lys Leu Thr Ser Arg Arg Leu His Ser His Asn Leu
           415                 420                 425
   aga cct cct gtg act gaa caa gat tgg caa aat gag gta tct gct tat   4347
   Arg Pro Pro Val Thr Glu Gln Asp Trp Gln Asn Glu Val Ser Ala Tyr
       430                 435                 440
   gga cat gag ggc ttt ggc ggt gat gcc aat gat gac ttt gtt gtg gag   4395
   Gly His Glu Gly Phe Gly Gly Asp Ala Asn Asp Asp Phe Val Val Glu
   445                 450                 455                 460
   att gcc aag gat ctt tca act act gaa gaa gct aag gaa aac gtt agg    4443
   Ile Ala Lys Asp Leu Ser Thr Thr Glu Glu Ala Lys Glu Asn Val Arg
                   465                 470                 475
   gcc att caa act gtt ttt aga ttg aga cat gcg atg act ggt tgt tac    4491
   Ala Ile Gln Thr Val Phe Arg Leu Arg His Ala Met Thr Gly Cys Tyr
               480                 485                 490
   ttg ttc tcc cac gaa gtc aag ctt ccc aag tgg gca tat gag caa caa    4539
   Leu Phe Ser His Glu Val Lys Leu Pro Lys Trp Ala Tyr Glu Gln Gln
           495                 500                 505
   gag gtt act tgt gct act caa ggt atc aaa cca cta tct tac tgg tac    4587
   Glu Val Thr Cys Ala Thr Gln Gly Ile Lys Pro Leu Ser Tyr Trp Tyr
       510                 515                 520
   gtt gag acc aac gaa aac cca ttc ttg gat aaa gag gtt gat gaa ata    4635
   Val Glu Thr Asn Glu Asn Pro Phe Leu Asp Lys Glu Val Asp Glu Ile
   525                 530                 535                 540
   gtt agc tat cct gtt ccg act ttc ttt caa aag gtt gcc gag cta cac    4683
   Val Ser Tyr Pro Val Pro Thr Phe Phe Gln Lys Val Ala Glu Leu His
                   545                 550                 555
   gcc aga atg tgg aag atc aac aag ggc tta act gat cat cat gtc tat    4731
   Ala Arg Met Trp Lys Ile Asn Lys Gly Leu Thr Asp His His Val Tyr
               560                 565                 570
   gaa tcc agt cca gat tct tgg ccc ttc ctg ctc aga ggt ata agc tac    4779
   Glu Ser Ser Pro Asp Ser Trp Pro Phe Leu Leu Arg Gly Ile Ser Tyr
           575                 580                 585
   tgg tca aaa aat cac tca caa att tat ttc ata ggt aat gct gtc act    4827
   Trp Ser Lys Asn His Ser Gln Ile Tyr Phe Ile Gly Asn Ala Val Thr
       590                 595                 600
   tgg tgg aca gtc acc gca agt att gct ttg ttc tct gtc ttt ttg gtt    4875
   Trp Trp Thr Val Thr Ala Ser Ile Ala Leu Phe Ser Val Phe Leu Val
   605                 610                 615                 620
   ttc tct att ctg aga tgg caa aga ggt ttt ggg ttc agc gtt gac cca    4923
   Phe Ser Ile Leu Arg Trp Gln Arg Gly Phe Gly Phe Ser Val Asp Pro
                   625                 630                 635
   act gtg ttc aac ttc aat gtt caa atg ctt cat tac atc cta gga tgg    4971
   Thr Val Phe Asn Phe Asn Val Gln Met Leu His Tyr Ile Leu Gly Trp
               640                 645                 650
   gta ctg cat tac ttg cca tct ttc ctt atg gcc cgt cag cta ttt ttg    5019
   Val Leu His Tyr Leu Pro Ser Phe Leu Met Ala Arg Gln Leu Phe Leu
           655                 660                 665
   cac cac tat cta cca tca ttg tac ttt ggt ata ttg gct ctc gga cat    5067
   His His Tyr Leu Pro Ser Leu Tyr Phe Gly Ile Leu Ala Leu Gly His
       670                 675                 680
   gtg ttt gag att att cac tct tat gtc ttc aaa aac aaa cag gtt gtg    5115
   Val Phe Glu Ile Ile His Ser Tyr Val Phe Lys Asn Lys Gln Val Val
   685                 690                 695                 700
   tct tac tcc ata ttc gtt ctc ttt ttt gcc gtt gcg ctt tct ttc ttc     5163
   Ser Tyr Ser Ile Phe Val Leu Phe Phe Ala Val Ala Leu Ser Phe Phe
                   705                 710                 715
   caa aga tat tct cca ttg atc tat gca gga cga tgg acc aag gac caa     5211
   Gln Arg Tyr Ser Pro Leu Ile Tyr Ala Gly Arg Trp Thr Lys Asp Gln
               720                 725                 730
   tgc aac gaa tcc aag ata ctc aag tgg gac ttt gac tgt aac acc ttc     5259
   Cys Asn Glu Ser Lys Ile Leu Lys Trp Asp Phe Asp Cys Asn Thr Phe
           735                 740                 745
   ccc agt cac aca tct cag tat gaa ata tgg gca tcc cct gta caa act     5307
   Pro Ser His Thr Ser Gln Tyr Glu Ile Trp Ala Ser Pro Val Gln Thr
       750                 755                 760
   tcc act cct aaa gaa gga acc cac tca gaa tct acc gtc gga gaa cct     5355
   Ser Thr Pro Lys Glu Gly Thr His Ser Glu Ser Thr Val Gly Glu Pro
   765                 770                 775                 780
   gac gtt gag aag ctg gga gag aca gtc taa gctgtgttta tatagccctg       5405
   Asp Val Glu Lys Leu Gly Glu Thr Val  *
                   785
   tacgtaaaat ctatgacaca agtttatggt tatttgtctt atgtaagcaa tatttggatt 5465
   gatgtctcga gaccatcaac tccatcactg ataagttgat cggatttgta tttctgtccc 5525
   ctatttacta attccctttc cagaaataga tcatgaatga ggcagaatat aagtgccaaa 5585
   gatgccggct gccgttgacc atagacggat ctctggaaga ccttagcata tcacaggcca 5645
   atcttttgac gggacgaaat gggaacttta caaagaacac aatccccttg gaggatgccg 5705
   tggaagaaga tttacccaag gtgcctcaga gccgacttaa cctctttaaa gaggtctacc 5765
   agaagatgga tcacgatttt accaatgcca gagatgaatt tgttgtgttg aacaagcaca 5825
   atgataacag cgacgtcaat gtggagtatg attacgaaga aaacaacact atcagtcgta 5885
   gaatcaacac aatgacgaat atcttcaata tcctcagcaa caagtacgaa attgattttc 5945
   cggtttgcta cgaatgcgcc acattgctga tggaggaatt gaagaatgag tacgaaaggg 6005
   tcaatgctga taaagaagtt tacgcaaagt ttctatccaa gcttcgcaaa caggacgcag 6065
   gtacaaatat gaaagaaaga actgctcaac tactggagca attggagaaa actaagcaag 6125
   aagagagaga taaagaaaag aagctccaag gcctatatga tgaaagagat agtttggaaa 6185
   aggtattagc ttctttagag aatgaaatgg aacagttgaa tattgaagag cagcaaattt 6245
   ttgaattaga gaacaaatat gaatatgagt taatggagtt caagaatgag caaagcagaa 6305
   tggaagcaat gtatgaggat ggtttgacgc aattagataa tttaagaaaa gtgaacgtct 6365
   ttaatgacgc tttcaatatc tcgcatgatg gtcaattcgg cactataaat gggctcaggt 6425
   tgggcacgtt agacagtaag agggtttctt ggtatgaaat aaatgctgcg ttgggtcaag 6485
   ttgttttgtt actcttcacg ttattgagca gacttgagct tgagctcaaa cattacaaga 6545
   tttttcccat tggctcgact tccaagattg aataccaagt tgacccagat tccaaacctg 6605
   ttactattaa ctgcttttct tcgggagaac agttactgga taagcttttt cattctaata 6665
   aactagatcc tgctatgaac gcaatcctag aaatcactat tcaaattgca gatcatttca 6725
   caaaacaaga tccaacaaac gaattgccct acaaaatgga gaacgaaaca atatcaaact 6785
   tgaatatcaa accttccaaa cgtaaatcca acgaggaatg gactttggca tgcaaacatc 6845
   tgttgaccaa tctcaaatgg ataattgcct tcagtagttc aacgtgaact agtgtattaa 6905
   aaaaaagaaa cagaaacttt attggattat aaaactattt atcaagttca aattaacata 6965
   gcgacgaaga gaccagctgc ggctaagact gaactaccta gtaccgcttg ggcaccgtta 7025
   ccagtttctg tacctgtgcc agtggtacca gtaccagtac cggttccagt gccagttcct 7085
   gtgcctgtgc ctgtgcctgt gccggttccg gttccagtgc ctgtgcctgt gcccgtgcct 7145
   gttgcagtgg tattagtgaa acctcctgtg ccagttgcag tggtattagt aaatcctcct 7205
   gttcctgtgg tgtttgtgag tcctccagtt tcggtcaagt ttccaggaac actaacatca 7265
   ggggttgaag tgatctctgg tggcaccgtg gggactgtga cattgacatc atttgtgaag 7325
   attggctcca actcagttgt agccttaaca acgcttaatg cgagagttgc accgatcaaa 7385
   cttttgaatt gcattttact tttgttactt ctaaaatgag atgaggaaag aaagaagaga 7445
   gaagtggaag cactgaaagt gtggtgttat atctgaaaaa ttcattacca atcaaaacgt 7505
   cagacgatga tatgtctaag cccgtgcaga aacgtctaga tcttttcaaa cgtaaagtac 7565
   ttcccctttt ggcacatcgt ggacttgcta ttccaaatat agacggggac cttttttaga 7625
   gtatccccgg gcgcctcgaa ttctggggta tttttttgct atagcatgaa ttggcaatag 7685
   ggattgggga caacgtgttt gacagaagac gtgtgtgtcc tgccaaaaag gggtaaaggt 7745
   gcatttgcca aggcctgtga atgatctgaa cactagagga aagcaagaag gctgtgtcgt 7805
   agtctgtatt ggctgtgttg tcgctgtgtc ggttgcttca aaactttatt cgagtccggt 7865
   acgcgtcaat gggtattttt caaaaagttt ctaactccct caatcaactt tggttttggc 7925
   cggatatggc atgccagaaa aggaagtttt actcctggcg atgatgttta caaatcaagc 7985
   ttagagggag taaccaatgc agataagttt gcgatggcgc tgatctttat gctctcaaca 8045
   ccttctcgac tattcagggt catttcgtgg ctttgtattt cgggcacaac tgatcaccga 8105
   ggatcaatga aattttcatg cacatcactg atccagtttc tgtcgaattt gcaattccag 8165
   ttgattgcag gacccgcgtt ctgcctacac attttctcgt gattgtggaa gtaattctaa 8225
   ttgacagtcg atcaccacaa tgacaatctt agttgacctt agattccagt ggaatgcagt 8285
   tgaattgtct tttcgtttaa ttagaggaga gtaacggacc agggctcctt tattgtatat 8345
   aataattata atttttttca ctatttcacc ttttcgcttg gaatataaaa ttctaattat 8405
   aattcaacag gaaatattgt ccaaaccaca tgaagttgtc atg                   8448
<210> 48
   <211> 789
   <212> PRT
   <213> Pichia pastoris
<400> 48
   Met Cys Gln Ile Phe Leu Pro Gln Asn Val Thr Arg Cys Ser Val Ser
    1               5                  10                  15
   Leu Leu Thr Met Ser Lys Thr Ser Pro Gln Glu Val Pro Glu Asn Thr
               20                  25                  30
   Thr Glu Leu Lys Ile Ser Lys Gly Glu Leu Arg Pro Phe Ile Val Thr
           35                  40                  45
   Ser Pro Ser Pro Gln Leu Ser Lys Ser Arg Ser Val Thr Ser Thr Lys
       50                  55                  60
   Glu Lys Leu Ile Leu Ala Ser Leu Phe Ile Phe Ala Met Val Ile Arg
   65                  70                  75                  80
   Phe His Asn Val Ala His Pro Asp Ser Val Val Phe Asp Glu Val His
                   85                  90                  95
   Phe Gly Gly Phe Ala Arg Lys Tyr Ile Leu Gly Thr Phe Phe Met Asp
               100                 105                 110
   Val His Pro Pro Leu Ala Lys Leu Leu Phe Ala Gly Val Gly Ser Leu
           115                 120                 125
   Gly Gly Tyr Asp Gly Glu Phe Glu Phe Lys Lys Ile Gly Asp Glu Phe
       130                 135                 140
   Pro Glu Asn Val Pro Tyr Val Leu Met Arg Tyr Leu Pro Ser Gly Met
   145                 150                 155                 160
   Gly Val Gly Thr Cys Ile Met Leu Tyr Leu Thr Leu Arg Ala Ser Gly
                   165                 170                 175
   Cys Gln Pro Ile Val Cys Ala Leu Thr Thr Ala Leu Leu Ile Ile Glu
               180                 185                 190
   Asn Ala Asn Val Thr Ile Ser Arg Phe Ile Leu Leu Asp Ser Pro Met
           195                 200                 205
   Leu Phe Phe Ile Ala Ser Thr Val Tyr Ser Phe Lys Lys Phe Gln Ile
       210                 215                 220
   Gln Glu Pro Phe Thr Phe Gln Trp Tyr Lys Thr Leu Ile Ala Thr Gly
   225                 230                 235                 240
   Val Ser Leu Gly Leu Ala Ala Ser Ser Lys Trp Val Gly Leu Phe Thr
                   245                 250                 255
   Val Ala Trp Ile Gly Leu Ile Thr Ile Trp Asp Leu Trp Phe Ile Ile
               260                 265                 270
   Gly Asp Leu Thr Val Ser Val Lys Lys Ile Phe Gly His Phe Ile Thr
           275                 280                 285
   Arg Ala Val Ala Phe Leu Val Val Pro Thr Leu Ile Tyr Leu Thr Phe
       290                 295                 300
   Phe Ala Ile His Leu Gln Val Leu Thr Lys Glu Gly Asp Gly Gly Ala
   305                 310                 315                 320
   Phe Met Ser Ser Val Phe Arg Ser Thr Leu Glu Gly Asn Ala Val Pro
                   325                 330                 335
   Lys Gln Ser Leu Ala Asn Val Gly Leu Gly Ser Leu Val Thr Ile Arg
               340                 345                 350
   His Leu Asn Thr Arg Gly Gly Tyr Leu His Ser His Asn His Leu Tyr
           355                 360                 365
   Glu Gly Gly Ser Gly Gln Gln Gln Val Thr Leu Tyr Pro His Ile Asp
       370                 375                 380
   Ser Asn Asn Gln Trp Ile Val Gln Asp Tyr Asn Ala Thr Glu Glu Pro
   385                 390                 395                 400
   Thr Glu Phe Val Pro Leu Lys Asp Gly Val Lys Ile Arg Leu Asn His
                   405                 410                 415
   Lys Leu Thr Ser Arg Arg Leu His Ser His Asn Leu Arg Pro Pro Val
               420                 425                 430
   Thr Glu Gln Asp Trp Gln Asn Glu Val Ser Ala Tyr Gly His Glu Gly
           435                 440                 445
   Phe Gly Gly Asp Ala Asn Asp Asp Phe Val Val Glu Ile Ala Lys Asp
       450                 455                 460
   Leu Ser Thr Thr Glu Glu Ala Lys Glu Asn Val Arg Ala Ile Gln Thr
   465                 470                 475                 480
   Val Phe Arg Leu Arg His Ala Met Thr Gly Cys Tyr Leu Phe Ser His
                   485                 490                 495
   Glu Val Lys Leu Pro Lys Trp Ala Tyr Glu Gln Gln Glu Val Thr Cys
               500                 505                 510
   Ala Thr Gln Gly Ile Lys Pro Leu Ser Tyr Trp Tyr Val Glu Thr Asn
           515                 520                 525
   Glu Asn Pro Phe Leu Asp Lys Glu Val Asp Glu Ile Val Ser Tyr Pro
       530                 535                 540
   Val Pro Thr Phe Phe Gln Lys Val Ala Glu Leu His Ala Arg Met Trp
   545                 550                 555                 560
   Lys Ile Asn Lys Gly Leu Thr Asp His His Val Tyr Glu Ser Ser Pro
                   565                 570                 575
   Asp Ser Trp Pro Phe Leu Leu Arg Gly Ile Ser Tyr Trp Ser Lys Asn
               580                 585                 590
   His Ser Gln Ile Tyr Phe Ile Gly Asn Ala Val Thr Trp Trp Thr Val
           595                 600                 605
   Thr Ala Ser Ile Ala Leu Phe Ser Val Phe Leu Val Phe Ser Ile Leu
       610                 615                 620
   Arg Trp Gln Arg Gly Phe Gly Phe Ser Val Asp Pro Thr Val Phe Asn
   625                 630                 635                 640
   Phe Asn Val Gln Met Leu His Tyr Ile Leu Gly Trp Val Leu His Tyr
                   645                 650                 655
   Leu Pro Ser Phe Leu Met Ala Arg Gln Leu Phe Leu His His Tyr Leu
               660                 665                 670
   Pro Ser Leu Tyr Phe Gly Ile Leu Ala Leu Gly His Val Phe Glu Ile
           675                 680                 685
   Ile His Ser Tyr Val Phe Lys Asn Lys Gln Val Val Ser Tyr Ser Ile
       690                 695                 700
   Phe Val Leu Phe Phe Ala Val Ala Leu Ser Phe Phe Gln Arg Tyr Ser
   705                 710                 715                 720
   Pro Leu Ile Tyr Ala Gly Arg Trp Thr Lys Asp Gln Cys Asn Glu Ser
                   725                 730                 735
   Lys Ile Leu Lys Trp Asp Phe Asp Cys Asn Thr Phe Pro Ser His Thr
               740                 745                 750
   Ser Gln Tyr Glu Ile Trp Ala Ser Pro Val Gln Thr Ser Thr Pro Lys
           755                 760                 765
   Glu Gly Thr His Ser Glu Ser Thr Val Gly Glu Pro Asp Val Glu Lys
       770                 775                 780
   Leu Gly Glu Thr Val
   785
<210> 49
   <211> 8400
   <212> DNA
   <213> Pichia pastoris
<220>
   <221> CDS
   <222> (3169)...(5394)
   <223> Encodes PMT4
<400> 49
   tagtaaagaa atcttgcagt ttaattcttc ctcttgtgtt tttagcgatg agacatcggc 60
   actcagagtt aagtttgctt gcatctgctc tgataacttt tgctgtgact ctgttgcaat 120
   gcttttggta acggtcaatt cgtctatggt ttgttgatac tttgacttta aggcagtaat 180
   attgtcctgt agtttatcat tatatgcttc caatgttttg acctttgatg aaatgttttt 240
   tcgattaaca gttagttcat cgaaggagag ctccaactct gatacttgca ttcttaaatt 300
   atttataatg gtatccttaa cttctagtga tttcgagtgg cttgcctggg cactcttaag 360
   ttcttttctc aactgtgcta tggatggctc aagcactaga atttgtttct ctgaatcgaa 420
   taattttatt tctagcttct gagcaagctc acaggcgctt actttttcgg aagttagaaa 480
   ctttgcttcg ttattcatgg cagacagttc tattcttaat tgcttatttt ctttcctaac 540
   ttccaaaatc tccgattcca ggggttcata tctacgggag gaaacctgat tgcatgactt 600
   ttcgaacgtt ttttgatcag aaagttgaca gattgtgcca tcagttgacg agacagcctc 660
   aaactgagtt gcttccatgt tgcacaaatt atcattgaat tcagccactt ctttctccaa 720
   atctccgttt accagctcct tcttctttcc tgaagcaata gatgatgatc gatgaatata 780
   gtcctctttc aatgggtttt ggatctcttt gtcccattga caagaagcta tgctccttga 840
   atccttcatt gacattgggt atgaaatttt gctaccatct acctttgcac taatttctgt 900
   gggcgaattg tgtgttttca gtagatcttc aagtgctttc ttttcgtttt ctatcttcat 960
   aagagatatt ctcaatttat ttacggtgtc agtggcagac aaattgttta atgaaacggt 1020
   atccagagac tcctgctcca ggtactctga ctgagctaca gggaagggtt tcttgtcgtg 1080
   tatggagaac ttctgctcaa gttgggcttt caaggaattc acttggtgat gcaaaagctc 1140
   attttcctca ttcaaagaag tattcatatt tttaagttcc tccagctcaa acgtactgcg 1200
   gccttccaaa gtgcagattt tatccttaag actcaatttc tcattcttca aactttccat 1260
   ctctctattg agcgtttcaa cctggtcagt tttcaacttg agttcttctg aaaatttgat 1320
   acttgaactt ttagcaaggg aagcttcatc aaaagtatct tgtagcttgg tttctaaaga 1380
   ccagttagaa tcaagtagcc tttgctgctc ttgttccaac tttttggaaa aaattgcctg 1440
   gtgagtgatc ttttcagcga gatcctcatt ttctttaact ttttgcttca atagagattt 1500
   gagtttttga ttatcagagt tcagcttccg acattcgact aaaaggttgt cactaatacc 1560
   tgttaaaaaa tctattttgt tcgtcttttt tttgcttggc gactttagag gtaatgcagg 1620
   aagggaagga tgaaattctg actcctggtg ccgatttctc agctttctcg gaagtggggg 1680
   tagctgaaat ggtacattgt ggttcgtatc accaagatcc atttttatgt ctttgtccat 1740
   tagaaaattc aagaatcttt caaaaaaaat agaaacagaa gatttagtaa acttaggtga 1800
   ggtgatataa acctaattgc ctgttttatt ttgatcatgt atgtaaattg tgaaaggtaa 1860
   atacgcgaaa cttatgtatg tattgcaaag atgcacaaga cacacaagga ttaatgggct 1920
   atttgctcta cattcgcaaa aaatagccag catttatttt ttgaatggat actcaataag 1980
   cccatcccta cgcttccata tctttttttt ctttttggta gtaacatgct ccacgaatac 2040
   ctcttcacaa gtagattttt taaatgagcg gataaagcgg gggtcccata gttcactagc 2100
   aactcctaag tctttgcagc atctcattaa agcattgctc ttacagcctt cagtagcagt 2160
   aggaattccc ttctctgaaa aaaaatcttg ctctccgcgt gcaatagaaa ctagtcggcc 2220
   ctgtacaatt aaagcatact ccctggttaa agtacctcct ccgaacttgc tcttgttgat 2280
   caaagtttct gaccttgggg ccagtcccca gccaccaggg ccaaacgctt tattgaggat 2340
   acgacgatac ttaatctctg gaagataaag tagtccatct ggtgtgattt cgacatcttc 2400
   gttgctaatt ggttgacata atatgttact actttcatta ctgaaggagc aaatacctag 2460
   tccatggaac gaatccgacc aattgattcc atcgccactt gtattagaga ttggggtgtc 2520
   gtttaactgt gaagttccaa acaaaattga taaactgctc tcgttcttag cttggccact 2580
   ttttggagtc tcaatagtag cgttttggct ctcgtgaatt ttctgcacag agtcggatga 2640
   agaaggtgca aatgcttcta gcattgtaga gtcgaccaca tagaaccttt ttaaagagtt 2700
   atgaaaataa ctcttggtag ggccaaatac aacccgatat cgtcttagca taagagctgc 2760
   ttctttggaa tatcgtttct tgtaagtaat tacgtgttgg ctaaacactt agaagtcagt 2820
   cgcgcatgcg gccaaaaaca gactagggat agaagatgaa ctgacaaaaa catcaagaag 2880
   gtgaagacat tcattctatg aaaactagtt tttatataaa attatggtct gcatttagag 2940
   agcaatgatg taatcaaaca tcaataagtg cttgtcgcat caatatttaa taggtaatca 3000
   tggagtattc tagtctaccg ccttaaaaaa agctcactcg atctagtgca gcttgattgt 3060
   gtacttcaat agtattccaa cgaccttaac atcttaacac catgtaaatt taagatccac 3120
   gtatacgata caatttcttt caatatcaat tctcgttcaa gccaactg atg ata aaa  3177
                                                        Met Ile Lys
                                                        1
   tca aga aag aga tcg aga aaa gtt tct ttg aac act gaa aag gag ctg   3225
   Ser Arg Lys Arg Ser Arg Lys Val Ser Leu Asn Thr Glu Lys Glu Leu
       5                   10                  15
   aaa aat agc cat att tct ctt gga gat gaa aga tgg tac act gtg ggt   3273
   Lys Asn Ser His Ile Ser Leu Gly Asp Glu Arg Trp Tyr Thr Val Gly
    20                 25                  30                  35
   ctt ctc ttg gtg aca atc aca gct ttc tgt act cga ttc tat gct atc   3321
   Leu Leu Leu Val Thr Ile Thr Ala Phe Cys Thr Arg Phe Tyr Ala Ile
                   40                  45                  50
   aac tat cca gat gag gtt gtt ttt gac gaa gtt cat ttc gga aaa ttt   3369
   Asn Tyr Pro Asp Glu Val Val Phe Asp Glu Val His Phe Gly Lys Phe
               55                  60                  65
   gct agc tac tat cta gag cgt act tat ttt ttt gat ctg cac cct ccg   3417
   Ala Ser Tyr Tyr Leu Glu Arg Thr Tyr Phe Phe Asp Leu His Pro Pro
           70                  75                  80
   ttt gcc aag ctc ctg att gcg ttt gtc ggc ttt tta gct ggg tac aat   3465
   Phe Ala Lys Leu Leu Ile Ala Phe Val Gly Phe Leu Ala Gly Tyr Asn
       85                  90                  95
   ggt gag ttc aag ttt aca act att ggt gaa tct tat atc aaa aac gag   3513
   Gly Glu Phe Lys Phe Thr Thr Ile Gly Glu Ser Tyr Ile Lys Asn Glu
   100                 105                 110                 115
   gtt ccc tac gta gtt tac aga tca ttg agc gct gtg caa gga tct tta   3561
   Val Pro Tyr Val Val Tyr Arg Ser Leu Ser Ala Val Gln Gly Ser Leu
                   120                 125                 130
   acg gtg cca att gtt tat ttg tgt ctc aaa gaa tgc gga tat aca gtt   3609
   Thr Val Pro Ile Val Tyr Leu Cys Leu Lys Glu Cys Gly Tyr Thr Val
               135                 140                 145
   ttg act tgt gtt ttt ggt gca tgt atc ata ttg ttt gat ggg gcc cac   3657
   Leu Thr Cys Val Phe Gly Ala Cys Ile Ile Leu Phe Asp Gly Ala His
           150                 155                 160
   gtt gct gag act aga cta atc ttg ctg gat gcc acg ttg att ttt ttc   3705
   Val Ala Glu Thr Arg Leu Ile Leu Leu Asp Ala Thr Leu Ile Phe Phe
       165                 170                 175
   gtt tca ttg tcc atc tat agc tat atc aaa ttc aca aaa caa aga tca   3753
   Val Ser Leu Ser Ile Tyr Ser Tyr Ile Lys Phe Thr Lys Gln Arg Ser
   180                 185                 190                 195
   gaa cca ttc ggc caa aag tgg tgg aag tgg ctg ttc ttt aca ggg gtg   3801
   Glu Pro Phe Gly Gln Lys Trp Trp Lys Trp Leu Phe Phe Thr Gly Val
                   200                 205                 210
   tct tta tct tgc gtc ata agt acc aag tat gtg ggg gtg ttc acc tat    3849
   Ser Leu Ser Cys Val Ile Ser Thr Lys Tyr Val Gly Val Phe Thr Tyr
               215                 220                 225
   ctt aca ata ggc tgt ggt gtc ctg ttt gac tta tgg agt tta ctg gat    3897
   Leu Thr Ile Gly Cys Gly Val Leu Phe Asp Leu Trp Ser Leu Leu Asp
           230                 235                 240
   tat aaa aag gga cat tcc ttg gca tat gtt ggt aaa cac ttt gct gca    3945
   Tyr Lys Lys Gly His Ser Leu Ala Tyr Val Gly Lys His Phe Ala Ala
       245                 250                 255
   cga ttt ttc ctt cta ata ctg gtc cct ttc ttg ata tat ctc aat tgg    3993
   Arg Phe Phe Leu Leu Ile Leu Val Pro Phe Leu Ile Tyr Leu Asn Trp
   260                 265                 270                 275
   ttt tat gtt cat ttc gct att cta agc aag tct ggc cca gga gac agt    4041
   Phe Tyr Val His Phe Ala Ile Leu Ser Lys Ser Gly Pro Gly Asp Ser
                   280                 285                 290
   ttt atg agc tct gaa ttc cag gag act ctc gga gat tct cct ctt gca    4089
   Phe Met Ser Ser Glu Phe Gln Glu Thr Leu Gly Asp Ser Pro Leu Ala
               295                 300                 305
   gct ttc gca aag gaa gtt cac ttt aac gac ata atc aca ata aag cat    4137
   Ala Phe Ala Lys Glu Val His Phe Asn Asp Ile Ile Thr Ile Lys His
           310                 315                 320
   aaa gag act gat gcc atg ttg cac tca cac ttg gca aac tac ccc ctc    4185
   Lys Glu Thr Asp Ala Met Leu His Ser His Leu Ala Asn Tyr Pro Leu
       325                 330                 335
   cgt tac gag gac ggg agg gta tca tct caa ggt caa caa gtt aca gca    4233
   Arg Tyr Glu Asp Gly Arg Val Ser Ser Gln Gly Gln Gln Val Thr Ala
   340                 345                 350                 355
   tac tct gga gag gac cca aac aat aat tgg cag att att tct ccc gaa    4281
   Tyr Ser Gly Glu Asp Pro Asn Asn Asn Trp Gln Ile Ile Ser Pro Glu
                   360                 365                 370
   gga ctt act ggc gtt gta act cag ggc gat gtc gtt aga ctg aga cac    4329
   Gly Leu Thr Gly Val Val Thr Gln Gly Asp Val Val Arg Leu Arg His
               375                 380                 385
   gtt ggg aca gat ggc tat cta ctg acg cat gat gtt gcg tct cct ttc    4377
   Val Gly Thr Asp Gly Tyr Leu Leu Thr His Asp Val Ala Ser Pro Phe
           390                 395                 400
   tat cca act aac gag gag ttt act gta gtg gga cag gag aaa gct act    4425
   Tyr Pro Thr Asn Glu Glu Phe Thr Val Val Gly Gln Glu Lys Ala Thr
       405                 410                 415
   caa cgc tgg aac gaa aca ctt ttt aga att gat ccc tat gac aag aag    4473
   Gln Arg Trp Asn Glu Thr Leu Phe Arg Ile Asp Pro Tyr Asp Lys Lys
   420                 425                 430                 435
   aaa acc cgt cct ttg aag tcg aaa gct tca ttt ttc aaa ctc att cat    4521
   Lys Thr Arg Pro Leu Lys Ser Lys Ala Ser Phe Phe Lys Leu Ile His
                   440                 445                 450
   gtt cct acg gtt gtg gcc atg tgg act cat aat gac cag ctt ctt cct    4569
   Val Pro Thr Val Val Ala Met Trp Thr His Asn Asp Gln Leu Leu Pro
               455                 460                 465
   gat tgg ggt ttc aac caa caa gaa gtc aat ggt aat aag aag ctt gct    4617
   Asp Trp Gly Phe Asn Gln Gln Glu Val Asn Gly Asn Lys Lys Leu Ala
           470                 475                 480
   gat gaa tca aac tta tgg gtt gta gac aat atc gtc gat att gca gag    4665
   Asp Glu Ser Asn Leu Trp Val Val Asp Asn Ile Val Asp Ile Ala Glu
       485                 490                 495
   gac gat cca agg aaa cac tac gtt cca aag gaa gtg aaa aat ttg cca    4713
   Asp Asp Pro Arg Lys His Tyr Val Pro Lys Glu Val Lys Asn Leu Pro
   500                 505                 510                 515
   ttt ttg acc aag tgg ttg gaa tta caa aga ctt atg ttt att cag aat    4761
   Phe Leu Thr Lys Trp Leu Glu Leu Gln Arg Leu Met Phe Ile Gln Asn
                   520                 525                 530
   aac aag ttg agc tca gat cat cca ttt gcg tct gac cct ata tct tgg    4809
   Asn Lys Leu Ser Ser Asp His Pro Phe Ala Ser Asp Pro Ile Ser Trp
               535                 540                 545
   cct ttt tca ctt agt ggg gtt tca ttt tgg aca aac aac gag tca cgc    4857
   Pro Phe Ser Leu Ser Gly Val Ser Phe Trp Thr Asn Asn Glu Ser Arg
           550                 555                 560
   aaa cag atc tat ttt gtc gga aat att cct gga tgg tgg atg gag gtt    4905
   Lys Gln Ile Tyr Phe Val Gly Asn Ile Pro Gly Trp Trp Met Glu Val
       565                 570                 575
   gca gca ttg gga tcc ttt cta gga ctc gtg ttt gca gat cag ttc acg    4953
   Ala Ala Leu Gly Ser Phe Leu Gly Leu Val Phe Ala Asp Gln Phe Thr
   580                 585                 590                 595
   aga aga aga aac agt ctt gtt ttg acc aat agc gcc agg tct cgg tta    5001
   Arg Arg Arg Asn Ser Leu Val Leu Thr Asn Ser Ala Arg Ser Arg Leu
                   600                 605                 610
   tac aat aat ttg ggg ttc ttc ttt gta ggc tgg tgt tgt cat tac cta    5049
   Tyr Asn Asn Leu Gly Phe Phe Phe Val Gly Trp Cys Cys His Tyr Leu
               615                 620                 625
   ccc ttt ttc cta atg agc cgt caa aaa ttt ttg cac cat tac tta cct    5097
   Pro Phe Phe Leu Met Ser Arg Gln Lys Phe Leu His His Tyr Leu Pro
           630                 635                 640
   gca cat tta ata gca gcc atg ttc act gct ggt ttc ttg gaa ttt att    5145
   Ala His Leu Ile Ala Ala Met Phe Thr Ala Gly Phe Leu Glu Phe Ile
       645                 650                 655
   ttt act gac aac aga act gaa gaa ttc aag gat cag aaa act tca tgt    5193
   Phe Thr Asp Asn Arg Thr Glu Glu Phe Lys Asp Gln Lys Thr Ser Cys
   660                 665                 670                 675
   gaa cct aac tct aat tct tca aag ccg aaa gag caa ttg att ctg tgg    5241
   Glu Pro Asn Ser Asn Ser Ser Lys Pro Lys Glu Gln Leu Ile Leu Trp
                   680                 685                 690
   tta agt ttc tcg tcc ttt gtc gct ttg cta cta agc atc att gtt tgg    5289
   Leu Ser Phe Ser Ser Phe Val Ala Leu Leu Leu Ser Ile Ile Val Trp
           695                 700                 705
   act ttc ttc ttt ttt gct cct cta aca tat ggt aat act gcg ctt tcg   5337
   Thr Phe Phe Phe Phe Ala Pro Leu Thr Tyr Gly Asn Thr Ala Leu Ser
           710                 715                 720
   gcg gag gag gtt cag cag cga caa tgg tta gat atg aag ctc caa ttc   5385
   Ala Glu Glu Val Gln Gln Arg Gln Trp Leu Asp Met Lys Leu Gln Phe
       725                 730                 735
   gcc aag taa gagtatacaa tgtgtagttc aacgcaaagg aaattctaac           5434
   Ala Lys  *
   740
   tttctgtgca atctggtgac aatttctaaa taactatcac aattggaaga agagattatc 5494
   ccaaatctta tcaaaaaatc gatgattgcc agtgcacaat taggcttgaa tttttcttgc 5554
   agcaacgaag agattacttc agtgatgttc attagcctga aatcttcact ttcgtggtct 5614
   atcggattag gaattagacc ttgtttcatc ggcaggtcgt atatgtattc cacttctggt 5674
   tgaataaaat cttcgggtgg tttgtttctg aacatatatg agatggctcc cactggactg 5734
   atatattgcg aaacatagtc ctcattcaac cctgcctcct cgtaacattc tttcaggcaa 5794
   gtttgcaaag tgccattagg atattccaag cctcctgcca cagtattatc taacataccg 5854
   ggaaatgttg gtttgtgtct gcttctccta ggtatccaaa gttgaatact gttaggatcg 5914
   gcagaatttt gcaaatatcc attgatatga actccataag taacaactcc caaaatatta 5974
   gaaaaagccc tttccaccaa catgtacatc ttatggttat cgcagtaaac tgcaaaaagc 6034
   tcatttctcc aaccgctaag ggtttcaaag agacgctgat ctctccaacg ctgagctatc 6094
   tttgcaaaca tctgcgttct tttattttcg gtatccagac taggaattat cttgacttcg 6154
   tgtttttcat tatttactat cacagcctgt gtttcgaact caaattgttt tgccaccttg 6214
   ggaattatat accctagtaa gatcccatca tgcgataaga atttatacac agatacttca 6274
   aattcatgaa aagatggctc atctttatga ggaacagaat caacagatct gactagatca 6334
   atatatggca ttggttgatt ttattcaatg gttatctatc tcaaacatgc tataaaaata 6394
   aggtaattcc tttatggtgt tagggtgtta tagtttttgc gtagaaaata attgtcatca 6454
   tttttgggca acctatgaaa caactactca gagaagttga gacatctctt ttgacaaatg 6514
   aaaccgaaat atcccctgcc cttaagctat taattactca gttaaataaa tcaacccatg 6574
   aagataaatc aacagaaaga aaaacgtttt ggctagcatt agacaattta aggcaaaaaa 6634
   tcggtctaca atcccaatca catgtccttt tctttctaca tctttttgaa gagctagctc 6694
   caactttaga aaatgagaaa atatttttaa cctggattac ttcttttttg aagttagcaa 6754
   ttaatagtgc aggggtacca cattgtgtgg tgaacgagtc aaggagaatt ataatgaatt 6814
   tattattgcc ctcaaaagct acaaacaccg aatacaattt gttaaagaat tctgctgcag 6874
   gcattcaatt acttgtgcaa gtgtatttgc taaaaactga tttagttgtt gattccactt 6934
   ctagtagtcc ccaggagtat gaagagaggg ttagattcat aaagaaaaac tgcagggatt 6994
   tactacaagg tcttgattta aataatcaag tactagaggc tatcagcaaa gaatttacgg 7054
   atcctcacta ccgcttcgag tgcttcgtac ttttgtcctc attaatgtcg tcatcagcct 7114
   tgttgtacca gataatgcaa acaactttgt ggcataatat acttttgtct atattgatag 7174
   ataaaagtaa cagtgtggtt gagtcaggaa tcaaggttct cagtatggtt ttgccccacg 7234
   tctgtgatgt aatagcggat tatctaccga ccattatggc gattttaagt aaaggtctgg 7294
   ggggtgttga aattgatgat gagtcaccat taccatcaaa ttggaaagta ttgaatgatc 7354
   aggatcctga aattattggt ccagcatttg ttagctataa acaactgttc actgtattat 7414
   acggcctgtt ccctcttagt ttaacatcat ttattcgcag tccatctaca tatatcgact 7474
   ctaacaagat tatagacgat ctcaagcttc agttgcttga aactaaagtg aagtcaaagt 7534
   gtcaggactt gctaaagtgt tttattgttc atccaaatta ttttatatat tcttcccagg 7594
   aggaagaaat tttgatactt tcaaggtggg acaaaatgca ctccccgaac gagatagcag 7654
   cattttgtta tcaattggaa ttccgtggga catcgaagga gaatgccttt gatatgaggg 7714
   tagatgacct tttggaaggt catcgatatc tatatttgaa agatatgaag gatgcgcaga 7774
   aagagagggc taaaaaatgt gaaaattcta ttatctcact cgaaagttca tctgatagta 7834
   agtcagtttc acaatacgac gaagactcga cgaaagaaac cacttgcagg catgtttcgt 7894
   tttatttaag agagatcctt ttggcaaaaa atgaattgga cttcacgcta catatcaatc 7954
   aggtacttgg agccgagtgt gagcttttga aaaaaaaatt gaacgaaatg gataccctac 8014
   gagatcaaaa caggttttta gctgacataa acgaaggttt acgaatacag caatctaagg 8074
   cgagtgagca aattacggaa ttgctcaaag aaaaagagcg ttctcaaaat gatttcaact 8134
   ctctggttac tcatatgctt aaacaatcta acgaattaaa agaaagggag tcgaaactag 8194
   tcgagattca tcaatcaaat gatgcagaga taggagattt aaattatagg ttggaaaaac 8254
   tgtgcaacct tatacaaccc aaagaattag aagtggaact gctcaagaag aagttgcgtg 8314
   tagcatcgat ccttttttcg caagataaat caaaatcttc aagcaagaca tctctagcac 8374
   atttgcacca ggcaggcgac gcaact 8400
<210> 50
   <211> 741
   <212> PRT
   <213> Pichia pastoris
<400> 50
   Met Ile Lys Ser Arg Lys Arg Ser Arg Lys Val Ser Leu Asn Thr Glu
    1               5                  10                  15
   Lys Glu Leu Lys Asn Ser His Ile Ser Leu Gly Asp Glu Arg Trp Tyr
               20                  25                  30
   Thr Val Gly Leu Leu Leu Val Thr Ile Thr Ala Phe Cys Thr Arg Phe
           35                  40                  45
   Tyr Ala Ile Asn Tyr Pro Asp Glu Val Val Phe Asp Glu Val His Phe
       50                  55                  60
   Gly Lys Phe Ala Ser Tyr Tyr Leu Glu Arg Thr Tyr Phe Phe Asp Leu
   65                  70                  75                  80
   His Pro Pro Phe Ala Lys Leu Leu Ile Ala Phe Val Gly Phe Leu Ala
                   85                  90                  95
   Gly Tyr Asn Gly Glu Phe Lys Phe Thr Thr Ile Gly Glu Ser Tyr Ile
               100                 105                 110
   Lys Asn Glu Val Pro Tyr Val Val Tyr Arg Ser Leu Ser Ala Val Gln
           115                 120                 125
   Gly Ser Leu Thr Val Pro Ile Val Tyr Leu Cys Leu Lys Glu Cys Gly
       130                 135                 140
   Tyr Thr Val Leu Thr Cys Val Phe Gly Ala Cys Ile Ile Leu Phe Asp
   145                 150                 155                 160
   Gly Ala His Val Ala Glu Thr Arg Leu Ile Leu Leu Asp Ala Thr Leu
                   165                 170                 175
   Ile Phe Phe Val Ser Leu Ser Ile Tyr Ser Tyr Ile Lys Phe Thr Lys
               180                 185                 190
   Gln Arg Ser Glu Pro Phe Gly Gln Lys Trp Trp Lys Trp Leu Phe Phe
           195                 200                 205
   Thr Gly Val Ser Leu Ser Cys Val Ile Ser Thr Lys Tyr Val Gly Val
       210                 215                 220
   Phe Thr Tyr Leu Thr Ile Gly Cys Gly Val Leu Phe Asp Leu Trp Ser
   225                 230                 235                 240
   Leu Leu Asp Tyr Lys Lys Gly His Ser Leu Ala Tyr Val Gly Lys His
                   245                 250                 255
   Phe Ala Ala Arg Phe Phe Leu Leu Ile Leu Val Pro Phe Leu Ile Tyr
               260                 265                 270
   Leu Asn Trp Phe Tyr Val His Phe Ala Ile Leu Ser Lys Ser Gly Pro
           275                 280                 285
   Gly Asp Ser Phe Met Ser Ser Glu Phe Gln Glu Thr Leu Gly Asp Ser
       290                 295                 300
   Pro Leu Ala Ala Phe Ala Lys Glu Val His Phe Asn Asp Ile Ile Thr
   305                 310                 315                 320
   Ile Lys His Lys Glu Thr Asp Ala Met Leu His Ser His Leu Ala Asn
                   325                 330                 335
   Tyr Pro Leu Arg Tyr Glu Asp Gly Arg Val Ser Ser Gln Gly Gln Gln
               340                 345                 350
   Val Thr Ala Tyr Ser Gly Glu Asp Pro Asn Asn Asn Trp Gln Ile Ile
           355                 360                 365
   Ser Pro Glu Gly Leu Thr Gly Val Val Thr Gln Gly Asp Val Val Arg
       370                 375                 380
   Leu Arg His Val Gly Thr Asp Gly Tyr Leu Leu Thr His Asp Val Ala
   385                 390                 395                 400
   Ser Pro Phe Tyr Pro Thr Asn Glu Glu Phe Thr Val Val Gly Gln Glu
                       405                 410                 415
   Lys Ala Thr Gln Arg Trp Asn Glu Thr Leu Phe Arg Ile Asp Pro Tyr
                   420                 425                 430
   Asp Lys Lys Lys Thr Arg Pro Leu Lys Ser Lys Ala Ser Phe Phe Lys
               435                 440                 445
   Leu Ile His Val Pro Thr Val Val Ala Met Trp Thr His Asn Asp Gln
           450                 455                 460
   Leu Leu Pro Asp Trp Gly Phe Asn Gln Gln Glu Val Asn Gly Asn Lys
   465                 470                 475                 480
   Lys Leu Ala Asp Glu Ser Asn Leu Trp Val Val Asp Asn Ile Val Asp
                   485                 490                 495
   Ile Ala Glu Asp Asp Pro Arg Lys His Tyr Val Pro Lys Glu Val Lys
               500                 505                 510
   Asn Leu Pro Phe Leu Thr Lys Trp Leu Glu Leu Gln Arg Leu Met Phe
           515                 520                 525
   Ile Gln Asn Asn Lys Leu Ser Ser Asp His Pro Phe Ala Ser Asp Pro
       530                 535                 540
   Ile Ser Trp Pro Phe Ser Leu Ser Gly Val Ser Phe Trp Thr Asn Asn
   545                 550                 555                 560
   Glu Ser Arg Lys Gln Ile Tyr Phe Val Gly Asn Ile Pro Gly Trp Trp
                   565                 570                 575
   Met Glu Val Ala Ala Leu Gly Ser Phe Leu Gly Leu Val Phe Ala Asp
               580                 585                 590
   Gln Phe Thr Arg Arg Arg Asn Ser Leu Val Leu Thr Asn Ser Ala Arg
           595                 600                 605
   Ser Arg Leu Tyr Asn Asn Leu Gly Phe Phe Phe Val Gly Trp Cys Cys
       610                 615                 620
   His Tyr Leu Pro Phe Phe Leu Met Ser Arg Gln Lys Phe Leu His His
   625                 630                 635                 640
   Tyr Leu Pro Ala His Leu Ile Ala Ala Met Phe Thr Ala Gly Phe Leu
                   645                 650                 655
   Glu Phe Ile Phe Thr Asp Asn Arg Thr Glu Glu Phe Lys Asp Gln Lys
               660                 665                 670
   Thr Ser Cys Glu Pro Asn Ser Asn Ser Ser Lys Pro Lys Glu Gln Leu
           675                 680                 685
   Ile Leu Trp Leu Ser Phe Ser Ser Phe Val Ala Leu Leu Leu Ser Ile
       690                 695                 700
   Ile Val Trp Thr Phe Phe Phe Phe Ala Pro Leu Thr Tyr Gly Asn Thr
   705                 710                 715                 720
   Ala Leu Ser Ala Glu Glu Val Gln Gln Arg Gln Trp Leu Asp Met Lys
                   725                 730                 735
   Leu Gln Phe Ala Lys
               740
<210> 51
   <211> 1380
   <212> DNA
   <213> Artificial Sequence
<220>
   <223> anti-DKK1 Heavy chain (VH + IgG2m4) with alpha-amylase leader
<400> 51
   acgatggtcg cttggtggtc tttgtttctg tacggtcttc aggtcgctgc acctgctttg 60
   gctgaggttc agttggttca atctggtgct gaggttaaga aacctggtgc ttccgttaag 120
   gtttcctgta aggcttccgg ttacactttc actgactact acatccactg ggttagacaa 180
   gctccaggtc aaggattgga atggatggga tggattcact ctaactccgg tgctactact 240
   tacgctcaga agttccaggc tagagttact atgtccagag acacttcttc ttccactgct 300
   tacatggaat tgtccagatt ggaatccgat gacactgcta tgtacttttg ttccagagag 360
   gactactggg gacagggaac tttggttact gtttcctccg cttctactaa agggccctct 420
   gtttttccat tggctccatg ttctagatcc acttccgaat ccactgctgc tttgggatgt 480
   ttggttaagg actacttccc agagccagtt actgtttctt ggaactccgg tgctttgact 540
   tctggtgttc acactttccc agctgttttg caatcttccg gtttgtactc cttgtcctcc 600
   gttgttactg ttacttcctc caacttcggt actcagactt acacttgtaa cgttgaccac 660
   aagccatcca acactaaggt tgacaagact gttgagagaa agtgttgtgt tgagtgtcca 720
   ccatgtccag ctccaccagt tgctggtcca tccgtttttt tgttcccacc aaagccaaag 780
   gacactttga tgatctccag aactccagag gttacatgtg ttgttgttga cgtttcccaa 840
   gaggacccag aggttcaatt caactggtac gttgacggtg ttgaagttca caacgctaag 900
   actaagccaa gagaagagca gttcaactcc actttcagag ttgtttccgt tttgactgtt 960
   ttgcaccagg attggttgaa cggtaaagaa tacaagtgta aggtttccaa caagggattg 1020
   ccatcctcca tcgaaaagac tatctccaag actaagggac aaccaagaga gccacaggtt 1080
   tacactttgc caccatccag agaagagatg actaagaacc aggtttcctt gacttgtttg 1140
   gttaaaggat tctacccatc cgacattgct gttgagtggg aatctaacgg tcaaccagag 1200
   aacaactaca agactactcc accaatgttg gattctgacg gttccttctt cttgtactcc 1260
   aagttgactg ttgacaagtc cagatggcaa cagggtaacg ttttctcctg ttccgttatg 1320
   catgaggctt tgcacaacca ctacactcaa aagtccttgt ctttgtcccc tggtaagtaa 1380
<210> 52
   <211> 438
   <212> PRT
   <213> Artificial Sequence
<220>
   <223> anti-DKK1 Heavy chain (VH + IgG2m4)
<400> 52
   Glu Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Asp Tyr
               20                  25                  30
   Tyr Ile His Trp Val Arg Gln Ala Pro Gly Gln Gly Leu Glu Trp Met
           35                  40                  45
   Gly Trp Ile His Ser Asn Ser Gly Ala Thr Thr Tyr Ala Gln Lys Phe
       50                  55                  60
   Gln Ala Arg Val Thr Met Ser Arg Asp Thr Ser Ser Ser Thr Ala Tyr
   65                  70                  75                  80
   Met Glu Leu Ser Arg Leu Glu Ser Asp Asp Thr Ala Met Tyr Phe Cys
                   85                  90                  95
   Ser Arg Glu Asp Tyr Trp Gly Gln Gly Thr Leu Val Thr Val Ser Ser
               100                 105                 110
   Ala Ser Thr Lys Gly Pro Ser Val Phe Pro Leu Ala Pro Cys Ser Arg
           115                 120                 125
   Ser Thr Ser Glu Ser Thr Ala Ala Leu Gly Cys Leu Val Lys Asp Tyr
       130                 135                 140
   Phe Pro Glu Pro Val Thr Val Ser Trp Asn Ser Gly Ala Leu Thr Ser
   145                 150                 155                 160
   Gly Val His Thr Phe Pro Ala Val Leu Gln Ser Ser Gly Leu Tyr Ser
                   165                 170                 175
   Leu Ser Ser Val Val Thr Val Thr Ser Ser Asn Phe Gly Thr Gln Thr
               180                 185                 190
   Tyr Thr Cys Asn Val Asp His Lys Pro Ser Asn Thr Lys Val Asp Lys
           195                 200                 205
   Thr Val Glu Arg Lys Cys Cys Val Glu Cys Pro Pro Cys Pro Ala Pro
       210                 215                 220
   Pro Val Ala Gly Pro Ser Val Phe Leu Phe Pro Pro Lys Pro Lys Asp
   225                 230                 235                 240
   Thr Leu Met Ile Ser Arg Thr Pro Glu Val Thr Cys Val Val Val Asp
                   245                 250                 255
   Val Ser Gln Glu Asp Pro Glu Val Gln Phe Asn Trp Tyr Val Asp Gly
               260                 265                 270
   Val Glu Val His Asn Ala Lys Thr Lys Pro Arg Glu Glu Gln Phe Asn
           275                 280                 285
   Ser Thr Phe Arg Val Val Ser Val Leu Thr Val Leu His Gln Asp Trp
       290                 295                 300
   Leu Asn Gly Lys Glu Tyr Lys Cys Lys Val Ser Asn Lys Gly Leu Pro
   305                 310                 315                 320
   Ser Ser Ile Glu Lys Thr Ile Ser Lys Thr Lys Gly Gln Pro Arg Glu
                   325                 330                 335
   Pro Gln Val Tyr Thr Leu Pro Pro Ser Arg Glu Glu Met Thr Lys Asn
               340                 345                 350
   Gln Val Ser Leu Thr Cys Leu Val Lys Gly Phe Tyr Pro Ser Asp Ile
           355                 360                 365
   Ala Val Glu Trp Glu Ser Asn Gly Gln Pro Glu Asn Asn Tyr Lys Thr
       370                 375                 380
   Thr Pro Pro Met Leu Asp Ser Asp Gly Ser Phe Phe Leu Tyr Ser Lys
   385                 390                 395                 400
   Leu Thr Val Asp Lys Ser Arg Trp Gln Gln Gly Asn Val Phe Ser Cys
                   405                 410                 415
   Ser Val Met His Glu Ala Leu His Asn His Tyr Thr Gln Lys Ser Leu
               420                 425                 430
   Ser Leu Ser Pro Gly Lys
           435
<210> 53
   <211> 717
   <212> DNA
   <213> Artificial Sequence
<220>
   <223> aanti-DKK1 Light chain (VL + lambda constant regions) with alpha-amylase leader
<400> 53
   acgatggtcg cttggtggtc tttgtttctg tacggtcttc aggtcgctgc acctgctttg 60
   gctcagtccg ttttgacaca accaccatct gtttctggtg ctccaggaca gagagttact 120
   atctcctgta ctggttcctc ttccaacatt ggtgctggtt acgatgttca ctggtatcaa 180
   cagttgccag gtactgctcc aaagttgttg atctacggtt actccaacag accatctggt 240
   gttccagaca gattctctgg ttctaagtct ggtgcttctg cttccttggc tatcactgga 300
   ttgagaccag atgacgaggc tgactactac tgtcaatcct acgacaactc cttgtcctct 360
   tacgttttcg gtggtggtac tcagttgact gttttgtccc agccaaaggc taatccaact 420
   gttactttgt tcccaccatc ttccgaagaa ctgcaggcta ataaggctac tttggtttgt 480
   ttgatctccg acttctaccc aggtgctgtt actgttgctt ggaaggctga tggttctcca 540
   gttaaggctg gtgttgagac tactaagcca tccaagcagt ccaataacaa gtacgctgct 600
   agctcttact tgtccttgac accagaacaa tggaagtccc acagatccta ctcttgtcag 660
   gttacacacg agggttctac tgttgaaaag actgttgctc caactgagtg ttcctaa    717
<210> 54
   <211> 217
   <212> PRT
   <213> Artificial Sequence
<220>
   <223> aanti-DKK1 Light chain (VL + lambda constant regions)
<400> 54
   Gln Ser Val Leu Thr Gln Pro Pro Ser Val Ser Gly Ala Pro Gly Gln
    1               5                  10                  15
   Arg Val Thr Ile Ser Cys Thr Gly Ser Ser Ser Asn Ile Gly Ala Gly
                   20                  25                  30
   Tyr Asp Val His Trp Tyr Gln Gln Leu Pro Gly Thr Ala Pro Lys Leu
               35                  40                  45
   Leu Ile Tyr Gly Tyr Ser Asn Arg Pro Ser Gly Val Pro Asp Arg Phe
       50                  55                  60
   Ser Gly Ser Lys Ser Gly Ala Ser Ala Ser Leu Ala Ile Thr Gly Leu
   65                  70                  75                  80
   Arg Pro Asp Asp Glu Ala Asp Tyr Tyr Cys Gln Ser Tyr Asp Asn Ser
                   85                  90                  95
   Leu Ser Ser Tyr Val Phe Gly Gly Gly Thr Gln Leu Thr Val Leu Ser
               100                 105                 110
   Gln Pro Lys Ala Asn Pro Thr Val Thr Leu Phe Pro Pro Ser Ser Glu
           115                 120                 125
   Glu Leu Gln Ala Asn Lys Ala Thr Leu Val Cys Leu Ile Ser Asp Phe
       130                 135                 140
   Tyr Pro Gly Ala Val Thr Val Ala Trp Lys Ala Asp Gly Ser Pro Val
   145                 150                 155                 160
   Lys Ala Gly Val Glu Thr Thr Lys Pro Ser Lys Gln Ser Asn Asn Lys
                   165                 170                 175
   Tyr Ala Ala Ser Ser Tyr Leu Ser Leu Thr Pro Glu Gln Trp Lys Ser
               180                 185                 190
   His Arg Ser Tyr Ser Cys Gln Val Thr His Glu Gly Ser Thr Val Glu
           195                 200                 205
   Lys Thr Val Ala Pro Thr Glu Cys Ser
       210                 215
<210> 55
   <211> 1908
   <212> DNA
   <213> Artificial Sequence
<220>
   <223> encodes human BIP
<400> 55
   gaggaagagg acaagaaaga ggatgttggt actgttgtcg gtatcgactt gggtactacc 60
   tactcctgtg tcggtgtttt caagaacggt agagtggaga ttatcgccaa cgaccagggt 120
   aacagaatta ctccatccta cgttgctttt accccagaag gagagagatt gatcggagac 180
   gctgctaaga accaattgac ctccaaccca gagaacactg ttttcgacgc caagagactg 240
   attggtagaa cttggaacga cccatccgtt caacaagaca tcaagttctt gcccttcaag 300
   gtcgtcgaga agaaaaccaa gccatacatc caggttgaca tcggtggtgg tcaaactaag 360
   actttcgctc cagaggaaat ctccgctatg gtcctgacta agatgaaaga gactgccgag 420
   gcttacttgg gtaaaaaggt tacccacgct gttgttactg ttccagctta cttcaacgac 480
   gctcagagac aagctactaa ggacgctggt actatcgctg gactgaacgt gatgagaatc 540
   atcaacgagc caactgctgc tgctattgcc tacggattgg acaagagaga gggagagaag 600
   aacatcttgg ttttcgactt gggtggtggt actttcgacg tttccttgtt gaccatcgac 660
   aacggtgttt tcgaagttgt tgctaccaac ggtgatactc acttgggtgg agaggacttc 720
   gatcagagag tgatggaaca cttcatcaag ctgtacaaga agaaaaccgg aaaggacgtt 780
   agaaaggaca acagagccgt tcagaagttg agaagagagg ttgagaaggc taaggctttg 840
   tcctcccaac accaagctag aatcgagatc gaatccttct acgagggtga agatttctcc 900
   gagaccttga ctagagccaa gttcgaagag ctgaacatgg acctgttcag atccactatg 960
   aagccagttc agaaggtttt ggaggattcc gacttgaaga agtccgacat cgacgagatt 1020
   gttttggttg gtggttccac cagaatccca aagatccagc agctggtcaa agagttcttc 1080
   aacggtaaag agccatccag aggtattaac ccagatgagg ctgttgctta cggtgctgct 1140
   gttcaagctg gtgttttgtc tggtgaccag gacactggtg acttggtttt gttgcatgtt 1200
   tgcccattga ctttgggtat cgagactgtt ggtggtgtta tgaccaagtt gatcccatcc 1260
   aacactgttg ttcccaccaa gaactcccaa attttctcca ctgcttccga caaccagcca 1320
   accgttacta ttaaggtcta cgaaggtgaa agaccattga ccaaggacaa ccacttgttg 1380
   ggaactttcg acttgactgg tattccacct gctccaagag gtgttccaca aatcgaggtt 1440
   accttcgaga tcgacgtcaa cggtatcttg agagttactg ccgaggataa gggaaccggt 1500
   aacaagaaca agatcaccat caccaacgac caaaacagat tgacccccga agagatcgaa 1560
   agaatggtca acgatgctga gaagttcgcc gaagaggata agaagctgaa agagagaatc 1620
   gacaccagaa acgagttgga atcctacgct tactccttga agaaccagat cggtgacaaa 1680
   gaaaagttgg gtggaaagct gtcatccgaa gataaagaaa ctatggaaaa ggccgtcgaa 1740
   gaaaagattg agtggctgga atctcaccaa gatgctgaca tcgaggactt caaggccaag 1800
   aagaaagagt tggaagagat cgtccagcca atcatttcta agttgtacgg ttctgctggt 1860
   ccaccaccaa ctggtgaaga agatactgcc gagcacgacg agttgtag              1908
<210> 56
   <211> 635
   <212> PRT
   <213> Artificial Sequence
<220>
   <223> human BIP without leader
<400> 56
   Glu Glu Glu Asp Lys Lys Glu Asp Val Gly Thr Val Val Gly Ile Asp
   1               5                   10                  15
   Leu Gly Thr Thr Tyr Ser Cys Val Gly Val Phe Lys Asn Gly Arg Val
               20                  25                  30
   Glu Ile Ile Ala Asn Asp Gln Gly Asn Arg Ile Thr Pro Ser Tyr Val
           35                  40                  45
   Ala Phe Thr Pro Glu Gly Glu Arg Leu Ile Gly Asp Ala Ala Lys Asn
       50                  55                  60
   Gln Leu Thr Ser Asn Pro Glu Asn Thr Val Phe Asp Ala Lys Arg Leu
   65                  70                  75                  80
   Ile Gly Arg Thr Trp Asn Asp Pro Ser Val Gln Gln Asp Ile Lys Phe
                   85                  90                  95
   Leu Pro Phe Lys Val Val Glu Lys Lys Thr Lys Pro Tyr Ile Gln Val
               100                 105                 110
   Asp Ile Gly Gly Gly Gln Thr Lys Thr Phe Ala Pro Glu Glu Ile Ser
           115                 120                 125
   Ala Met Val Leu Thr Lys Met Lys Glu Thr Ala Glu Ala Tyr Leu Gly
       130                 135                 140
   Lys Lys Val Thr His Ala Val Val Thr Val Pro Ala Tyr Phe Asn Asp
   145                 150                 155                 160
   Ala Gln Arg Gln Ala Thr Lys Asp Ala Gly Thr Ile Ala Gly Leu Asn
                   165                 170                 175
   Val Met Arg Ile Ile Asn Glu Pro Thr Ala Ala Ala Ile Ala Tyr Gly
               180                 185                 190
   Leu Asp Lys Arg Glu Gly Glu Lys Asn Ile Leu Val Phe Asp Leu Gly
           195                 200                 205
   Gly Gly Thr Phe Asp Val Ser Leu Leu Thr Ile Asp Asn Gly Val Phe
       210                 215                 220
   Glu Val Val Ala Thr Asn Gly Asp Thr His Leu Gly Gly Glu Asp Phe
   225                 230                 235                 240
   Asp Gln Arg Val Met Glu His Phe Ile Lys Leu Tyr Lys Lys Lys Thr
                   245                 250                 255
   Gly Lys Asp Val Arg Lys Asp Asn Arg Ala Val Gln Lys Leu Arg Arg
               260                 265                 270
   Glu Val Glu Lys Ala Lys Ala Leu Ser Ser Gln His Gln Ala Arg Ile
           275                 280                 285
   Glu Ile Glu Ser Phe Tyr Glu Gly Glu Asp Phe Ser Glu Thr Leu Thr
       290                 295                 300
   Arg Ala Lys Phe Glu Glu Leu Asn Met Asp Leu Phe Arg Ser Thr Met
   305                 310                 315                 320
   Lys Pro Val Gln Lys Val Leu Glu Asp Ser Asp Leu Lys Lys Ser Asp
                   325                 330                 335
   Ile Asp Glu Ile Val Leu Val Gly Gly Ser Thr Arg Ile Pro Lys Ile
               340                 345                 350
   Gln Gln Leu Val Lys Glu Phe Phe Asn Gly Lys Glu Pro Ser Arg Gly
           355                 360                 365
   Ile Asn Pro Asp Glu Ala Val Ala Tyr Gly Ala Ala Val Gln Ala Gly
       370                 375                 380
   Val Leu Ser Gly Asp Gln Asp Thr Gly Asp Leu Val Leu Leu His Val
   385                 390                 395                 400
   Cys Pro Leu Thr Leu Gly Ile Glu Thr Val Gly Gly Val Met Thr Lys
                   405                 410                 415
   Leu Ile Pro Ser Asn Thr Val Val Pro Thr Lys Asn Ser Gln Ile Phe
               420                 425                 430
   Ser Thr Ala Ser Asp Asn Gln Pro Thr Val Thr Ile Lys Val Tyr Glu
           435                 440                 445
   Gly Glu Arg Pro Leu Thr Lys Asp Asn His Leu Leu Gly Thr Phe Asp
       450                 455                 460
   Leu Thr Gly Ile Pro Pro Ala Pro Arg Gly Val Pro Gln Ile Glu Val
   465                 470                 475                 480
   Thr Phe Glu Ile Asp Val Asn Gly Ile Leu Arg Val Thr Ala Glu Asp
                   485                 490                 495
   Lys Gly Thr Gly Asn Lys Asn Lys Ile Thr Ile Thr Asn Asp Gln Asn
               500                 505                 510
   Arg Leu Thr Pro Glu Glu Ile Glu Arg Met Val Asn Asp Ala Glu Lys
           515                 520                 525
   Phe Ala Glu Glu Asp Lys Lys Leu Lys Glu Arg Ile Asp Thr Arg Asn
       530                 535                 540
   Glu Leu Glu Ser Tyr Ala Tyr Ser Leu Lys Asn Gln Ile Gly Asp Lys
   545                 550                 555                 560
   Glu Lys Leu Gly Gly Lys Leu Ser Ser Glu Asp Lys Glu Thr Met Glu
                   565                 570                 575
   Lys Ala Val Glu Glu Lys Ile Glu Trp Leu Glu Ser His Gln Asp Ala
               580                 585                 590
   Asp Ile Glu Asp Phe Lys Ala Lys Lys Lys Glu Leu Glu Glu Ile Val
           595                 600                 605
   Gln Pro Ile Ile Ser Lys Leu Tyr Gly Ser Ala Gly Pro Pro Pro Thr
       610                 615                 620
   Gly Glu Glu Asp Thr Ala Glu His Asp Glu Leu
   625                 630                 635
<210> 57
   <211> 1896
   <212> DNA
   <213> Artificial Sequence
<220>
   <223> chimeric BIP
<400> 57
   gacgatgtcg aatcttatgg aacagtgatt ggtatcgatt tgggtaccac gtactcttgt 60
   gtcggtgtga tgaagtcggg tcgtgtagaa attcttgcta atgaccaagg taacagaatc 120
   actccttcct acgttagttt cactgaagac gagagactgg ttggtgatgc tgctaagaac 180
   ttagctgctt ctaacccaaa aaacaccatc tttgatatta agagattgat cggtatgaag 240
   tatgatgccc cagaggtcca aagagacttg aagcgtcttc cttacactgt caagagcaag 300
   aacggccaac ctgtcgtttc tgtcgagtac aagggtgagg agaagtcttt cactcctgag 360
   gagatttccg ccatggtctt gggtaagatg aagttgatcg ctgaggacta cttaggaaag 420
   aaagtcactc atgctgtcgt taccgttcca gcctacttca acgacgctca acgtcaagcc 480
   actaaggatg ccggtctcat cgccggtttg actgttctga gaattgtgaa cgagcctacc 540
   gccgctgccc ttgcttacgg tttggacaag actggtgagg aaagacagat catcgtctac 600
   gacttgggtg gaggaacctt cgatgtttct ctgctttcta ttgagggtgg tgctttcgag 660
   gttcttgcta ccgccggtga cacccacttg ggtggtgagg actttgacta cagagttgtt 720
   cgccacttcg ttaagatttt caagaagaag cataacattg acatcagcaa caatgataag 780
   gctttaggta agctgaagag agaggtcgaa aaggccaagc gtactttgtc ttcccagatg 840
   actaccagaa ttgagattga ctctttcgtc gacggtatcg acttctctga gcaactgtct 900
   agagctaagt ttgaggagat caacattgaa ttattcaaga agacactgaa accagttgaa 960
   caagtcctca aagacgctgg tgtcaagaaa tctgaaattg atgacattgt cttggttggt 1020
   ggttctacca gaattccaaa ggttcaacaa ttattggagg attactttga cggaaagaag 1080
   gcttctaagg gaattaaccc agatgaagct gtcgcatacg gtgctgctgt tcaggctggt 1140
   gttttgtctg gtgatcaaga tacaggtgac ctggtactgc ttgatgtatg tccccttaca 1200
   cttggtattg aaactgtggg aggtgtcatg accaaactga ttccaaggaa cacagtggtg 1260
   cctaccaaga agtctcagat cttttctaca gcttctgata atcaaccaac tgttacaatc 1320
   aaggtctatg aaggtgaaag acccctgaca aaagacaatc atcttctggg tacatttgat 1380
   ctgactggaa ttcctcctgc tcctcgtggg gtcccacaga ttgaagtcac ctttgagata 1440
   gatgtgaatg gtattcttcg agtgacagct gaagacaagg gtacagggaa caaaaataag 1500
   atcacaatca ccaatgacca gaatcgcctg acacctgaag aaatcgaaag gatggttaat 1560
   gatgctgaga agtttgctga ggaagacaaa aagctcaagg agcgcattga tactagaaat 1620
   gagttggaaa gctatgccta ttctctaaag aatcagattg gagataaaga aaagctggga 1680
   ggtaaacttt cctctgaaga taaggagacc atggaaaaag ctgtagaaga aaagattgaa 1740
   tggctggaaa gccaccaaga tgctgacatt gaagacttca aagctaagaa gaaggaactg 1800
   gaagaaattg ttcaaccaat tatcagcaaa ctctatggaa gtgcaggccc tcccccaact 1860
   ggtgaagagg atacagcaga acatgatgag ttgtag 1896
<210> 58
   <211> 631
   <212> PRT
   <213> Artificial Sequence
<220>
   <223> chimeric BIP
<400> 58
   Asp Asp Val Glu Ser Tyr Gly Thr Val Ile Gly Ile Asp Leu Gly Thr
    1               5                  10                  15
   Thr Tyr Ser Cys Val Gly Val Met Lys Ser Gly Arg Val Glu Ile Leu
               20                  25                  30
   Ala Asn Asp Gln Gly Asn Arg Ile Thr Pro Ser Tyr Val Ser Phe Thr
           35                  40                  45
   Glu Asp Glu Arg Leu Val Gly Asp Ala Ala Lys Asn Leu Ala Ala Ser
       50                  55                  60
   Asn Pro Lys Asn Thr Ile Phe Asp Ile Lys Arg Leu Ile Gly Met Lys
   65                  70                  75                  80
   Tyr Asp Ala Pro Glu Val Gln Arg Asp Leu Lys Arg Leu Pro Tyr Thr
                   85                  90                  95
   Val Lys Ser Lys Asn Gly Gln Pro Val Val Ser Val Glu Tyr Lys Gly
               100                 105                 110
   Glu Glu Lys Ser Phe Thr Pro Glu Glu Ile Ser Ala Met Val Leu Gly
           115                 120                 125
   Lys Met Lys Leu Ile Ala Glu Asp Tyr Leu Gly Lys Lys Val Thr His
       130                 135                 140
   Ala Val Val Thr Val Pro Ala Tyr Phe Asn Asp Ala Gln Arg Gln Ala
   145                 150                 155                 160
   Thr Lys Asp Ala Gly Leu Ile Ala Gly Leu Thr Val Leu Arg Ile Val
                   165                 170                 175
   Asn Glu Pro Thr Ala Ala Ala Leu Ala Tyr Gly Leu Asp Lys Thr Gly
               180                 185                 190
   Glu Glu Arg Gln Ile Ile Val Tyr Asp Leu Gly Gly Gly Thr Phe Asp
           195                 200                 205
   Val Ser Leu Leu Ser Ile Glu Gly Gly Ala Phe Glu Val Leu Ala Thr
       210                 215                 220
   Ala Gly Asp Thr His Leu Gly Gly Glu Asp Phe Asp Tyr Arg Val Val
   225                 230                 235                 240
   Arg His Phe Val Lys Ile Phe Lys Lys Lys His Asn Ile Asp Ile Ser
                   245                 250                 255
   Asn Asn Asp Lys Ala Leu Gly Lys Leu Lys Arg Glu Val Glu Lys Ala
                   260                 265                 270
   Lys Arg Thr Leu Ser Ser Gln Met Thr Thr Arg Ile Glu Ile Asp Ser
               275                 280                 285
   Phe Val Asp Gly Ile Asp Phe Ser Glu Gln Leu Ser Arg Ala Lys Phe
       290                 295                 300
   Glu Glu Ile Asn Ile Glu Leu Phe Lys Lys Thr Leu Lys Pro Val Glu
   305                 310                 315                 320
   Gln Val Leu Lys Asp Ala Gly Val Lys Lys Ser Glu Ile Asp Asp Ile
                   325                 330                 335
   Val Leu Val Gly Gly Ser Thr Arg Ile Pro Lys Val Gln Gln Leu Leu
               340                 345                 350
   Glu Asp Tyr Phe Asp Gly Lys Lys Ala Ser Lys Gly Ile Asn Pro Asp
           355                 360                 365
   Glu Ala Val Ala Tyr Gly Ala Ala Val Gln Ala Gly Val Leu Ser Gly
       370                 375                 380
   Asp Gln Asp Thr Gly Asp Leu Val Leu Leu Asp Val Cys Pro Leu Thr
   385                 390                 395                 400
   Leu Gly Ile Glu Thr Val Gly Gly Val Met Thr Lys Leu Ile Pro Arg
                   405                 410                 415
   Asn Thr Val Val Pro Thr Lys Lys Ser Gln Ile Phe Ser Thr Ala Ser
               420                 425                 430
   Asp Asn Gln Pro Thr Val Thr Ile Lys Val Tyr Glu Gly Glu Arg Pro
           435                 440                 445
   Leu Thr Lys Asp Asn His Leu Leu Gly Thr Phe Asp Leu Thr Gly Ile
       450                 455                 460
   Pro Pro Ala Pro Arg Gly Val Pro Gln Ile Glu Val Thr Phe Glu Ile
   465                 470                 475                 480
   Asp Val Asn Gly Ile Leu Arg Val Thr Ala Glu Asp Lys Gly Thr Gly
                   485                 490                 495
   Asn Lys Asn Lys Ile Thr Ile Thr Asn Asp Gln Asn Arg Leu Thr Pro
               500                 505                 510
   Glu Glu Ile Glu Arg Met Val Asn Asp Ala Glu Lys Phe Ala Glu Glu
           515                 520                 525
   Asp Lys Lys Leu Lys Glu Arg Ile Asp Thr Arg Asn Glu Leu Glu Ser
       530                 535                 540
   Tyr Ala Tyr Ser Leu Lys Asn Gln Ile Gly Asp Lys Glu Lys Leu Gly
   545                 550                 555                 560
   Gly Lys Leu Ser Ser Glu Asp Lys Glu Thr Met Glu Lys Ala Val Glu
                   565                 570                 575
   Glu Lys Ile Glu Trp Leu Glu Ser His Gln Asp Ala Asp Ile Glu Asp
               580                 585                 590
   Phe Lys Ala Lys Lys Lys Glu Leu Glu Glu Ile Val Gln Pro Ile Ile
           595                 600                 605
   Ser Lys Leu Tyr Gly Ser Ala Gly Pro Pro Pro Thr Gly Glu Glu Asp
       610                 615                 620
   Thr Ala Glu His Asp Glu Leu
   625                 630
<210> 59
   <211> 254
   <212> DNA
   <213> Artificial Sequence
<220>
   <223> Pichia pastoris PDI1 promoter
<400> 59
   aacacgaaca ctgtaaatag aataaaagaa aacttggata gtagaacttc aatgtagtgt 60
   ttctattgtc ttacgcggct ctttagattg caatccccag aatggaatcg tccatctttc 120
   tcaacccact caaagataat ctaccagaca tacctacgcc ctccatccca gcaccacgtc 180
   gcgatcaccc ctaaaacttc aataattgaa cacgtactga tttccaaacc ttcttcttct 240
   tcctatctat aaga                                                   254
<210> 60
   <211> 2775
   <212> DNA
   <213> Pichia pastoris
<400> 60
   atgacagcta atgaaaatcc ttttgagaat gagctgacag gatcttctga atctgccccc 60
   cctgcattgg aatcgaagac tggagagtct cttaagtatt gcaaatatac cgtggatcag 120
   gtcatagaag agtttcaaac ggatggtctc aaaggattgt gcaattccca ggacatcgta 180
   tatcggaggt ctgttcatgg gccaaatgaa atggaagtcg aagaggaaga gagtcttttt 240
   tcgaaattct tgtcaagttt ctacagcgat ccattgattc tgttactgat gggttccgct 300
   gtgattagct ttttgatgtc taacattgat gatgcgatat ctatcactat ggcaattacg 360
   atcgttgtca cagttggatt tgttcaagag tatcgatccg agaaatcatt ggaggcattg 420
   aacaagttag tccctgccga agctcatcta actaggaatg ggaacactga aactgttctt 480
   gctgccaacc tagtcccagg agacttggtg gatttttcgg ttggtgacag aattccggct 540
   gatgtgagaa ttattcacgc ttcccacttg agtatcgacg agagcaacct aactggtgaa 600
   aatgaaccag tttctaaaga cagcaaacct gttgaaagtg atgacccaaa cattcccttg 660
   aacagccgtt catgtattgg gtatatgggc actttagttc gtgatggtaa tggcaaaggt 720
   attgtcatcg gaacagccaa aaacacagct tttggctctg ttttcgaaat gatgagctct 780
   attgagaaac caaagactcc tcttcaacag gctatggata aacttggtaa ggatttgtct 840
   gctttttcct tcggaatcat cggccttatt tgcttggttg gtgtttttca aggtagaccc 900
   tggttggaaa tgttccagat ctctgtatcc ttggctgttg ctgcgattcc agaaggtctt 960
   cctattattg tgactgtgac tcttgctctt ggtgtgttgc gtatggctaa acagagggcc 1020
   atcgtcaaaa gactgcctag tgttgaaact ttgggatccg tcaatgttat ctgtagtgat 1080
   aagacgggaa cattgaccca aaatcatatg accgttaaca gattatggac tgtggatatg 1140
   ggcgatgaat tcttgaaaat tgaacaaggg gagtcctatg ccaattatct caaacccgat 1200
   acgctaaaag ttctgcaaac tggtaatata gtcaacaatg ccaaatattc aaatgaaaag 1260
   gaaaaatacc tcggaaaccc aactgatatt gcaattattg aatctttaga aaaatttgat 1320
   ttgcaggaca ttagagcaac aaaggaaaga atgttggaga ttccattttc ttcgtccaag 1380
   aaatatcagg ccgtcagtgt tcactctgga gacaaaagca aatctgaaat ttttgttaaa 1440
   ggcgctctga acaaagtttt ggaaagatgt tcaagatatt acaatgctga aggtatcgcc 1500
   actccactca cagatgaaat tagaagaaaa tccttgcaaa tggccgatac gttagcatct 1560
   tcaggattga gaatactgtc gtttgcttac gacaaaggca attttgaaga aactggcgat 1620
   ggaccatcgg atatgatctt ttgtggtctt ttaggtatga acgatcctcc tagaccatct 1680
   gtaagtaaat caattttgaa attcatgaga ggtggggttc acattattat gattacagga 1740
   gattcagaat ccacggccgt agccgttgcc aaacaggtcg gaatggtaat tgacaattca 1800
   aaatatgctg tcctcagtgg agacgatata gatgctatga gtacagagca actgtctcag 1860
   gcgatctcac attgttctgt atttgcccgg actactccaa aacataaggt gtccattgta 1920
   agagcactac aggccagagg agatattgtt gcaatgactg gtgacggtgt caatgatgcc 1980
   ccagctctaa aactggccga catcggaatt gccatgggta atatggggac cgatgttgcc 2040
   aaagaggcag ccgacatggt tttgactgat gatgactttt ctacaatctt atctgcaatc 2100
   caggagggta aaggtatttt ctacaacatc cagaactttt taacgttcca actttctact 2160
   tcaattgctg ctctttcgtt aattgctctg agtactgctt tcaacctgcc aaatccattg 2220
   aatgccatgc agattttgtg gatcaatatt atcatggatg gacctccagc tcagtctttg 2280
   ggtgttgagc cagttgataa agctgtgatg aacaaaccac caagaaagcg aaatgataaa 2340
   attctgacag gtaaggtgat tcaaagggta gtacaaagta gttttatcat tgtttgtggt 2400
   actctgtacg tatacatgca tgagatcaaa gataatgagg tcacagcaag agacactacg 2460
   atgaccttta catgctttgt attctttgac atgttcaacg cattaacgac aagacaccat 2520
   tctaaaagta ttgcagaact tggatggaat aatactatgt tcaacttttc cgttgcagct 2580
   tctattttgg gtcaactagg agctatttac attccatttt tgcagtctat tttccagact 2640
   gaacctctga gcctcaaaga tttggtccat ttattgttgt tatcgagttc agtatggatt 2700
   gtagacgagc ttcgaaaact ctacgtcagg agacgtgacg catccccata caatggatac 2760
   agcatggctg tttga                                                  2775
<210> 61
   <211> 924
   <212> PRT
   <213> Pichia pastoris
<400> 61
   Met Thr Ala Asn Glu Asn Pro Phe Glu Asn Glu Leu Thr Gly Ser Ser
   1               5                   10                  15
   Glu Ser Ala Pro Pro Ala Leu Glu Ser Lys Thr Gly Glu Ser Leu Lys
               20                  25                  30
   Tyr Cys Lys Tyr Thr Val Asp Gln Val Ile Glu Glu Phe Gln Thr Asp
           35                  40                  45
   Gly Leu Lys Gly Leu Cys Asn Ser Gln Asp Ile Val Tyr Arg Arg Ser
       50                  55                  60
   Val His Gly Pro Asn Glu Met Glu Val Glu Glu Glu Glu Ser Leu Phe
   65                  70                  75                  80
   Ser Lys Phe Leu Ser Ser Phe Tyr Ser Asp Pro Leu Ile Leu Leu Leu
                   85                  90                  95
   Met Gly Ser Ala Val Ile Ser Phe Leu Met Ser Asn Ile Asp Asp Ala
               100                 105                 110
   Ile Ser Ile Thr Met Ala Ile Thr Ile Val Val Thr Val Gly Phe Val
           115                 120                 125
   Gln Glu Tyr Arg Ser Glu Lys Ser Leu Glu Ala Leu Asn Lys Leu Val
       130                 135                 140
   Pro Ala Glu Ala His Leu Thr Arg Asn Gly Asn Thr Glu Thr Val Leu
   145                 150                 155                 160
   Ala Ala Asn Leu Val Pro Gly Asp Leu Val Asp Phe Ser Val Gly Asp
                   165                 170                 175
   Arg Ile Pro Ala Asp Val Arg Ile Ile His Ala Ser His Leu Ser Ile
               180                 185                 190
   Asp Glu Ser Asn Leu Thr Gly Glu Asn Glu Pro Val Ser Lys Asp Ser
           195                 200                 205
   Lys Pro Val Glu Ser Asp Asp Pro Asn Ile Pro Leu Asn Ser Arg Ser
       210                 215                 220
   Cys Ile Gly Tyr Met Gly Thr Leu Val Arg Asp Gly Asn Gly Lys Gly
   225                 230                 235                 240
   Ile Val Ile Gly Thr Ala Lys Asn Thr Ala Phe Gly Ser Val Phe Glu
                   245                 250                 255
   Met Met Ser Ser Ile Glu Lys Pro Lys Thr Pro Leu Gln Gln Ala Met
               260                 265                 270
   Asp Lys Leu Gly Lys Asp Leu Ser Ala Phe Ser Phe Gly Ile Ile Gly
           275                 280                 285
   Leu Ile Cys Leu Val Gly Val Phe Gln Gly Arg Pro Trp Leu Glu Met
       290                 295                 300
   Phe Gln Ile Ser Val Ser Leu Ala Val Ala Ala Ile Pro Glu Gly Leu
   305                 310                 315                 320
   Pro Ile Ile Val Thr Val Thr Leu Ala Leu Gly Val Leu Arg Met Ala
                   325                 330                 335
   Lys Gln Arg Ala Ile Val Lys Arg Leu Pro Ser Val Glu Thr Leu Gly
               340                 345                 350
   Ser Val Asn Val Ile Cys Ser Asp Lys Thr Gly Thr Leu Thr Gln Asn
           355                 360                 365
   His Met Thr Val Asn Arg Leu Trp Thr Val Asp Met Gly Asp Glu Phe
       370                 375                 380
   Leu Lys Ile Glu Gln Gly Glu Ser Tyr Ala Asn Tyr Leu Lys Pro Asp
   385                 390                 395                 400
   Thr Leu Lys Val Leu Gln Thr Gly Asn Ile Val Asn Asn Ala Lys Tyr
                   405                 410                 415
   Ser Asn Glu Lys Glu Lys Tyr Leu Gly Asn Pro Thr Asp Ile Ala Ile
               420                 425                 430
   Ile Glu Ser Leu Glu Lys Phe Asp Leu Gln Asp Ile Arg Ala Thr Lys
           435                 440                 445
   Glu Arg Met Leu Glu Ile Pro Phe Ser Ser Ser Lys Lys Tyr Gln Ala
       450                 455                 460
   Val Ser Val His Ser Gly Asp Lys Ser Lys Ser Glu Ile Phe Val Lys
   465                 470                 475                 480
   Gly Ala Leu Asn Lys Val Leu Glu Arg Cys Ser Arg Tyr Tyr Asn Ala
                       485                 490                 495
   Glu Gly Ile Ala Thr Pro Leu Thr Asp Glu Ile Arg Arg Lys Ser Leu
                   500                 505                 510
   Gln Met Ala Asp Thr Leu Ala Ser Ser Gly Leu Arg Ile Leu Ser Phe
           515                 520                 525
   Ala Tyr Asp Lys Gly Asn Phe Glu Glu Thr Gly Asp Gly Pro Ser Asp
       530                 535                 540
   Met Ile Phe Cys Gly Leu Leu Gly Met Asn Asp Pro Pro Arg Pro Ser
   545                 550                 555                 560
   Val Ser Lys Ser Ile Leu Lys Phe Met Arg Gly Gly Val His Ile Ile
                       565                 570                 575
   Met Ile Thr Gly Asp Ser Glu Ser Thr Ala Val Ala Val Ala Lys Gln
                   580                 585                 590
   Val Gly Met Val Ile Asp Asn Ser Lys Tyr Ala Val Leu Ser Gly Asp
           595                 600                 605
   Asp Ile Asp Ala Met Ser Thr Glu Gln Leu Ser Gln Ala Ile Ser His
       610                 615                 620
   Cys Ser Val Phe Ala Arg Thr Thr Pro Lys His Lys Val Ser Ile Val
   625                 630                 635                 640
   Arg Ala Leu Gln Ala Arg Gly Asp Ile Val Ala Met Thr Gly Asp Gly
                       645                 650                 655
   Val Asn Asp Ala Pro Ala Leu Lys Leu Ala Asp Ile Gly Ile Ala Met
                   660                 665                 670
   Gly Asn Met Gly Thr Asp Val Ala Lys Glu Ala Ala Asp Met Val Leu
           675                 680                 685
   Thr Asp Asp Asp Phe Ser Thr Ile Leu Ser Ala Ile Gln Glu Gly Lys
       690                 695                 700
   Gly Ile Phe Tyr Asn Ile Gln Asn Phe Leu Thr Phe Gln Leu Ser Thr
   705                 710                 715                 720
   Ser Ile Ala Ala Leu Ser Leu Ile Ala Leu Ser Thr Ala Phe Asn Leu
                       725                 730                 735
   Pro Asn Pro Leu Asn Ala Met Gln Ile Leu Trp Ile Asn Ile Ile Met
                   740                 745                 750
   Asp Gly Pro Pro Ala Gln Ser Leu Gly Val Glu Pro Val Asp Lys Ala
           755                 760                 765
   Val Met Asn Lys Pro Pro Arg Lys Arg Asn Asp Lys Ile Leu Thr Gly
       770                 775                 780
   Lys Val Ile Gln Arg Val Val Gln Ser Ser Phe Ile Ile Val Cys Gly
   785                 790                 795                 800
   Thr Leu Tyr Val Tyr Met His Glu Ile Lys Asp Asn Glu Val Thr Ala
                       805                 810                 815
   Arg Asp Thr Thr Met Thr Phe Thr Cys Phe Val Phe Phe Asp Met Phe
                   820                 825                 830
   Asn Ala Leu Thr Thr Arg His His Ser Lys Ser Ile Ala Glu Leu Gly
           835                 840                 845
   Trp Asn Asn Thr Met Phe Asn Phe Ser Val Ala Ala Ser Ile Leu Gly
       850                 855                 860
   Gln Leu Gly Ala Ile Tyr Ile Pro Phe Leu Gln Ser Ile Phe Gln Thr
   865                 870                 875                 880
   Glu Pro Leu Ser Leu Lys Asp Leu Val His Leu Leu Leu Leu Ser Ser
                       885                 890                 895
   Ser Val Trp Ile Val Asp Glu Leu Arg Lys Leu Tyr Val Arg Arg Arg
                   900                 905                 910
   Asp Ala Ser Pro Tyr Asn Gly Tyr Ser Met Ala Val
           915                 920
<210> 62
   <211> 3186
   <212> DNA
   <213> Artificial Sequence
<220>
   <223> Arabidopsis thalian DNA encoding ECA1 codon-optimized for Pichia expression
<400> 62
<210> 63
   <211> 1061
   <212> PRT
   <213> Arabidopsis thaliana
<400> 63
   Met Gly Lys Gly Ser Glu Asp Leu Val Lys Lys Glu Ser Leu Asn Ser
    1               5                  10                  15
   Thr Pro Val Asn Ser Asp Thr Phe Pro Ala Trp Ala Lys Asp Val Ala
               20                  25                  30
   Glu Cys Glu Glu His Phe Val Val Ser Arg Glu Lys Gly Leu Ser Ser
           35                  40                  45
   Asp Glu Val Leu Lys Arg His Gln Ile Tyr Gly Leu Asn Glu Leu Glu
       50                  55                  60
   Lys Pro Glu Gly Thr Ser Ile Phe Lys Leu Ile Leu Glu Gln Phe Asn
   65                  70                  75                  80
   Asp Thr Leu Val Arg Ile Leu Leu Ala Ala Ala Val Ile Ser Phe Val
                   85                  90                  95
   Leu Ala Phe Phe Asp Gly Asp Glu Gly Gly Glu Met Gly Ile Thr Ala
               100                 105                 110
   Phe Val Glu Pro Leu Val Ile Phe Leu Ile Leu Ile Val Asn Ala Ile
           115                 120                 125
   Val Gly Ile Trp Gln Glu Thr Asn Ala Glu Lys Ala Leu Glu Ala Leu
       130                 135                 140
   Lys Glu Ile Gln Ser Gln Gln Ala Thr Val Met Arg Asp Gly Thr Lys
   145                 150                 155                 160
   Val Ser Ser Leu Pro Ala Lys Glu Leu Val Pro Gly Asp Ile Val Glu
                   165                 170                 175
   Leu Arg Val Gly Asp Lys Val Pro Ala Asp Met Arg Val Val Ala Leu
               180                 185                 190
   Ile Ser Ser Thr Leu Arg Val Glu Gln Gly Ser Leu Thr Gly Glu Ser
           195                 200                 205
   Glu Ala Val Ser Lys Thr Thr Lys His Val Asp Glu Asn Ala Asp Ile
       210                 215                 220
   Gln Gly Lys Lys Cys Met Val Phe Ala Gly Thr Thr Val Val Asn Gly
   225                 230                 235                 240
   Asn Cys Ile Cys Leu Val Thr Asp Thr Gly Met Asn Thr Glu Ile Gly
                   245                 250                 255
   Arg Val His Ser Gln Ile Gln Glu Ala Ala Gln His Glu Glu Asp Thr
               260                 265                 270
   Pro Leu Lys Lys Lys Leu Asn Glu Phe Gly Glu Val Leu Thr Met Ile
           275                 280                 285
   Ile Gly Leu Ile Cys Ala Leu Val Trp Leu Ile Asn Val Lys Tyr Phe
       290                 295                 300
   Leu Ser Trp Glu Tyr Val Asp Gly Trp Pro Arg Asn Phe Lys Phe Ser
   305                 310                 315                 320
   Phe Glu Lys Cys Thr Tyr Tyr Phe Glu Ile Ala Val Ala Leu Ala Val
                   325                 330                 335
   Ala Ala Ile Pro Glu Gly Leu Pro Ala Val Ile Thr Thr Cys Leu Ala
               340                 345                 350
   Leu Gly Thr Arg Lys Met Ala Gln Lys Asn Ala Leu Val Arg Lys Leu
           355                 360                 365
   Pro Ser Val Glu Thr Leu Gly Cys Thr Thr Val Ile Cys Ser Asp Lys
       370                 375                 380
   Thr Gly Thr Leu Thr Thr Asn Gln Met Ala Val Ser Lys Leu Val Ala
   385                 390                 395                 400
   Met Gly Ser Arg Ile Gly Thr Leu Arg Ser Phe Asn Val Glu Gly Thr
                   405                 410                 415
   Ser Phe Asp Pro Arg Asp Gly Lys Ile Glu Asp Trp Pro Met Gly Arg
                   420                 425                 430
   Met Asp Ala Asn Leu Gln Met Ile Ala Lys Ile Ala Ala Ile Cys Asn
               435                 440                 445
   Asp Ala Asn Val Glu Gln Ser Asp Gln Gln Phe Val Ser Arg Gly Met
       450                 455                 460
   Pro Thr Glu Ala Ala Leu Lys Val Leu Val Glu Lys Met Gly Phe Pro
   465                 470                 475                 480
   Glu Gly Leu Asn Glu Ala Ser Ser Asp Gly Asp Val Leu Arg Cys Cys
                   485                 490                 495
   Arg Leu Trp Ser Glu Leu Glu Gln Arg Ile Ala Thr Leu Glu Phe Asp
               500                 505                 510
   Arg Asp Arg Lys Ser Met Gly Val Met Val Asp Ser Ser Ser Gly Asn
           515                 520                 525
   Lys Leu Leu Leu Val Lys Gly Ala Val Glu Asn Val Leu Glu Arg Ser
       530                 535                 540
   Thr His Ile Gln Leu Leu Asp Gly Ser Lys Arg Glu Leu Asp Gln Tyr
   545                 550                 555                 560
   Ser Arg Asp Leu Ile Leu Gln Ser Leu Arg Asp Met Ser Leu Ser Ala
                   565                 570                 575
   Leu Arg Cys Leu Gly Phe Ala Tyr Ser Asp Val Pro Ser Asp Phe Ala
               580                 585                 590
   Thr Tyr Asp Gly Ser Glu Asp His Pro Ala His Gln Gln Leu Leu Asn
           595                 600                 605
   Pro Ser Asn Tyr Ser Ser Ile Glu Ser Asn Leu Ile Phe Val Gly Phe
       610                 615                 620
   Val Gly Leu Arg Asp Pro Pro Arg Lys Glu Val Arg Gln Ala Ile Ala
   625                 630                 635                 640
   Asp Cys Arg Thr Ala Gly Ile Arg Val Met Val Ile Thr Gly Asp Asn
                   645                 650                 655
   Lys Ser Thr Ala Glu Ala Ile Cys Arg Glu Ile Gly Val Phe Glu Ala
               660                 665                 670
   Asp Glu Asp Ile Ser Ser Arg Ser Leu Thr Gly Ile Glu Phe Met Asp
           675                 680                 685
   Val Gln Asp Gln Lys Asn His Leu Arg Gln Thr Gly Gly Leu Leu Phe
       690                 695                 700
   Ser Arg Ala Glu Pro Lys His Lys Gln Glu Ile Val Arg Leu Leu Lys
   705                 710                 715                 720
   Glu Asp Gly Glu Val Val Ala Met Thr Gly Asp Gly Val Asn Asp Ala
                   725                 730                 735
   Pro Ala Leu Lys Leu Ala Asp Ile Gly Val Ala Met Gly Ile Ser Gly
               740                 745                 750
   Thr Glu Val Ala Lys Glu Ala Ser Asp Met Val Leu Ala Asp Asp Asn
           755                 760                 765
   Phe Ser Thr Ile Val Ala Ala Val Gly Glu Gly Arg Ser Ile Tyr Asn
       770                 775                 780
   Asn Met Lys Ala Phe Ile Arg Tyr Met Ile Ser Ser Asn Ile Gly Glu
   785                 790                 795                 800
   Val Ala Ser Ile Phe Leu Thr Ala Ala Leu Gly Ile Pro Glu Gly Met
                   805                 810                 815
   Ile Pro Val Gln Leu Leu Trp Val Asn Leu Val Thr Asp Gly Pro Pro
               820                 825                 830
   Ala Thr Ala Leu Gly Phe Asn Pro Pro Asp Lys Asp Ile Met Lys Lys
           835                 840                 845
   Pro Pro Arg Arg Ser Asp Asp Ser Leu Ile Thr Ala Trp Ile Leu Phe
       850                 855                 860
   Arg Tyr Met Val Ile Gly Leu Tyr Val Gly Val Ala Thr Val Gly Val
   865                 870                 875                 880
   Phe Ile Ile Trp Tyr Thr His Ser Ser Phe Met Gly Ile Asp Leu Ser
                   885                 890                 895
   Gln Asp Gly His Ser Leu Val Ser Tyr Ser Gln Leu Ala His Trp Gly
               900                 905                 910
   Gln Cys Ser Ser Trp Glu Gly Phe Lys Val Ser Pro Phe Thr Ala Gly
           915                 920                 925
   Ser Gln Thr Phe Ser Phe Asp Ser Asn Pro Cys Asp Tyr Phe Gln Gln
       930                 935                 940
   Gly Lys Ile Lys Ala Ser Thr Leu Ser Leu Ser Val Leu Val Ala Ile
   945                 950                 955                 960
   Glu Met Phe Asn Ser Leu Asn Ala Leu Ser Glu Asp Gly Ser Leu Val
                   965                 970                 975
   Thr Met Pro Pro Trp Val Asn Pro Trp Leu Leu Leu Ala Met Ala Val
               980                 985                 990
   Ser Phe Gly Leu His Phe Val Ile Leu Tyr Val Pro Phe Leu Ala Gln
           995                 1000                1005
   Val Phe Gly Ile Val Pro Leu Ser Leu Asn Glu Trp Leu Leu Val Leu
       1010                1015                1020
   Ala Val Ser Leu Pro Val Ile Leu Ile Asp Glu Val Leu Lys Phe Val
   1025                1030                1035                1040
   Gly Arg Cys Thr Ser Gly Tyr Arg Tyr Ser Pro Arg Thr Leu Ser Thr
                   1045                1050                1055
   Lys Gln Lys Glu Glu
               1060
<210> 64
   <211> 39
   <212> DNA
   <213> Artificial Sequence
<220>
   <223> PpPMR1/UP PCR primer
<400> 64
   gaattcatga cagctaatga aaatcctttt gagaatgag 39
<210> 65
   <211> 37
   <212> DNA
   <213> Artificial Sequence
<220>
   <223> PpPMR1/LP PCR primer
<400> 65
   ggccggcctc aaacagccat gctgtatcca ttgtatg 37
<210> 66
   <211> 25
   <212> DNA
   <213> Artificial Sequence
<220>
   <223> 5'AOX1 PCR primer
<400> 66
   gcgactggtt ccaattgaca agctt 25
<210> 67
   <211> 30
   <212> DNA
   <213> Artificial Sequence
<220>
   <223> PpPMR1/cLP PCR primer
<400> 67
   ggttgctctc gtcgatactc aagtgggaag 30
<210> 68
   <211> 30
   <212> DNA
   <213> Artificial Sequence
<220>
   <223> AtECA1/cLP PCR primer
<400> 68
   gtcggctgga accttatcac caactctcag 30
<210> 69
   <211> 1314
   <212> DNA
   <213> Homo sapiens
<400> 69
   atgagatttc cttcaatttt tactgctgtt ttattcgcag catcctccgc attagcttac 60
   ccatacgacg tcccagacta cgcttaccca tacgacgtcc cagactacgc tgagcccgcc 120
   gtctacttca aggagcagtt tctggacgga gacgggtgga cttcccgctg gatcgaatcc 180
   aaacacaagt cagattttgg caaattcgtt ctcagttccg gcaagttcta cggtgacgag 240
   gagaaagata aaggtttgca gacaagccag gatgcacgct tttatgctct gtcggccagt 300
   ttcgagcctt tcagcaacaa aggccagacg ctggtggtgc agttcacggt gaaacatgag 360
   cagaacatcg actgtggggg cggctatgtg aagctgtttc ctaatagttt ggaccagaca 420
   gacatgcacg gagactcaga atacaacatc atgtttggtc ccgacatctg tggccctggc 480
   accaagaagg ttcatgtcat cttcaactac aagggcaaga acgtgctgat caacaaggac 540
   atccgttgca aggatgatga gtttacacac ctgtacacac tgattgtgcg gccagacaac 600
   acctatgagg tgaagattga caacagccag gtggagtccg gctccttgga agacgattgg 660
   gacttcctgc cacccaagaa gataaaggat cctgatgctt caaaaccgga agactgggat 720
   gagcgggcca agatcgatga tcccacagac tccaagcctg aggactggga caagcccgag 780
   catatccctg accctgatgc taagaagccc gaggactggg atgaagagat ggacggagag 840
   tgggaacccc cagtgattca gaaccctgag tacaagggtg agtggaagcc ccggcagatc 900
   gacaacccag attacaaggg cacttggatc cacccagaaa ttgacaaccc cgagtattct 960
   cccgatccca gtatctatgc ctatgataac tttggcgtgc tgggcctgga cctctggcag 1020
   gtcaagtctg gcaccatctt tgacaacttc ctcatcacca acgatgaggc atacgctgag 1080
   gagtttggca acgagacgtg gggcgtaaca aaggcagcag agaaacaaat gaaggacaaa 1140
   caggacgagg agcagaggct taaggaggag gaagaagaca agaaacgcaa agaggaggag 1200
   gaggcagagg acaaggagga tgatgaggac aaagatgagg atgaggagga tgaggaggac 1260
   aaggaggaag atgaggagga agatgtcccc ggccaggccc atgacgagct gtag       1314
<210> 70
   <211> 437
   <212> PRT
   <213> Artificial Sequence
<220>
   <223> Human calreticulin (hCRT)-protein with Saccharomyces cerevisiae mating factor pre-signal peptide leader
<400> 70
   Met Arg Phe Pro Ser Ile Phe Thr Ala Val Leu Phe Ala Ala Ser Ser
    1               5                  10                  15
   Ala Leu Ala Tyr Pro Tyr Asp Val Pro Asp Tyr Ala Tyr Pro Tyr Asp
               20                  25                  30
   Val Pro Asp Tyr Ala Glu Pro Ala Val Tyr Phe Lys Glu Gln Phe Leu
           35                  40                  45
   Asp Gly Asp Gly Trp Thr Ser Arg Trp Ile Glu Ser Lys His Lys Ser
       50                  55                  60
   Asp Phe Gly Lys Phe Val Leu Ser Ser Gly Lys Phe Tyr Gly Asp Glu
   65                  70                  75                  80
   Glu Lys Asp Lys Gly Leu Gln Thr Ser Gln Asp Ala Arg Phe Tyr Ala
                   85                  90                  95
   Leu Ser Ala Ser Phe Glu Pro Phe Ser Asn Lys Gly Gln Thr Leu Val
               100                 105                 110
   Val Gln Phe Thr Val Lys His Glu Gln Asn Ile Asp Cys Gly Gly Gly
           115                 120                 125
   Tyr Val Lys Leu Phe Pro Asn Ser Leu Asp Gln Thr Asp Met His Gly
       130                 135                 140
   Asp Ser Glu Tyr Asn Ile Met Phe Gly Pro Asp Ile Cys Gly Pro Gly
   145                 150                 155                 160
   Thr Lys Lys Val His Val Ile Phe Asn Tyr Lys Gly Lys Asn Val Leu
                   165                 170                 175
   Ile Asn Lys Asp Ile Arg Cys Lys Asp Asp Glu Phe Thr His Leu Tyr
               180                 185                 190
   Thr Leu Ile Val Arg Pro Asp Asn Thr Tyr Glu Val Lys Ile Asp Asn
           195                 200                 205
   Ser Gln Val Glu Ser Gly Ser Leu Glu Asp Asp Trp Asp Phe Leu Pro
       210                 215                 220
   Pro Lys Lys Ile Lys Asp Pro Asp Ala Ser Lys Pro Glu Asp Trp Asp
   225                 230                 235                 240
   Glu Arg Ala Lys Ile Asp Asp Pro Thr Asp Ser Lys Pro Glu Asp Trp
                   245                 250                 255
   Asp Lys Pro Glu His Ile Pro Asp Pro Asp Ala Lys Lys Pro Glu Asp
               260                 265                 270
   Trp Asp Glu Glu Met Asp Gly Glu Trp Glu Pro Pro Val Ile Gln Asn
           275                 280                 285
   Pro Glu Tyr Lys Gly Glu Trp Lys Pro Arg Gln Ile Asp Asn Pro Asp
       290                 295                 300
   Tyr Lys Gly Thr Trp Ile His Pro Glu Ile Asp Asn Pro Glu Tyr Ser
   305                 310                 315                 320
   Pro Asp Pro Ser Ile Tyr Ala Tyr Asp Asn Phe Gly Val Leu Gly Leu
                   325                 330                 335
   Asp Leu Trp Gln Val Lys Ser Gly Thr Ile Phe Asp Asn Phe Leu Ile
               340                 345                 350
   Thr Asn Asp Glu Ala Tyr Ala Glu Glu Phe Gly Asn Glu Thr Trp Gly
           355                 360                 365
   Val Thr Lys Ala Ala Glu Lys Gln Met Lys Asp Lys Gln Asp Glu Glu
       370                 375                 380
   Gln Arg Leu Lys Glu Glu Glu Glu Asp Lys Lys Arg Lys Glu Glu Glu
   385                 390                 395                 400
   Glu Ala Glu Asp Lys Glu Asp Asp Glu Asp Lys Asp Glu Asp Glu Glu
                   405                 410                 415
   Asp Glu Glu Asp Lys Glu Glu Asp Glu Glu Glu Asp Val Pro Gly Gln
               420                 425                 430
   Ala His Asp Glu Leu
           435
<210> 71
   <211> 1512
   <212> DNA
   <213> Homo sapiens
<400> 71
   atgcaattca actggaacat caagactgtt gcttccatct tgtccgcttt gactttggct 60
   caagcttctg acgttttgga gttgactgac gacaacttcg agtccagaat ttctgacact 120
   ggttccgctg gattgatgtt ggttgagttc ttcgctccat ggtgtggtca ttgtaagaga 180
   ttggctccag aatacgaagc tgctgctact agattgaagg gtatcgttcc attggctaag 240
   gttgactgta ctgctaacac taacacttgt aacaagtacg gtgtttccgg ttacccaact 300
   ttgaagatct tcagagatgg tgaagaagct ggagcttacg acggtccaag aactgctgac 360
   ggtatcgttt cccacttgaa gaagcaagct ggtccagctt ctgttccatt gagaactgag 420
   gaggagttca agaagttcat ctccgacaag gacgcttcta tcgttggttt cttcgacgat 480
   tctttctctg aagctcactc cgaattcttg aaggctgctt ccaacttgag agacaactac 540
   agattcgctc acactaacgt tgagtccttg gttaacgagt acgacgataa cggtgaaggt 600
   atcatcttgt tcagaccatc ccacttgact aacaagttcg aggacaagac agttgcttac 660
   actgagcaga agatgacttc cggaaagatc aagaagttta tccaagagaa catcttcggt 720
   atctgtccac acatgactga ggacaacaag gacttgattc agggaaagga cttgttgatc 780
   gcttactacg acgttgacta cgagaagaac gctaagggtt ccaactactg gagaaacaga 840
   gttatgatgg ttgctaagaa gttcttggac gctggtcaca agttgaactt cgctgttgct 900
   tctagaaaga ctttctccca cgagttgtct gatttcggat tggaatccac tgctggagag 960
   attccagttg ttgctatcag aactgctaag ggagagaagt tcgttatgca agaggagttc 1020
   tccagagatg gaaaggcttt ggagagattc ttgcaggatt acttcgacgg taacttgaag 1080
   agatacttga agtccgagcc aattccagaa tctaacgacg gtccagttaa agttgttgtt 1140
   gctgagaact tcgacgagat cgttaacaac gagaacaagg acgttttgat cgagttttac 1200
   gctccttggt gtggacactg taaaaacttg gagccaaagt acaaggaatt gggtgaaaag 1260
   ttgtccaagg acccaaacat cgttatcgct aagatggacg ctactgctaa cgatgttcca 1320
   tccccatacg aagttagagg tttcccaact atctacttct ccccagctaa caagaagttg 1380
   aacccaaaga agtacgaggg aggtagagaa ttgtccgact tcatctccta cttgcagaga 1440
   gaggctacta atccaccagt tatccaagag gagaagccaa agaagaagaa gaaagctcac 1500
   gacgagttgt ag                                                      1512
<210> 72
   <211> 503
   <212> PRT
   <213> Homo sapiens
<400> 72
   Met Gln Phe Asn Trp Asn Ile Lys Thr Val Ala Ser Ile Leu Ser Ala
    1               5                  10                  15
   Leu Thr Leu Ala Gln Ala Ser Asp Val Leu Glu Leu Thr Asp Asp Asn
               20                  25                  30
   Phe Glu Ser Arg Ile Ser Asp Thr Gly Ser Ala Gly Leu Met Leu Val
           35                  40                  45
   Glu Phe Phe Ala Pro Trp Cys Gly His Cys Lys Arg Leu Ala Pro Glu
       50                  55                  60
   Tyr Glu Ala Ala Ala Thr Arg Leu Lys Gly Ile Val Pro Leu Ala Lys
   65                  70                  75                  80
   Val Asp Cys Thr Ala Asn Thr Asn Thr Cys Asn Lys Tyr Gly Val Ser
                   85                  90                  95
   Gly Tyr Pro Thr Leu Lys Ile Phe Arg Asp Gly Glu Glu Ala Gly Ala
               100                 105                 110
   Tyr Asp Gly Pro Arg Thr Ala Asp Gly Ile Val Ser His Leu Lys Lys
           115                 120                 125
   Gln Ala Gly Pro Ala Ser Val Pro Leu Arg Thr Glu Glu Glu Phe Lys
       130                 135                 140
   Lys Phe Ile Ser Asp Lys Asp Ala Ser Ile Val Gly Phe Phe Asp Asp
   145                 150                 155                 160
   Ser Phe Ser Glu Ala His Ser Glu Phe Leu Lys Ala Ala Ser Asn Leu
                   165                 170                 175
   Arg Asp Asn Tyr Arg Phe Ala His Thr Asn Val Glu Ser Leu Val Asn
               180                 185                 190
   Glu Tyr Asp Asp Asn Gly Glu Gly Ile Ile Leu Phe Arg Pro Ser His
           195                 200                 205
   Leu Thr Asn Lys Phe Glu Asp Lys Thr Val Ala Tyr Thr Glu Gln Lys
       210                 215                 220
   Met Thr Ser Gly Lys Ile Lys Lys Phe Ile Gln Glu Asn Ile Phe Gly
   225                 230                 235                 240
   Ile Cys Pro His Met Thr Glu Asp Asn Lys Asp Leu Ile Gln Gly Lys
                   245                 250                 255
   Asp Leu Leu Ile Ala Tyr Tyr Asp Val Asp Tyr Glu Lys Asn Ala Lys
               260                 265                 270
   Gly Ser Asn Tyr Trp Arg Asn Arg Val Met Met Val Ala Lys Lys Phe
           275                 280                 285
   Leu Asp Ala Gly His Lys Leu Asn Phe Ala Val Ala Ser Arg Lys Thr
       290                 295                 300
   Phe Ser His Glu Leu Ser Asp Phe Gly Leu Glu Ser Thr Ala Gly Glu
   305                 310                 315                 320
   Ile Pro Val Val Ala Ile Arg Thr Ala Lys Gly Glu Lys Phe Val Met
                   325                 330                 335
   Gln Glu Glu Phe Ser Arg Asp Gly Lys Ala Leu Glu Arg Phe Leu Gln
               340                 345                 350
   Asp Tyr Phe Asp Gly Asn Leu Lys Arg Tyr Leu Lys Ser Glu Pro Ile
           355                 360                 365
   Pro Glu Ser Asn Asp Gly Pro Val Lys Val Val Val Ala Glu Asn Phe
       370                 375                 380
   Asp Glu Ile Val Asn Asn Glu Asn Lys Asp Val Leu Ile Glu Phe Tyr
   385                 390                 395                 400
   Ala Pro Trp Cys Gly His Cys Lys Asn Leu Glu Pro Lys Tyr Lys Glu
                   405                 410                 415
   Leu Gly Glu Lys Leu Ser Lys Asp Pro Asn Ile Val Ile Ala Lys Met
               420                 425                 430
   Asp Ala Thr Ala Asn Asp Val Pro Ser Pro Tyr Glu Val Arg Gly Phe
           435                 440                 445
   Pro Thr Ile Tyr Phe Ser Pro Ala Asn Lys Lys Leu Asn Pro Lys Lys
       450                 455                 460
   Tyr Glu Gly Gly Arg Glu Leu Ser Asp Phe Ile Ser Tyr Leu Gln Arg
   465                 470                 475                 480
   Glu Ala Thr Asn Pro Pro Val Ile Gln Glu Glu Lys Pro Lys Lys Lys
                   485                 490                 495
   Lys Lys Ala His Asp Glu Leu
               500
<210> 73
   <211> 55
   <212> DNA
   <213> Artificial Sequence
<220>
   <223> hCRT-BstZ17I-HA/UP PCR primer
<400> 73
   gtatacccat acgacgtccc agactacgct gagcccgccg tctacttcaa ggagc 55
<210> 74
   <211> 45
   <212> DNA
   <213> Artificial Sequence
<220>
   <223> hCRT-PacI/LP PCR primer
<400> 74
   ttaattaact acagctcgtc atgggcctgg ccggggacat cttcc 45
<210> 75
   <211> 7
   <212> PRT
   <213> Artificial Sequence
<220>
   <223> Synthetic peptide that binds CRT
<400> 75
   Lys Leu Gly Phe Phe Lys Arg
   1               5
<210> 76
   <211> 1068
   <212> DNA
   <213> Artificial Sequence
<220>
   <223> Encodes human ERdj3 with Saccharomyces cerevisiae mating factor pre-signal peptide leader
<400> 76
   atgagatttc cttcaatttt tactgctgtt ttattcgcag catcctccgc attagctggt 60
   agagacttct acaagatttt gggtgttcca agatccgctt ccatcaagga catcaagaag 120
   gcttacagaa agttggcttt gcaattgcac ccagacagaa acccagatga cccacaagct 180
   caagagaagt tccaagactt gggtgctgct tacgaagttt tgtccgattc cgagaagaga 240
   aagcagtacg acacttacgg tgaagaagga ttgaaggacg gtcaccaatc ttctcacggt 300
   gacatcttct cccacttttt cggtgacttc ggtttcatgt tcggtggtac tccaagacaa 360
   caggacagaa acatcccaag aggttccgac attatcgttg acttggaggt tacattggaa 420
   gaggtttacg ctggtaactt cgttgaagtt gttagaaaca agccagttgc tagacaagct 480
   ccaggtaaaa gaaagtgtaa ctgtagacaa gagatgagaa ctactcagtt gggtcctggt 540
   agattccaaa tgacacagga agttgtttgc gacgagtgtc caaacgttaa gttggttaac 600
   gaagagagaa ctttggaggt tgagatcgag ccaggtgtta gagatggaat ggaataccca 660
   ttcatcggtg aaggtgaacc acatgttgat ggtgaacctg gtgacttgag attcagaatc 720
   aaagttgtta agcacccaat cttcgagaga agaggtgacg acttgtacac taacgttact 780
   atttccttgg ttgaatcctt ggttggtttc gagatggaca tcactcattt ggacggtcac 840
   aaggttcaca tttccagaga caagatcact agaccaggtg ctaagttgtg gaagaagggt 900
   gaaggattgc caaacttcga caacaacaac atcaagggat ctttgatcat cactttcgac 960
   gttgacttcc caaaagagca gttgactgaa gaagctagag agggtatcaa gcagttgttg 1020
   aagcaaggtt ccgttcagaa ggtttacaac ggattgcagg gatactaa              1068
<210> 77
   <211> 355
   <212> PRT
   <213> Artificial Sequence
<220>
   <223> Encodes human ERdj3 with Saccharomyces cerevisiae mating factor pre-signal peptide leader
<400> 77
   Met Arg Phe Pro Ser Ile Phe Thr Ala Val Leu Phe Ala Ala Ser Ser
    1               5                  10                  15
   Ala Leu Ala Gly Arg Asp Phe Tyr Lys Ile Leu Gly Val Pro Arg Ser
               20                  25                  30
   Ala Ser Ile Lys Asp Ile Lys Lys Ala Tyr Arg Lys Leu Ala Leu Gln
           35                  40                  45
   Leu His Pro Asp Arg Asn Pro Asp Asp Pro Gln Ala Gln Glu Lys Phe
       50                  55                  60
   Gln Asp Leu Gly Ala Ala Tyr Glu Val Leu Ser Asp Ser Glu Lys Arg
   65                  70                  75                  80
   Lys Gln Tyr Asp Thr Tyr Gly Glu Glu Gly Leu Lys Asp Gly His Gln
                   85                  90                  95
   Ser Ser His Gly Asp Ile Phe Ser His Phe Phe Gly Asp Phe Gly Phe
               100                 105                 110
   Met Phe Gly Gly Thr Pro Arg Gln Gln Asp Arg Asn Ile Pro Arg Gly
           115                 120                 125
   Ser Asp Ile Ile Val Asp Leu Glu Val Thr Leu Glu Glu Val Tyr Ala
       130                 135                 140
   Gly Asn Phe Val Glu Val Val Arg Asn Lys Pro Val Ala Arg Gln Ala
   145                 150                 155                 160
   Pro Gly Lys Arg Lys Cys Asn Cys Arg Gln Glu Met Arg Thr Thr Gln
                   165                 170                 175
   Leu Gly Pro Gly Arg Phe Gln Met Thr Gln Glu Val Val Cys Asp Glu
               180                 185                 190
   Cys Pro Asn Val Lys Leu Val Asn Glu Glu Arg Thr Leu Glu Val Glu
           195                 200                 205
   Ile Glu Pro Gly Val Arg Asp Gly Met Glu Tyr Pro Phe Ile Gly Glu
       210                 215                 220
   Gly Glu Pro His Val Asp Gly Glu Pro Gly Asp Leu Arg Phe Arg Ile
   225                 230                 235                 240
   Lys Val Val Lys His Pro Ile Phe Glu Arg Arg Gly Asp Asp Leu Tyr
                   245                 250                 255
   Thr Asn Val Thr Ile Ser Leu Val Glu Ser Leu Val Gly Phe Glu Met
               260                 265                 270
   Asp Ile Thr His Leu Asp Gly His Lys Val His Ile Ser Arg Asp Lys
           275                 280                 285
   Ile Thr Arg Pro Gly Ala Lys Leu Trp Lys Lys Gly Glu Gly Leu Pro
       290                 295                 300
   Asn Phe Asp Asn Asn Asn Ile Lys Gly Ser Leu Ile Ile Thr Phe Asp
   305                 310                 315                 320
   Val Asp Phe Pro Lys Glu Gln Leu Thr Glu Glu Ala Arg Glu Gly Ile
                   325                 330                 335
   Lys Gln Leu Leu Lys Gln Gly Ser Val Gln Lys Val Tyr Asn Gly Leu
               340                 345                 350
   Gln Gly Tyr
           355

## Description

### BACKGROUND OF THE INVENTION

### (1) Field of the Invention

The present invention relates to use of Protein Disulfide Isomerase (PDI) to improve protein production in *Pichia pastoris* recombinant expression systems. In general, *Pichia pastoris* host cells comprise a nucleic acid human PDI and a deletion or disruption of the gene encoding PDI. These host cells are useful for producing recombinant glycoproteins in large amounts and for producing recombinant glycoproteins that have reduced *O*-glycosylation.

### (2) Description of Related Art

Molecular chaperones play a critical role in the folding and secretion of proteins, and in particular, for the folding and secretion of antibodies. In lower eukaryotes, and particularly in yeast, Protein Disulfide Isomerase (PDI) is a chaperone protein, which functions to help create the disulphide bonds between multimeric proteins, such as those between antibody heavy and light chains. There have been past attempts to increase antibody expression levels in *P. pastoris* by overexpressing human PDI chaperone protein and/or overexpressing endogenous PDI. See for example, Wittrup et al., U.S. Patent No. 5,772,245; Toyoshima et al., U.S. Patent Nos. 5,700,678 and 5,874,247; Ng et al., U.S. Application Publication No. 2002/0068325; Toman et al., J. Biol. Chem. 275: 23303-23309 (2000); Keizer-Gunnink et al., Martix Biol. 19: 29-36 (2000); Vad et al., J. Biotechnol. 116: 251-260 (2005); Inana et al., Biotechnol. Bioengineer. 93: 771-778 (2005); Zhang et al., Biotechnol. Prog. 22: 1090-1095 (2006); Damasceno et al., Appl. Microbiol. Biotechnol. 74: 381-389 (2006); and, Huo et al., Protein express. Purif. 54: 234-239 (2007).

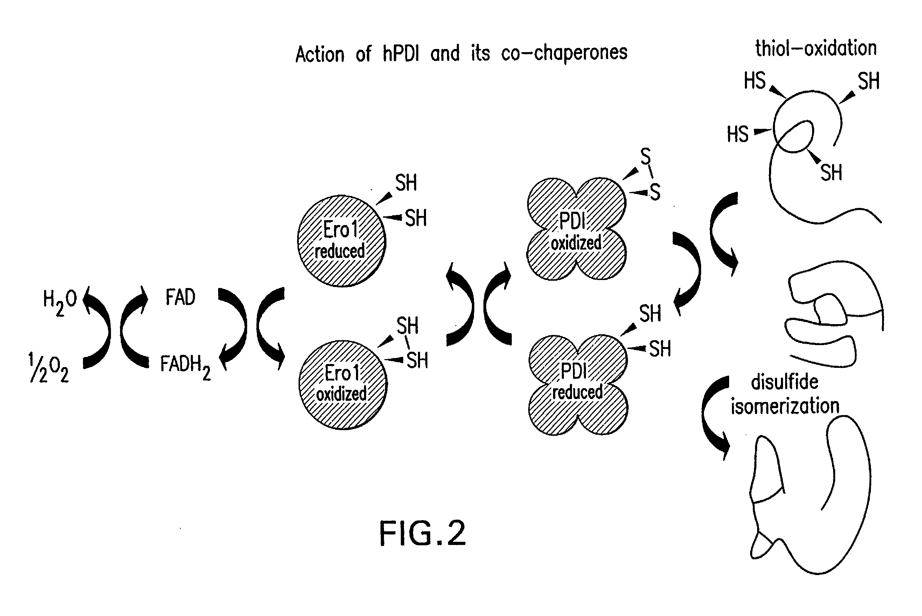

Protein disulfide isomerase (PDI) can produce a substantial increase for a substantial decrease in the recovery of disulfide-containing proteins, when compared with the uncatalyzed reaction; a high concentration of PDI in the endoplasmic reticulum (ER) is essential for the expression of disulfide-containing proteins (Puig and Gilbert, J. Biol. Chem., 269:7764-7771 (1994)). The action of PDI and its co-chaperones is shown in Figure 2.

In Gunther et al., J.Biol. Chem., 268:7728-7732 (1993) the *Trg1*/*Pdi1* gene of *Saccharomyces cerevisiae* was replaced by a murine gene of the protein disulfide isomerase family. It was found that two unglycosylated mammalian proteins PDI and ERp72 were capable of replacing at least some of the critical functions of Trg1, even though the three proteins diverged considerably in the sequences surrounding the thioredoxin-related domains; whereas ERp61 was inactive.

Kimura et al, Biochem. Biophys. Res.Comm., 320(2004) 359-365 discloses the transformation of a *Saccharomyces cerevisiae* strain deficient for yeast PDI with CDNAs encoding human PDI.

Xiao et al, J.Biol.Chem., 276(2002) 27975-27980 describes the rescue of a normally lethal pdiΔD mutation in *S.cerevisiae* with a full length rat PDI.

Development of further protein expression systems for yeasts and filamentous fungi, such as *Pichia pastoris,* based on improved vectors and host cell lines in which effective chaperone proteins would facilitate development of genetically enhanced yeast strains for the recombinant production of proteins, and in particular, for recombinant production of antibodies.

The present invention provides improved methods and materials for the production of recombinant proteins using auxiliary genes and PDI. In one embodiment, genetic engineering to humanize the chaperone pathway resulted in improved yield of recombinant antibody produced in *Pichia pastoris* cells.

As described herein, there are many attributes of the methods and materials of the present invention which provide unobvious advantages for such expression processes over prior known expression processes.

### BRIEF SUMMARY OF THE INVENTION

The present inventors have found that expression of recombinant proteins in a *Pichia pastoris* host cell comprising a deletion or disruption of an endogenous gene encoding a Protein Disulphide Isomerase (PDI), a nucleic acid molecule encoding a human PDI integrated into the genome of the host cell and a nucleic acid molecule encoding a recombinant human protein, wherein the human PDI is overexpressed. The *Pichia pastoris* host cell that expresses human PDI but not its endogenous PDI is able to produce active, correctly folded recombinant proteins in high amounts. This is an improvement in productivity compared to production of the recombinant protein in *Pichia pastoris* host cells that retain the endogenous PDI gene.

The present inventors have also found that by improving protein expression as described herein provides the further advantage that healthy, viable recombinant host cells that have a deletion or disruption of one or more of its endogenous protein *O*-mannosyltransferases *(PMT)* genes can be constructed. Deleting or disrupting one or more of the *PMT* genes in a lower eukaryotic cell results in a reduction in the amount of *O*-glycosylation of recombinant proteins produced in the cell. However, when *PMT* deletions are made in lower eukaryotic host cells that further include a deletion in one or genes encoding mannosyltransferases and express the endogenous chaperone proteins, the resulting cells often proved to be non-viable or low-producing cells, rendering them inappropriate for commercial use.

The chaperone protein Protein Disulphide Isomerase (PDI) is disrupted or deleted such that the endogenous *PDI1* is no longer present in the host cell and a nucleic acid molecule encoding a mammalian PDI protein is introduced into the host cell and expressed in the host cell. In further embodiments, the nucleic acid molecule for expressing the PDI comprises regulatory elements, such as promoter and transcription termination sequences, which are functional in the host cell, operably linked to an open reading frame encoding the human PDI protein. In other embodiments, the endogenous PDI gene is replaced with a nucleic acid molecule encoding a human PDI gene. This can be accomplished by homologous recombination or a single substitution event in which the endogenous PDI1 gene is looped out by the mammalian PDI gene, comprising overlapping sequences on both ends.

In further aspects, the *Pichia pastoris* host cells of the invention are further transformed with a recombinant vector comprising regulatory nucleotide sequences derived from *Pichia pastoris* host cells and a coding sequence encoding a selected human protein to be produced by the above host cells. In certain aspects, the selected human protein is a therapeutic protein, and may be a glycoprotein, such as an antibody.

The present invention also provides *Pichia pastoris* host cells wherein, in addition to replacing the genes encoding one or more of the endogenous chaperone proteins as described above, the function of at least one endogenous gene encoding a protein *O*-mannosyltransferase *(PMT)* protein is reduced, disrupted, or deleted. In particular embodiments, the function of at least one endogenous *PMT* gene selected from the group consisting of the *PMT1* and *PMT4* genes is reduced, disrupted, or deleted.

In further embodiments, the host cell further expresses a vector comprising regulatory nucleotide sequences derived from or functional in *Pichia pastoris* cells operably linked with an open reading frame encoding a human therapeutic glycoprotein, such as an antibody, which is introduced into the host cell. The host cell is then further be engineered to reduce or eliminate the function of at least one endogenous *Pichia pastoris* gene encoding a protein *O*-mannosyltransferase *(PMT)* protein selected from the group consisting of *PMT1* and *PMT4* to provide a host cell that is capable of making recombinant proteins having reduced *O-*glycosylation compared to host cells that have functional *PMT* genes. In further aspects, the host cells are further contacted with one or more inhibitors of *PMT* gene expression or *PMT* protein function.

The *Pichia pastoris* host cells of the present invention have been modified by reduction or elimination of the function of at least an endogenous gene encoding PDI. Reduction or elimination of the function of endogenous genes can be accomplished by any method known in the art, and can be accomplished by alteration of the genetic locus of the endogenous gene, for example, by mutation, insertion or deletion of genetic sequences sufficient to reduce or eliminate the function of the endogenous gene. In one embodiment, the endogenous gene encoding PDI is either deleted or altered in a manner which reduces or eliminates its function.

Thus, further provided are methods for producing a recombinant protein in the host cells disclosed herein, for example, in one embodiment, the method comprises providing a *Pichia pastoris* host cell in which the function of an endogenous gene encoding PDI has been disrupted or deleted and a nucleic acid molecule encoding human PDI is expressed in the host cell; introducing a nucleic acid molecule into the host cell encoding the recombinant protein; and growing the host cell under conditions suitable for producing the recombinant protein. In another embodiment, the method comprises providing a *Pichia pastoris* host cell in which the function of (i) an endogenous gene encoding PDI; and (ii) at least one endogenous gene encoding a protein O-mannosyltransferase *(PMT)* protein; have been reduced, disrupted, or deleted; and a nucleic acid molecule encoding human PDI is expressed in the host cell; introducing a nucleic acid molecule into the host cell encoding the recombinant protein; and growing the host cell under conditions suitable for producing the recombinant protein. In another embodiment, the method comprises providing a *Pichia pastoris* host cell in which the function of the endogenous gene encoding a chaperone protein PDI; and at least one endogenous gene encoding a protein *O-*mannosyltransferase-1 *(PMT*1*)* or *PMT*4 protein; have been reduced, disrupted, or deleted; and a nucleic acid molecule encoding human PDI is expressed in the host cell; introducing a nucleic acid molecule into the host cell encoding the recombinant protein; and growing the host cell under conditions suitable for producing the recombinant protein.

It has further been found that overexpressing an Ca²⁺ ATPase in the above host cells herein effects a decrease in *O*-glycan occupancy. It has also been found that overexpressing a calreticulin and an ERp57 protein in the above host cells also effected a reduction in *O*-glycan occupancy. Thus, in further embodiments of the above host cells, the host cell further includes one or more nucleic acid molecules encoding one or more exogenous or endogenous Ca²⁺ ATPases operably linked to a heterologous promoter. In further embodiments, the Ca²⁺ ATPase is the Ca²⁺ ATPase encoded by the *Pichia pastoris PMR1* gene or the *Arabidopsis thaliana* ECA1 gene. In further embodiments, the host cells further include one or more nucleic acid molecules encoding a calreticulin and/or an ERp57. Other Ca²⁺ ATPases that are suitable include but are not limited to human SERCA2b protein (ATP2A2 ATPase, Ca⁺⁺ transporting, cardiac muscle, slow twitch 2) and the *Pichia pastoris COD1* protein (homologue of *Saccharomyces cerevisiae* SPF1). Other proteins that are suitable include but are not limited to human UGGT (UDP-glucose:glycoprotein glucosyltransferase) protein and human ERp27 protein.

The *Pichia pastoris* host cell further includes a nucleic acid molecule encoding a recombinant protein, which in particular aspects, is a glycoprotein, which in further aspects is an antibody or fragment thereof such as Fc or Fab.

In further embodiments, the function of at least one endogenous gene encoding a protein *O*-mannosyltransferase (*PMT*) protein has been reduced, disrupted, or deleted. In particular aspects, the *PMT* protein is selected from the group consisting of *PMT*1 and *PMT4.* Thus, the host cell can further include reduction, disruption, or deletion of the *PMT1* or *PMT4* alone or reduction, disruption, or deletion of both the *PMT1* and *PMT4.* Thus, further provided is a *Pichia pastoris* host cell in which the function of (a) an endogenous gene encoding PDI; and (b) at least one endogenous gene encoding a protein *O*-mannosyltransferase (*PMT*) protein; have been reduced, disrupted, or deleted; and a nucleic acid molecule encoding human PDI is expressed in the host cell.

In further embodiments, the host cell further includes a nucleic acid molecule encoding an endogenous or heterologous Ca²⁺ ATPase. In particular aspects, the Ca2+ ATP is selected from the group consisting of the *Pichia pastoris PMR1* and the *Arabidopsis thaliana* ECA1. Thus, further provided is a *Pichia pastoris* host cell in which the function of (a) an endogenous gene encoding PDI has been reduced, disrupted, or deleted; and nucleic acid molecules encoding human PDI and at least one Ca²⁺ ATPase are expressed in the host cell. Further provided is a *Pichia pastoris* cell in which the function of (a) an endogenous gene encoding PDI; and (b) at least one endogenous gene encoding a protein *O*-mannosyltransferase *(PMT)* protein; have been reduced, disrupted, or deleted; and nucleic acid molecules encoding human PDI and at least one Ca²⁺ ATPase are expressed in the host cell.

In still further aspects, the host cell further includes a nucleic acid molecule encoding the human ERp57 chaparone protein or a nucleic acid molecule encoding a calreticulin (CRT) protein, or both. In particular aspects, the calreticulin protein is the human CRT and the ERp57 is the human ERp57. Thus, further provided is a *Pichia pastoris* host cell in which the function of (a) an endogenous gene encoding PDI has been reduced, disrupted, or deleted; and nucleic acid molecules encoding human PDI and at least one of CRT or ERp57 are expressed in the host cell. Further provided is a *Pichia pastoris* host cell in which the function of (a) an endogenous gene encoding PDI has been reduced, disrupted, or deleted; and nucleic acid molecules encoding human PDI, at least one of CRT or ERp57, and at least one Ca²⁺ ATPase are expressed in the host cell. Further provided is a *Pichia pastoris* host cell in which the function of (a) an endogenous gene encoding PDI; and (b) at least one endogenous gene encoding a protein O-mannosyltransferase (*PMT*) protein; have been reduced, disrupted, or deleted; and nucleic acid molecules encoding human PDI, at least one of CRT or ERp57, and at least one Ca²⁺ ATPase are expressed in the host cell.

Thus, provided is a method for producing a recombinant protein comprising: (a) providing a *Pichia pastoris* host cell in which the function of an endogenous gene encoding PDI has been disrupted or deleted and a nucleic acid molecule encoding human PDI is expressed in the host cell; (b) introducing a nucleic acid molecule into the host cell encoding the recombinant protein: and (c) growing the host cell under conditions suitable for producing the recombinant protein.

Further provided is a method for producing a recombinant protein comprising: (a) providing *a Pichia pastoris* host cell in which the function of (a) an endogenous gene encoding PDI; and (b) at least one endogenous gene encoding a protein O-mannosyltransferase *(PMT)* protein; have been reduced, disrupted, or deleted; and a nucleic acid molecule encoding human PDI is expressed in the host cell; (b) introducing a nucleic acid molecule into the host cell encoding the recombinant protein: and (c) growing the host cell under conditions suitable for producing the recombinant protein.

Further provided is a method for producing a recombinant protein comprising: (a) providing a *Pichia pastoris* host cell in which the function of (a) an endogenous gene encoding PDI; and (b) at least one endogenous gene encoding a protein O-mannosyltransferase *(PMT)* protein; have been reduced, disrupted, or deleted; and nucleic acid molecules encoding human PDI and at least one Ca²⁺ ATPase are expressed in the host cell; (b) introducing a nucleic acid molecule into the host cell encoding the recombinant protein: and (c) growing the host cell under conditions suitable for producing the recombinant protein.

Further provided is a method for producing a recombinant protein comprising: (a) providing a *Pichia pastoris* host cell in which the function of (a) an endogenous gene encoding PDI has been reduced, disrupted, or deleted; and nucleic acid molecules encoding human PDI and at least one of CRT or ERp57 are expressed in the host cell; (b) introducing a nucleic acid molecule into the host cell encoding the recombinant protein: and (c) growing the host cell under conditions suitable for producing the recombinant protein.

Further provided is a method for producing a recombinant protein comprising: (a) providing a *Pichia pastoris* host cell in which the function of (a) an endogenous gene encoding PDI has been reduced, disrupted, or deleted; and nucleic acid molecules encoding human PDI, at least one of CRT or ERp57, and at least one Ca²⁺ ATPase are expressed in the host cell; (b) introducing a nucleic acid molecule into the host cell encoding the recombinant protein: and (c) growing the host cell under conditions suitable for producing the recombinant protein.

Further provided is a method for producing a recombinant protein comprising: (a) providing a *Pichia pastoris* host cell in which the function of (a) an endogenous gene encoding PDI; and (b) at least one endogenous gene encoding a protein *O*-mannosyltransferase (*PMT*) protein; have been reduced, disrupted, or deleted; and nucleic acid molecules encoding human PDI, at least one of CRT or ERp57, and at least one Ca²⁺ ATPase are expressed in the host cell; (b) introducing a nucleic acid molecule into the host cell encoding the recombinant protein: and (c) growing the host cell under conditions suitable for producing the recombinant protein.

Further provided is a method for producing a recombinant protein with reduced *O*-glycosylation or *O*-glycan occupancy comprising: (a) providing a *Pichia pastoris* host cell in which the function of an endogenous gene encoding PDI has been disrupted or deleted and a nucleic acid molecule encoding human PDI is expressed in the host cell; (b) introducing a nucleic acid molecule into the host cell encoding the recombinant protein: and (c) growing the host cell under conditions suitable for producing the recombinant protein.

Further provided is a method for producing a recombinant protein with reduced *O*-glycosylation or *O*-glycan occupancy comprising: (a) providing a *Pichia pastoris* host cell in which the function of (a) an endogenous gene encoding human PDI; and (b) at least one endogenous gene encoding a protein *O*-mannosyltransferase (*PMT*) protein; have been reduced, disrupted, or deleted; and a nucleic acid molecule encoding human PDI is expressed in the host cell; (b) introducing a nucleic acid molecule into the host cell encoding the recombinant protein: and (c) growing the host cell under conditions suitable for producing the recombinant protein.

Further provided is a method for producing a recombinant protein with reduced *O*-glycosylation or *O*-glycan occupancy comprising: (a) providing a *Pichia pastoris* host cell in which the function of (a) an endogenous gene encoding PDI; and (b) at least one endogenous gene encoding a protein *O*-mannosyltransferase (*PMT*) protein; have been reduced, disrupted, or deleted; and nucleic acid molecules encoding human PDI and at least one Ca²⁺ A-TPase are expressed in the host cell; (b) introducing a nucleic acid molecule into the host cell encoding the recombinant protein: and (c) growing the host cell under conditions suitable for producing the recombinant protein.

Further provided is a method for producing a recombinant protein with reduced *O*-glycosylation or *O*-glycan occupancy comprising: (a) providing a *Pichia pastoris* host cell in which the function of (a) an endogenous gene encoding PDI has been reduced, disrupted, or deleted; and nucleic acid molecules encoding human PDI and at least one of CRT or ERp57 are expressed in the host cell; (b) introducing a nucleic acid molecule into the host cell encoding the recombinant protein: and (c) growing the host cell under conditions suitable for producing the recombinant protein.

Further provided is a method for producing a recombinant protein with reduced *O*-glycosylation or *O*-glycan occupancy comprising: (a) providing a *Pichia pastoris* host cell in which the function of (a) an endogenous gene encoding PDI has been reduced, disrupted, or deleted; and nucleic acid molecules encoding human PDI, at least one of CRT or ERp57, and at least one Ca²⁺ ATPase are expressed in the host cell; (b) introducing a nucleic acid molecule into the host cell encoding the recombinant protein: and (c) growing the host cell under conditions suitable for producing the recombinant protein.

Further provided is a method for producing a recombinant protein with reduced *O*-glycosylation or *O*-glycan occupancy comprising: (a) providing a *Pichia pastoris* host cell in which the function of (a) an endogenous gene encoding PDI and (b) at least one endogenous gene encoding a protein *O*-mannosyltransferase *(PMT)* protein; have been reduced, disrupted, or deleted; and nucleic acid molecules encoding human PDI, at least one of CRT or ERp57, and at least one Ca²⁺ ATPase are expressed in the host cell; (b) introducing a nucleic acid molecule into the host cell encoding the recombinant protein: and (c) growing the host cell under conditions suitable for producing the recombinant protein.

In particular embodiments, any one of the aforementioned host cells can further include genetic modifications that enable the host cells to produce glycoproteins have predominantly particular *N*-glycan structures thereon or particular mixtures of *N*-glycan structures thereon. For example, the host cells have been genetically engineered to produce *N-*glycans having a Man₃GlcNAc₂ or Man₅GlcNAc₂ core structure, which in particular aspects include one or more additional sugars such as GlcNAc, Galactose, or sialic acid on the non-reducing end, and optionally fucose on the GlcNAc at the reducing end. Thus, the *N*-glycans include both bi-antennary and multi-antennary glycoforms and glycoforms that are bisected. Examples of N-glycans include but are not limited to Man₈GlcNAc₂, Man₇GlcNAc₂, Man₆GlcNAc₂, Man₅GlcNAc₂, GlcNAcMan₅GlcNAc₂, GalGlcNAcMan₅GlcNAc₂, NANAGalGlcNAcMan₅GlcNAc₂, Man₃GlcNAc₂, GlcNAc₍₁₋₄₎Man₃GlcNAc₂, Gal₍₁₋₄₎GlcNAc₍₁₋₄₎Man₃GlcNAc₂, NANA₍₁₋₄₎Gal₍₁₋₄₎GlcNAc₍₁₋₄₎Man₃GlcNAc₂.

### Definitions

Unless otherwise defined herein, scientific and technical terms and phrases used in connection with the present invention shall have the meanings that are commonly understood by those of ordinary skill in the art. Further, unless otherwise required by context, singular terms shall include the plural and plural terms shall include the singular. Generally, nomenclatures used in connection with, and techniques of biochemistry, enzymology, molecular and cellular biology, microbiology, genetics and protein and nucleic acid chemistry and hybridization described herein are those well known and commonly used in the art. The methods and techniques of the present invention are generally performed according to conventional methods well known in the art and as described in various general and more specific references that are cited and discussed throughout the present specification unless otherwise indicated. *See, e.g.,* Sambrook et al. Molecular Cloning: A Laboratory Manual, 2d ed., Cold Spring Harbor Laboratory Press, Cold Spring Harbor, N.Y. (1989); Ausubel et al., Current Protocols in Molecular Biology, Greene Publishing Associates (1992, and Supplements to 2002); Harlow and Lane, Antibodies: A Laboratory Manual, Cold Spring Harbor Laboratory Press, Cold Spring Harbor, N.Y. (1990); Taylor and Drickamer, Introduction to Glycobiology, Oxford Univ. Press (2003); Worthington Enzyme Manual, Worthington Biochemical Corp., Freehold, NJ; Handbook of Biochemistry: Section A Proteins, Vol I, CRC Press (1976); Handbook of Biochemistry: Section A Proteins, Vol II, CRC Press (1976); Essentials of Glycobiology, Cold Spring Harbor Laboratory Press (1999).

The following terms, unless otherwise indicated, shall be understood to have the following meanings:

As used herein, the terms "*N*-glycan" and "glycoform" are used interchangeably and refer to an *N*-linked oligosaccharide, e.g., one that is attached by an asparagine-*N-*acetylglucosamine linkage to an asparagine residue of a polypeptide. *N*-linked glycoproteins contain an *N*-acetylglucosamine residue linked to the amide nitrogen of an asparagine residue in the protein. The predominant sugars found on glycoproteins are glucose, galactose, mannose, fucose, *N*-acelylgalactosamine (GalNAc), *N*-acetylglucosamine (GlcNAc) and sialic acid (e.g., *N-*acetyl-neuraminic acid (NANA)). The processing of the sugar groups occurs cotranslationally in the lumen of the ER and continues in the Golgi apparatus for *N*-linked glycoproteins.

*N*-glycans have a common pentasaccharide core of Man₃GlcNAc₂ ("Man" refers to mannose; "Glc" refers to glucose; and "NAc" refers to *N-*acetyl; GlcNAc refers to *N-*acetylglucosamine). *N*-glycans differ with respect to the number of branches (antennae) comprising peripheral sugars (e.g., GlcNAc, galactose, fucose and sialic acid) that are added to the Man₃GlcNAc₂ ("Man3") core structure which is also referred to as the "trimannose core", the "pentasaccharide core" or the "paucimannose core". *N*-glycans are classified according to their branched constituents (e.g., high mannose, complex or hybrid). A "high mannose" type *N*-glycan has five or more mannose residues. A "complex" type *N*-glycan typically has at least one GlcNAc attached to the 1,3 mannose arm and at least one GlcNAc attached to the 1,6 mannose arm of a "trimannose" core. Complex *N*-glycans may also have galactose ("Gal") or *N-*acetylgalactosamine ("GalNAc") residues that are optionally modified with sialic acid or derivatives (e.g., "NANA" or "NeuAc", where "Neu" refers to neuraminic acid and "Ac" refers to acetyl). Complex *N*-glycans may also have intrachain substitutions comprising "bisecting" GlcNAc and core fucose ("Fuc"). Complex *N*-glycans may also have multiple antennae on the "trimannose core," often referred to as "multiple antennary glycans." A "hybrid" *N*-glycan has at least one GlcNAc on the terminal of the 1,3 mannose arm of the trimannose core and zero or more mannoses on the 1,6 mannose arm of the trimannose core. The various *N*-glycans are also referred to as "glycoforms."

Abbreviations used herein are of common usage in the art, see, e.g., abbreviations of sugars, above. Other common abbreviations include "PNGase", or "glycanase" or "glucosidase" which all refer to peptide *N*-glycosidase F (EC 3.2.2.18).

The term "vector" as used herein is intended to refer to a nucleic acid molecule capable of transporting another nucleic acid molecule to which it has been linked. One type of vector is a "plasmid vector", which refers to a circular double stranded DNA loop into which additional DNA segments may be ligated. Other vectors include cosmids, bacterial artificial chromosomes (BAC) and yeast artificial chromosomes (YAC). Another type of vector is a viral vector, wherein additional DNA segments may be ligated into the viral genome (discussed in more detail below). Certain vectors are capable of autonomous replication in a host cell into which they are introduced (*e.g*., vectors having an origin of replication which functions in the host cell). Other vectors can be integrated into the genome of a host cell upon introduction into the host cell, and are thereby replicated along with the host genome. Moreover, certain preferred vectors are capable of directing the expression of genes to which they are operatively linked. Such vectors are referred to herein as "recombinant expression vectors" (or simply, "expression vectors").

As used herein, the term "sequence of interest" or "gene of interest" refers to a nucleic acid sequence, typically encoding a protein, that is not normally produced in the host cell. The methods disclosed herein allow efficient expression of one or more sequences of interest or genes of interest stably integrated into a host cell genome. Non-limiting examples of sequences of interest include sequences encoding one or more polypeptides having an enzymatic activity, *e.g.,* an enzyme which affects *N-*glycan synthesis in a host such as mannosyltransferases, *N-*acetylglucosaminyltransferases, UDP*-N-*acetylglucosamine transporters, galactosyltransferases, UDP-*N*-acetylgalactosyltransferase, sialyltransferases and fucosyltransferases.

The term "marker sequence" or "marker gene" refers to a nucleic acid sequence capable of expressing an activity that allows either positive or negative selection for the presence or absence of the sequence within a host cell. For example, the *Pichia pastoris URA5* gene is a marker gene because its presence can be selected for by the ability of cells containing the gene to grow in the absence of uracil. Its presence can also be selected against by the inability of cells containing the gene to grow in the presence of 5-FOA. Marker sequences or genes do not necessarily need to display both positive and negative selectability. Non-limiting examples of marker sequences or genes from *Pichia pastoris* include *ADE1, ARG4, HIS4* and *URA3.* For antibiotic resistance marker genes, kanamycin, neomycin, geneticin (or G418), paromomycin and hygromycin resistance genes are commonly used to allow for growth in the presence of these antibiotics.

"Operatively linked" expression control sequences refers to a linkage in which the expression control sequence is contiguous with the gene of interest to control the gene of interest, as well as expression control sequences that act in *trans* or at a distance to control the gene of interest

The term "expression control sequence" or "regulatory sequences" are used interchangeably and as used herein refer to polynucleotide sequences which are necessary to affect the expression of coding sequences to which they are operatively linked. Expression control sequences are sequences which control the transcription, post-transcriptional events and translation of nucleic acid sequences. Expression control sequences include appropriate transcription initiation, termination, promoter and enhancer sequences; efficient RNA processing signals such as splicing and polyadenylation signals; sequences that stabilize cytoplasmic mRNA; sequences that enhance translation efficiency (*e.g*., ribosome binding sites); sequences that enhance protein stability; and when desired, sequences that enhance protein secretion. The nature of such control sequences differs depending upon the host organism; in prokaryotes, such control sequences generally include promoter, ribosomal binding site, and transcription termination sequence. The term "control sequences" is intended to include, at a minimum, all components whose presence is essential for expression, and can also include additional components whose presence is advantageous, for example, leader sequences and fusion partner sequences.

The term "recombinant host cell" ("expression host cell", "expression host system", "expression system" or simply "host cell"), as used herein, is intended to refer to a cell into which a recombinant vector has been introduced. It should be understood that such terms are intended to refer not only to the particular subject cell but to the progeny of such a cell. Because certain modifications may occur in succeeding generations due to either mutation or environmental influences, such progeny may not, in fact, be identical to the parent cell, but are still included within the scope of the term "host cell" as used herein. A recombinant host cell may be an isolated cell or cell line grown in culture or may be a cell which resides in a living tissue or organism.

The term "eukaryotic" refers to a nucleated cell or organism, and includes insect cells, plant cells, mammalian cells, animal cells and lower eukaryotic cells.

The term "lower eukaryotic cells" includes yeast and filamentous fungi.

The function of a gene encoding a protein is said to be 'reduced' when that gene has been modified, for example, by deletion, insertion, mutation or substitution of one or more nucleotides, such that the modified gene encodes a protein which has at least 20% to 50% lower activity, in particular aspects, at least 40% lower activity or at least 50% lower activity, when measured in a standard assay, as compared to the protein encoded by the corresponding gene without such modification. The function of a gene encoding a protein is said to be 'eliminated' when the gene has been modified, for example, by deletion, insertion, mutation or substitution of one or more nucleotides, such that the modified gene encodes a protein which has at least 90% to 99% lower activity, in particular aspects, at least 95% lower activity or at least 99% lower activity, when measured in a standard assay, as compared to the protein encoded by the corresponding gene without such modification.
Unless otherwise defined, all technical and scientific terms used herein have the same meaning as commonly understood by one of ordinary skill in the art to which this invention pertains.
Exemplary methods and materials are described below, although methods and materials similar or equivalent to those described herein can also be used in the practice of the present invention and will be apparent to those of skill in the art. The materials, methods, and examples are illustrative only and not intended to be limiting in any manner.

### BRIEF DESCRIPTION OF THE DRAWINGS

Figure 1 illustrates representative results from deep-well plate screening where human anti-DKK1 antibody is produced in *Pichia pastoris* host cells in which the endogenous *PDI1* gene is expressed (Panel A), both in the presence of the endogenous *PDI1* gene and the human PDI gene (Panel B), and in a cell line expressing the human PDI gene and in which the endogenous *PDI1* gene function has been knocked out (Panel C).
Figure 2 illustrates the action of human PDI and its co-chaperones in thiol-redox reactions in the endoplasmic reticulum.
Figures 3A and 3B show the genealogy of yeast strains described in the examples for illustrating the invention.

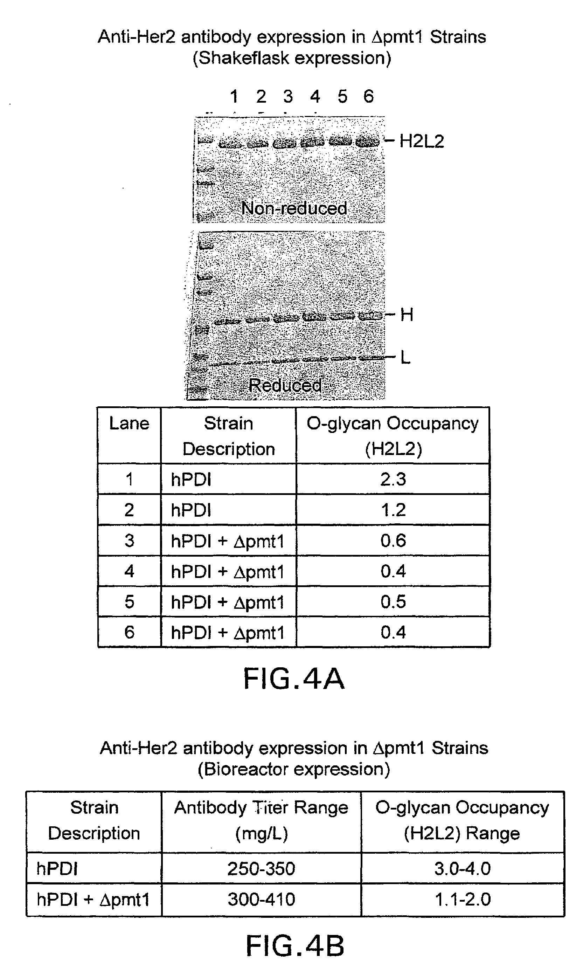

Figures 4A and 4B shows representative results from shakeflask (A) and 0.5L bioreactor (B) expression studies in which human anti-Her2 antibody was produced in *Pichia pastoris* strains in which the human PDI gene (hPDI) replaced the endogenous *PDI1* and strains in which the human PDI replaced the endogenous *PDI1* and the *PMT1* gene is disrupted (hPDI + Δpmt1). Antibodies were recovered and resolved by polyacrylamide gel electrophoresis on non-reducing and reducing polyacrylamide gels. Lanes 1-2 shows antibodies produced from two clones produced from transformation of strain yGLY2696 with plasmid vector pGLY2988 encoding the anti-Her2 antibody and lanes 3-6 shows the antibodies produced from four clones produced from transformation of strain yGLY2696 in which the *PMT1* gene was deleted and with plasmid vector pGLY2988 encoding the anti-Her2 antibody.

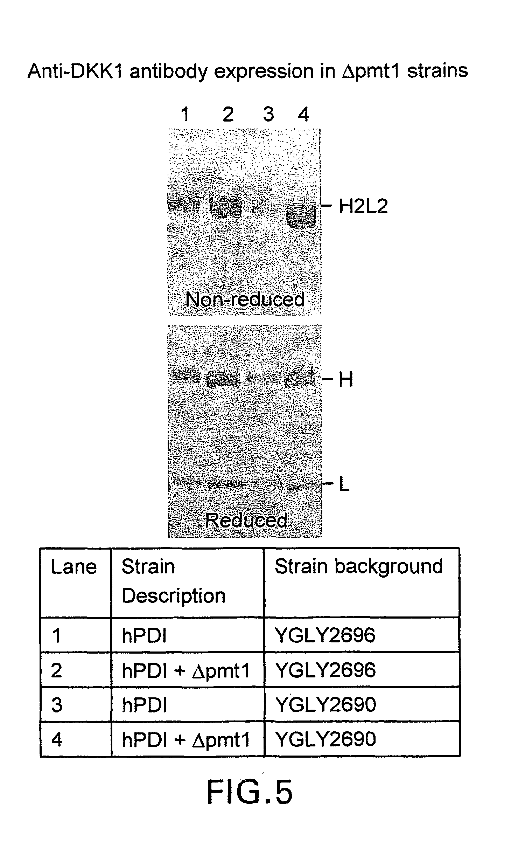

Figure 5 shows representative results from a shakeflask expression study in which human anti-DKK1 antibody was produced in *Pichia pastoris* strains in which the human PDI (hPDI) gene replaced the endogenous *PDI1* and strains in which the human PDI replaced the endogenous *PDI1* and the *PMT1* gene disrupted (hPDI + Δpmt1). Antibodies were recovered and resolved by polyacrylamide gel electrophoresis on non-reducing and reducing polyacrylamide gels. Lanes 1 and 3 shows antibodies produced from two clones produced from transformation of strains yGLY2696 and yGLY2690 with plasmid vector pGLY2260 encoding the anti-DKK1 antibody and lanes 2 and 4 shows the antibodies produced from two clones produced from transformation of strains yGLY2696 and yGLY2690 in which the *PMT1* gene was deleted with plasmid vector pGLY2260 encoding the anti-DKK1 antibody.

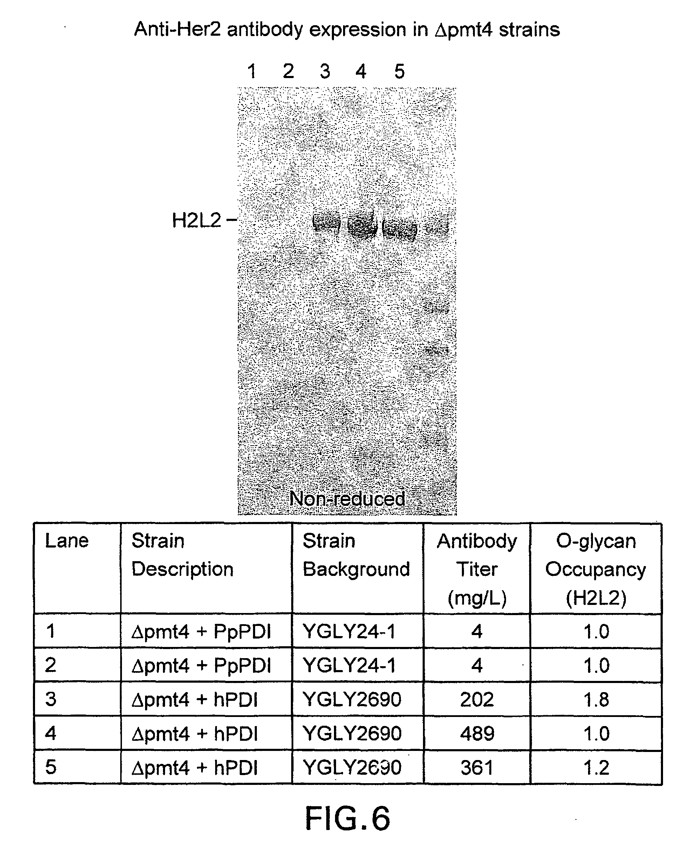

Figure 6 shows results from a 0.5 L bioreactor expression study where human anti-Her2 antibody is produced in *Pichia pastoris* strains in which the human PDI gene (hPD1) replaced the endogenous *PDI1,* strains in which the human PDI replaced the endogenous *PDI1* and the *PMT4* gene disrupted (hPDI + Δpmt4), and strains that express only the endogenous PDI1 but in which the *PMT4* gene is disrupted (PpPD1 + Δpmt4). Antibodies were recovered and resolved by polyacrylamide gel electrophoresis on non-reducing polyacrylamide gels. Lanes 1 and 2 shows antibodies produced from two clones from transformation of strain yGLY24-1 with plasmid vector pGLY2988 encoding the anti-Her2 antibody and lanes 3-5 show anti-Her2 antibodies produced from three clones produced from transformation of strain yGLY2690 in which the *PMT4* gene was deleted.

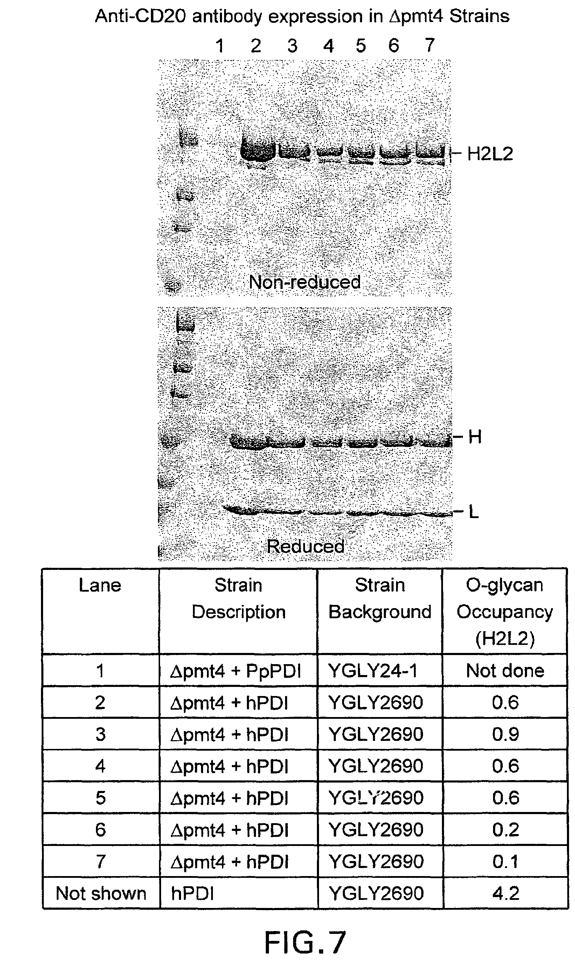

Figure 7 shows results from a shakeflask expression study where human anti-CD20 antibody is produced in *Pichia pastoris* strains in which the human PDI replaced the endogenous *PDI1* and the *PMT4* gene is disrupted (hPDI + Δpmt4) and strains that express only the endogenous PDI1 but in which the *PMT4* gene is disrupted (PpPDI + Δpmt4). Antibodies were recovered and resolved by polyacrylamide gel electrophoresis on non-reducing and reducing polyacrylamide gels. Lane 1 shows antibodies produced from strain yGLY24-1 transformed with plasmid vector pGLY3200 encoding the anti-CD20 antibody; lanes 2-7 show anti-CD20 antibodies produced from six clones produced from transformation of strain yGLY2690 in which the *PMT4* gene was deleted.
Figure 8 illustrates the construction of plasmid vector pGLY642 encoding the human PDI (PDI) and targeting the *Pichia pastoris PDI1* locus.
Figure 9 illustrates the construction of plasmid vector pGLY2232 encoding the human ERO1α (hERO1α) and targeting the *Pichia pastoris PrB1* locus.
Figure 10 illustrates the construction of plasmid vector pGLY2233 encoding the human GRP94 and targeting the *Pichia pastoris PEP4* locus.
Figure 11 illustrates the construction of plasmid vector pGFI207t encoding the T. *reesei* α-1,2 mannosidase (TrMNS1) and mouse α-1,2 mannosidase IA (FB53) and targeting the *Pichia pastoris PRO* locus.
Figure 12 illustrates the construction of plasmid vector pGLY1162 encoding the *T. reesei* α-1,2 mannosidase (TrMNS1) and targeting the *Pichia pastoris PRO* locus.
Figure 13 is maps of plasmid vector pGLY2260 and 2261 encoding the anti-DKK1 antibody heavy chain (GFI710H) and light chain (GFI710L) or two light chains (GFI710L) and targeting the *Pichia pastoris TRP2* locus*.*
Figure 14 is a map of plasmid vector pGLY2012 encoding the anti-ADDL antibody heavy chain (Hc) and light chain (Lc) and targeting the *Pichia pastoris TRP2 locus.*
Figure 15 is a map of plasmid vector pGLY2988 encoding the anti-HER2 antibody (anti-HER2) heavy chain (Hc) and light chain (Lc) and targeting the *Pichia pastoris TRP2* locus.
Figure 16 is a map of plasmid vector pGLY3200 encoding the anti-CD20 antibody heavy chain (Hc) and light chain (Lc) and targeting the *Pichia pastoris TRP2* locus.
Figure 17 is a map of plasmid vector pGLY3822 encoding the *Pichia pastoris PMR1* and targeting the *Pichia pastoris URA6* locus.
Figure 18 is a map of plasmid vector pGLY3827 encoding the *Arabidopsis thaliana* ECA1 (AtECA1) and targeting the *Pichia pastoris URA6* locus.
Figure 19 is a map of plasmid vector pGLY1234 encoding the human CRT (hCRT) and human ERp57(hERp57) and targeting the *Pichia pastoris HIS3* locus.

### DETAILED DESCRIPTION OF THE INVENTION

Molecular chaperones play a critical role in the folding and secretion of antibodies. One chaperone protein in particular, Protein Disulfide Isomerase (PDI), functions to catalyze inter and intra disulphide bond formation that link the antibody heavy and light chains. Protein disulfide isomerase (PDI) can produce a substantial increase or a substantial decrease in the recovery of disulfide-containing proteins, when compared with the uncatalyzed reaction; a high concentration of PDI in the endoplasmic reticulum (ER) is essential for the expression of disulfide-containing proteins [Puig and Gilbert, J. Biol. Chem., 269:7764-7771 (1994)]. Past attempts to increase antibody expression levels in *Pichia pastoris* by overexpressing human PDI chaperone protein and/or overexpressing endogenous PDI1 have been with limited success. We have undertaken humanization of the chaperone pathway in *Pichia pastoris* to explore the possibility of antibody yield improvement through direct genetic engineering.

We have found in a *Pichia pastoris* model that replacement of the yeast gene encoding the endogenous PDI1 protein with an expression cassette encoding a heterologous PDI protein resulted in approximately a five-fold improvement in the yield of recombinant human antibody produced by the recombinant yeast cells as compared to the yield produced by recombinant yeast cells that expressed only the endogenous PDI1 protein and about a three-fold increase in yield compared to the yield produced by recombinant yeast cells that co-expressed the heterologous PDI protein with the endogenous PDI1 protein.

Without being limited to any scientific theory of the mechanism of the invention, it is believed that heterologous recombinant proteins may interact more efficiently with heterologous chaperone proteins than host cell chaperone proteins in the course of their folding and assembly along the secretory pathway. In the case of co-expression, the heterologous chaperone protein may compete with the endogenous chaperone protein for its substrate, i.e., heterologous recombinant proteins. It is further believed that the heterologous PDI protein and recombinant protein be from the same species. Therefore, replacement of the gene encoding PDI with an expression cassette encoding human PDI may be a better means for producing recombinant host cells for producing recombinant proteins than merely co-expressing human PDI with endogenous PDI.

In addition, further improvements in recombinant protein yield may be obtained by overexpressing in the recombinant host cell the heterologous PDI protein and an additional heterologous co-chaperone proteins, such as ERO1α and or the GRP94 proteins. In further aspects, the recombinant host cell can further overexpress FAD, FLC1, and ERp44 proteins. Since these genes are related in function, it may be desirable to include the nucleic acid molecules that encode these genes in a single vector, which transformed into the host cell. Expression of the proteins may be effected by operably linking the nucleic acid molecules encoding the proteins to a heterologous or homologous promoter. In particular aspects, expression of the human PDI may be effected by a homologous promoter such as the KAR2 promoter or a promoter from another ER-specific gene.

As exemplified in the Examples using *Pichia pastoris* as a model, the methods disclosed herein are particularly useful in the production of recombinant human glycoproteins, including antibodies, from *Pichia pastoris* host cells. For example, secretion of recombinant proteins from *Pichia pastoris* proceeds more efficiently as the folding and assembly of the protein of interest is assisted by human PDI, and optionally including other mammalian-derived chaperone proteins, such as ERO1α and GRP94, thereby improving yield. As exemplified in the Examples, the methods herein will especially benefit antibody production in which the heavy and light chains must be properly assembled through disulphide bonds in order to achieve activity.

Thus, the methods herein provide significant advantages with respect to addressing the problem of low productivity in the secretion of recombinant antibodies from *Pichia pastoris.* In the past, yeast, human or mouse chaperone proteins were overexpressed with limited success while the present invention demonstrates that improved productivity of correctly folded and secreted heterologous proteins, such as antibodies, can be obtained through replacement of the host cells' endogenous PDI with human PDI. The overexpression of human PDI, combined with the deletion of the endogenous gene encoding PDI unexpectedly results in improved productivity of glycoproteins, compared with overexpression of human PDI alone.

We further found that host cells, transformed with nucleic acid molecules encoding human PDI as described above, can be further genetically manipulated to improve other characteristics of the recombinant proteins produced therefrom.

*Pichia pastoris* cells contain a family of genes known as protein *O-*mannosyltransferases (*PMTs*) involved in the transfer of mannose to seryl and threonyl residues of secretory proteins. We found that *Pichia pastoris* cell lines, which have been genetically altered to express one or more humanized or chimeric chaperone genes, are better able to tolerate deletion of one or more *PMT* genes, with little or no effect on cell growth or protein expression. *PMT* genes which may be deleted include *PMT1, PMT2, PMT4, PMT5,* and *PMT6.* In general, *Pichia pastoris* host cells in which both the *OCH1* gene and the *PMT* gene is deleted either grow poorly or not at all. Deletion or functional knockout of the *OCH1* gene is necessary for constructing recombinant *Pichia pastoris* host cells that can make human glycoproteins that have human-like *N*-glycans. Because it is desirable to produce human glycoproteins that have no or reduced *O*-glycosylation, there has been a need to find means for reducing *O*-glycosylation in recombinant *Pichia pastoris* host cells that are also capable of producing human glycoproteins with human-like *N*-glycans. We found that *Pichia pastoris* host cells containing one or more chaperone genes as disclosed herein can be further genetically altered to contain a deletion or functional knockout of the *OCH1* gene and a deletion or functional knockout of one or more *PMT* genes, such as *PMT1, PMT4, PMT5,* and/or *PMT6.* These recombinant cells are viable and produce human glycoproteins with human-like *N*-glycans in high yield and with reduced *O-*glycosylation. In addition, a further reduction in *O*-glycosylation was achieved by growing the cells in the presence of a *PMT* protein inhibitor.

As exemplified in the Examples, we demonstrate that the methods disclosed herein are particularly useful in the production of recombinant human glycoproteins, including antibodies, from *Pichia pastoris* with improved properties, since the host cells of the present, invention exhibit tolerance to chemical *PMT* protein inhibitors and/or deletion of *PMT* genes. The Examples show that the recombinant proteins have reduced *O*-glycosylation occupancy and length of *O*-glycans compared with prior lower eukaryotic expression systems. As exemplified in the Examples, the methods herein will especially benefit antibody production in which the heavy and light chains must be properly assembled through disulphide bonds in order to achieve activity and the antibodies must have reduced or no *O*-glycosylation.

We have further found that over-expression of *Pichia pastoris* Golgi Ca²⁺ ATPase (PpPMR1) or *Arabidopsis thaliana* ER Ca²⁺-ATPase (AtECA1) effected about a 2-fold reduction in *O*-glycan occupancy compared to the above strains wherein the endogenous PDI1 had been replaced with the human PDI but which did not express either Ca²⁺ ATPase. Thus, in further embodiments, any one of the host cells disclosed herein can further include one or more nucleic acid molecules encoding an endogenous or exogenous Golgi or ER Ca²⁺ ATPase, wherein the Ca²⁺ ATPase is operably linked to a heterologous promoter. These host cells can be used to produce glycoproteins with reduced *O*-glycosylation.

Calreticulin (CRT) is a multifunctional protein that acts as a major Ca(2+)-binding (storage) protein in the lumen of the endoplasmic reticulum. It is also found in the nucleus, suggesting that it may have a role in transcription regulation. Calreticulin binds to the synthetic peptide KLGFFKR (SEQ ID NO:75), which is almost identical to an amino acid sequence in the DNA-binding domain of the superfamily of nuclear receptors. Calreticulin binds to antibodies in certain sera of systemic lupus and Sjogren patients which contain anti-Ro/SSA antibodies, it is highly conserved among species, and it is located in the endoplasmic and sarcoplasmic reticulum where it may bind calcium. Calreticulin binds to misfolded proteins and prevents them from being exported from the Endoplasmic reticulum to the Golgi apparatus.

ERp57 is a chaperone protein of the endoplasmic reticulum that interacts with lectin chaperones calreticulin and calnexin to modulate folding of newly synthesized glycoproteins. The protein was once thought to be a phospholipase; however, it has been demonstrated that the protein actually has protein disulfide isomerase activity. Thus, the ERp57 is a lumenal protein of the endoplasmic reticulum (ER) and a member of the protein disulfide isomerase (PDI) family. It is thought that complexes of lectins and this protein mediate protein folding by promoting formation of disulfide bonds in their glycoprotein substrates. In contrast to archetypal PDI, ERp57 interacts specifically with newly synthesized glycoproteins.

We have further found that over-expression of the human CRT and human ERp57 in *Pichia pastoris* affected about a one-third reduction in *O*-glycan occupancy compared to strains wherein the endogenous PDI1 had been replaced with the human PDI but which did not express the hCRT and hERp57. Thus, in further embodiments, any one of the host cells herein can further include one or more nucleic acid molecules encoding a calreticulin and an ERp57 protein, each operably linked to a heterologous promoter. These host cells can be used to produce glycoproteins with reduced *O*-glycosylation.

Thus, the methods herein provide significant advantages with respect to addressing the problem of low productivity in the secretion of recombinant antibodies from *Pichia pastoris.* In the past, yeast, human or mouse chaperone proteins were overexpressed with limited success while the present invention demonstrates that improved productivity of correctly folded and secreted heterologous proteins, such as antibodies, can be obtained through replacement of the host cells' endogenous PDI with human PDI. The overexpression of human PDI combined with the deletion of the endogenous gene encoding PDI unexpectedly results in improved productivity of glycoproteins, compared with overexpression of the mammalian-derived protein alone.

Therefore, the present invention provides methods for increasing production of an overexpressed gene product present in a *Pichia pastoris* host cell, which includes expressing human PDI in the host cell in place of endogenous PDI and thereby increasing production of the overexpressed gene product. Also provided is a method of increasing production of an overexpressed gene product from a host cell by disrupting or deleting a gene encoding PDI and expressing a nucleic acid molecule encoding human PDI encoded in an expression vector present in or provided to the host cell, thereby increasing the production of the overexpressed gene product. Further provided is a method for increasing production of overexpressed gene products from a host cell, which comprises expressing human PDI in the host cell in place of endogenous PDI. In the present context, an overexpressed gene product is one which is expressed at levels greater than normal endogenous expression for that gene product.

In one embodiment, the method comprises deleting or disrupting expression of endogenous PDI and effecting the expression of human PDI and an overexpressed gene product in a host cell, and cultivating said host cell under conditions suitable for secretion of the overexpressed gene product. The expression of PDI and the overexpressed gene product can be effected by inducing expression of a nucleic acid molecule encoding PDI and a nucleic acid molecule encoding the overexpressed gene product wherein said nucleic acid molecules are present in a host cell.

In another embodiment, the expression of human PDI and the overexpressed gene product are effected by introducing a first nucleic acid molecule encoding human PDI and a second nucleic acid molecule encoding a gene product to be overexpressed into a host cell in which expression of at least one gene encoding endogenous PDI has been disrupted or deleted under conditions suitable for expression of the first and second nucleic acid molecules. In further aspects, one or both of said first and second nucleic acid molecules are present in expression vectors. In further aspects, one or both of said first and second nucleic acid molecules are present in expression/integration vectors. Expression of the second protein is effected by inducing expression of a nucleic acid molecule encoding the gene product to be overexpressed by introducing a nucleic acid molecule encoding said second gene product into the host cell.

The present invention further provides methods for increasing production of an overexpressed gene product present in a *Pichia pastoris* host cell with reduced *O*-glycosylation, which includes expressing human PDI in the host cell in place of endogenous PDI and wherein the host cell has had one or more genes in the protein *O*-mannosyltransferase *(PMT)* family disrupted or deleted, thereby increasing production of the overexpressed gene product with reduced *O*-glycosylation. Also provided is a method of increasing production of an overexpressed gene product with reduced *O*-glycosylation from a host cell by disrupting or deleting a gene encoding endogenous PDI and a gene encoding a *PMT* and expressing a nucleic acid molecule encoding human PDI encoded in an expression vector present in or provided to the host cell, thereby increasing the production of the overexpressed gene product. Further provided is a method for increasing production of over expressed gene products with reduced *O-*glycosylation from a host cell, which comprises expressing human PDI in the host cell in place of endogenous PDI and wherein at least one *PMT* gene has been disrupted or deleted.

In one embodiment, the method comprises deleting or disrupting expression of endogenous PDI and at least one *PMT* gene and effecting the expression of human PDI and an overexpressed gene product in a host cell, and cultivating said host cell under conditions suitable for secretion of the overexpressed gene product with reduced *O*-glycosylation. The expression of PDI and the overexpressed gene product can be effected by inducing expression of a nucleic acid molecule encoding PDI and a nucleic acid molecule encoding the overexpressed gene product wherein said nucleic acid molecules are present in a host cell.

In another embodiment, the expression of human PDI and the overexpressed gene product are effected by introducing a first nucleic acid molecule encoding human PDI and a second nucleic acid molecule encoding a gene product to be overexpressed into a host cell in which expression of endogenous PDI and at least one *PMT* gene have been disrupted or deleted under conditions suitable for expression of the first and second nucleic acid molecules. In further aspects, one or both of said first and second nucleic acid molecules are present in expression vectors. In further aspects, one or both of said first and second nucleic acid molecules are present in expression/integration vectors. Expression of the second protein is effected by inducing expression of a nucleic acid molecule encoding the gene product to be overexpressed by introducing a nucleic acid molecule encoding said second gene product into the host cell.

Attempts to increase expression levels of heterologous human proteins in yeast cell lines by overexpressing human BiP, using constitutive promoters such as GAPDH, have been largely unsuccessful. Knockouts of *Pichia pastoris KAR2,* the homolog of human BiP, have been harmful to cells. The limitations of the prior art can be overcome by constructing a chimeric BiP gene, in which the human ATPase domain is replaced by the ATPase domain of *Pichia pastoris KAR2,* fused to the human BiP peptide binding domain, under the control of the *KAR2,* or other ER-specific promoter from *Pichia pastoris.* Further improvements in yield may be obtained by combining the replacement of the endogenous PDI1 gene, as described above, with the use of chimeric BiP and human ERdj3 .

In further aspects, the overexpressed gene product is a secreted gene product. Procedures for observing whether an overexpressed gene product is secreted are readily available to the skilled artisan. For example, Goeddel, (Ed.) 1990, Gene Expression Technology, Methods in Enzymology, Vol 185, Academic Press, and Sambrook et al. 1989, Molecular Cloning: A Laboratory Manual, Vols. 1-3, Cold Spring Harbor Press, N.Y., provide procedures for detecting secreted gene products.

To secrete an overexpressed gene product the host cell is cultivated under conditions sufficient for secretion of the overexpressed gene product. Such conditions include temperature, nutrient and cell density conditions that permit secretion by the cell. Moreover, such conditions are conditions under which the cell can perform basic cellular functions of transcription, translation and passage of proteins from one cellular compartment to another and are known to the skilled artisan.

Moreover, as is known to the skilled artisan a secreted gene product can be detected in the culture medium used to maintain or grow the present host cells. The culture medium can be separated from the host cells by known procedures, for example, centrifugation or filtration. The overexpressed gene product can then be detected in the cell-free culture medium by taking advantage of known properties characteristic of the overexpressed gene product. Such properties can include the distinct immunological, enzymatic or physical properties of the overexpressed gene product. For example, if an overexpressed gene product has a unique enzyme activity an assay for that activity can be performed on the culture medium used by the host cells. Moreover, when antibodies reactive against a given overexpressed gene product are available, such antibodies can be used to detect the gene product in any known immunological assay *(See* Harlowe, et al., 1988, Antibodies: A Laboratory Manual, Cold Spring Harbor Laboratory Press)

In addition, a secreted gene product can be a fusion protein wherein the gene product includes a heterologous signal or leader peptide that facilitates the secretion of the gene product. Secretion signal peptides are discrete amino acid sequences, which cause the host cell to direct a gene product through internal and external cellular membranes and into the extracellular environment. Secretion signal peptides are present at the *N*-terminus of a nascent polypeptide gene product targeted for secretion. Additional eukaryotic secretion signals can also be present along the polypeptide chain of the gene product in the form of carbohydrates attached to specific amino acids, i.e. glycosylation secretion signals.

*N*-terminal signal peptides include a hydrophobic domain of about 10 to about 30 amino acids which can be preceded by a short charged domain of about two to about 10 amino acids. Moreover, the signal peptide is present at the N-terminus of gene products destined for secretion. In general, the particular sequence of a signal sequence is not critical but signal sequences are rich in hydrophobic amino acids such as alanine (Ala), valine (Val), leucine (Leu), isoleucine (IIe), proline (Pro), phenylalanine (Phe), tryptophan (Trp), methionine (Met) and the like.

Many signal peptides are known (Michaelis et al., Ann. Rev. Microbiol. 36: 425 (1982). For example, the yeast acid phosphatase, yeast invertase, and the yeast α-factor signal peptides have been attached to heterologous polypeptide coding regions and used successfully for secretion of the heterologous polypeptide (See for example, Sato et al. Gene 83: 355-365 (1989); Chang et al. Mol. Cell. Biol. 6: 1812-1819 (1986); and Brake et al. Proc. Natl. Acad. Sci. USA 81: 4642-4646 (1984). Therefore, the skilled artisan can readily design or obtain a nucleic acid molecule which encodes a coding region for an overexpressed gene product which also has a signal peptide at the 5'-end.

Examples of overexpressed gene products which are preferably secreted by the present methods include mammalian gene products such as enzymes, cytokines, growth factors, hormones, vaccines, antibodies and the like. More particularly, overexpressed gene products include but are not limited to gene products such as erythropoietin, insulin, somatotropin, growth hormone releasing factor, platelet derived growth factor, epidermal growth factor, transforming growth factor α., transforming growth factor β, epidermal growth factor, fibroblast growth factor, nerve growth factor, insulin-like growth factor I, insulin-like growth factor II, clotting Factor VIII, superoxide dismutase, α-interferon, γ-interferon, interleukin-1, interleukin-2, interleukin-3, interleukin-4, interleukin-5, interleukin-6, granulocyte colony stimulating factor, multi-lineage colony stimulating activity, granulocyte-macrophage stimulating factor, macrophage colony stimulating factor, T cell growth factor, lymphotoxin, immunoglobulins, antibodies, and the like. Further included are fusion proteins, including but not limited to, peptides and polypeptides fused to the constant region of an immunoglobulin or antibody. Particularly useful overexpressed gene products are human gene products.

The terms "antibody", "antibodies", and "immunoglobulin(s)" encompass any recombinant monoclonal antibody produced by recombinant DNA technology and further is meant to include humanized and chimeric antibodies.

The present methods can readily be adapted to enhance secretion of any overexpressed gene product which can be used as a vaccine. Overexpressed gene products which can be used as vaccines include any structural, membrane-associated, membrane-bound or secreted gene product of a mammalian pathogen. Mammalian pathogens include viruses, bacteria, single-celled or multi-celled parasites which can infect or attack a mammal. For example, viral vaccines can include vaccines against viruses such as human immunodeficiency virus (HIV), *R. rickettsii,* vaccinia, *Shigella,* poliovirus, adenovirus, influenza, hepatitis A, hepatitis B, dengue virus, Japanese B encephalitis, Varicella zoster, cytomegalovirus, hepatitis A, rotavirus, as well as vaccines against viral diseases like Lyme disease, measles, yellow fever, mumps, rabies, herpes, influenza, parainfluenza and the like. Bacterial vaccines can include vaccines against bacteria such as *Vibrio cholerae, Salmonella typhi, Bordetella pertussis, Streptococcus pneumoniae, Hemophilus influenza, Clostridium tetani, Corynebacterium diphtheriae, Mycobacterium leprae, Neisseria gonorrhoea, Neisseria meningitidis, Coccidioides immitis,* and the like.

The overexpressed gene products and human PDI of the present invention are expressed recombinantly, that is, by placing a nucleic acid molecule encoding a gene product or a chaperone protein into an expression vector. Such an expression vector minimally contains a sequence which effects expression of the gene product or human PDI when the sequence is operably linked to a nucleic acid molecule encoding the gene product or PDI. Such an expression vector can also contain additional elements like origins of replication, selectable markers, transcription or termination signals, centromeres, autonomous replication sequences, and the like.

According to the present invention, first and second nucleic acid molecules encoding an overexpressed gene product and human PDI, respectively, can be placed within expression vectors to permit regulated expression of the overexpressed gene product and/or the heterologous chaperone protein. While human PDI and the overexpressed gene product can be encoded in the same expression vector, the human PDI is preferably encoded in an expression vector which is separate from the vector encoding the overexpressed gene product. Placement of nucleic acid molecules encoding the human PDI and the overexpressed gene product in separate expression vectors can increase the amount of secreted overexpressed gene product.

As used herein, an expression vector can be a replicable or a non-replicable expression vector. A replicable expression vector can replicate either independently of host cell chromosomal DNA or because such a vector has integrated into host cell chromosomal DNA. Upon integration into host cell chromosomal DNA such an expression vector can lose some structural elements but retains the nucleic acid molecule encoding the gene product or the chaperone protein and a segment which can effect expression of the gene product or the heterologous chaperone protein. Therefore, the expression vectors of the present invention can be chromosomally integrating or chromosomally nonintegrating expression vectors.

Human PDI is overexpressed in a host cell by introduction of integrating or nonintegrating expression vectors into the host cell. Following introduction of at least one expression vector encoding human PDI, the gene product is then overexpressed by inducing expression of an endogenous gene encoding the gene product, or by introducing into the host cell an expression vector encoding the gene product. In another embodiment, cell lines are established which constitutively or inducibly express human PDI. An expression vector encoding the gene product to be overexpressed is introduced into such cell lines to achieve increased secretion of the overexpressed gene product.

The present expression vectors can be replicable in one host cell type, e.g., *Escherichia coli,* and undergo little or no replication in another host cell type, e.g., a eukaryotic host cell, so long as an expression vector permits expression of human PDI or overexpressed gene products and thereby facilitates secretion of such gene products in *Pichia pastoris.*

Expression vectors as described herein include DNA or RNA molecules engineered for controlled expression of a desired gene, that is, a gene encoding PDI or a overexpressed gene product. Such vectors also encode nucleic acid molecule segments which are operably linked to nucleic acid molecules encoding PDI or the present overexpressed gene products. Operably linked in this context means that such segments can effect expression of nucleic acid molecules encoding PDI or overexpressed gene products. These nucleic acid sequences include promoters, enhancers, upstream control elements, transcription factors or repressor binding sites, termination signals and other elements which can control gene expression in the contemplated host cell. Preferably the vectors are vectors, bacteriophages, cosmids, or viruses.

Expression vectors of the present invention function in yeast or mammalian cells. Yeast vectors can include the yeast 2µ circle and derivatives thereof, yeast vectors encoding yeast autonomous replication sequences, yeast minichromosomes, any yeast integrating vector and the like. A comprehensive listing of many types of yeast vectors is provided in Parent et al. (Yeast 1: 83-138 (1985)).

Elements or nucleic acid sequences capable of effecting expression of a gene product include promoters, enhancer elements, upstream activating sequences, transcription termination signals and polyadenylation sites. All such promoter and transcriptional regulatory elements, singly or in combination, are contemplated for use in the present expression vectors. Moreover, genetically-engineered and mutated regulatory sequences are also contemplated herein.

Promoters are DNA sequence elements for controlling gene expression. In particular, promoters specify transcription initiation sites and can include a TATA box and upstream promoter elements. The promoters selected are those which would be expected to be operable in the particular host system selected. Yeast promoters are used in the present expression vectors when *Pichia pastoris* is used Examples of yeast promoters include but are not limited to the GAPDH, AOX1, GAL1, PGK, GAP, TPI, CYC1, ADH2, PHO5, CUP1, MFα1, PMA1, PDI, TEF, and GUT1 promoters. Romanos et al. (Yeast 8: 423-488 (1992)) provide a review of yeast promoters and expression vectors.

The promoters that are operably linked to the nucleic acid molecules disclosed herein can be constitutive promoters or inducible promoters. Inducible promoters, that is. promoters which direct transcription at an increased or decreased rate upon binding of a transcription factor. Transcription factors as used herein include any factor that can bind to a regulatory or control region of a promoter an thereby affect transcription. The synthesis or the promoter binding ability of a transcription factor within the host cell can be controlled by exposing the host to an inducer or removing an inducer from the host cell medium. Accordingly to regulate expression of an inducible promoter, an inducer is added or removed from the growth medium of the host cell. Such inducers can include sugars, phosphate, alcohol, metal ions, hormones, heat, cold and the like. For example, commonly used inducers in yeast are glucose, galactose, and the like.

Transcription termination sequences that are selected are those that are operable in the particular host cell selected. Yeast transcription termination sequences are used in the present expression vectors. Transcription termination sequences include but are not limited to the *Pichia pastoris ALG3* transcription termination sequence (ALG3 TT), and *Pichia pastoris PMA1* transcription termination sequence (PpPMA1 TT).

The expression vectors of the present invention can also encode selectable markers. Selectable markers are genetic functions that confer an identifiable trait upon a host cell so that cells transformed with a vector carrying the selectable marker can be distinguished from non-transformed cells. Inclusion of a selectable marker into a vector can also be used to ensure that genetic functions linked to the marker are retained in the host cell population. Such selectable markers can confer any easily identified dominant trait, e.g. drug resistance, the ability to synthesize or metabolize cellular nutrients and the like.

Yeast selectable markers include drug resistance markers and genetic functions which allow the yeast host cell to synthesize essential cellular nutrients, e.g. amino acids. Drug resistance markers which are commonly used in yeast include chloramphenicol, kanamycin, methotrexate, G418 (geneticin), Zeocin, and the like. Genetic functions which allow the yeast host cell to synthesize essential cellular nutrients are used with available yeast strains having auxotrophic mutations in the corresponding genomic function. Common yeast selectable markers provide genetic functions for synthesizing leucine (*LEU2*), tryptophan (*TRP1* and *TRP2*), uracil (*URA3, URA5, URA6*), histidine (*HIS3*), lysine (*LYS2*), adenine (*ADE1* or *ADE2*), and the like. Other yeast selectable markers include the *ARR3* gene from *S. cerevisiae,* which confers arsenite resistance to yeast cells that are grown in the presence of arsenite (Bobrowicz et al., Yeast, 13:819-828 (1997); Wysocki et al., J. Biol. Chem. 272:30061-066 (1997)). A number of suitable integration sites include those enumerated in U.S. Published application No. 20070072262 and include homologs to loci known for *Saccharomyces cerevisiae* and other yeast or fungi.

Therefore the present expression vectors can encode selectable markers which are useful for identifying and maintaining vector-containing host cells within a cell population present in culture. In some circumstances selectable markers can also be used to amplify the copy number of the expression vector. After inducing transcription from the present expression vectors to produce an RNA encoding an overexpressed gene product or human PDI, the RNA is translated by cellular factors to produce the gene product or human PDI.

In yeast and other eukaryotes, translation of a messenger RNA (mRNA) is initiated by ribosomal binding to the 5' cap of the mRNA and migration of the ribosome along the mRNA to the first AUG start codon where polypeptide synthesis can begin. Expression in yeast generally does not require specific number of nucleotides between a ribosomal-binding site and an initiation codon, as is sometimes required in prokaryotic expression systems. However, for expression in a yeast host cell, the first AUG codon in an mRNA is preferably the desired translational start codon.

Moreover, when expression is performed in a yeast host cell the presence of long untranslated leader sequences, e.g. longer than 50-100 nucleotides, can diminish translation of an mRNA. Yeast mRNA leader sequences have an average length of about 50 nucleotides, are rich in adenine, have little secondary structure and almost always use the first AUG for initiation. Since leader sequences which do not have these characteristics can decrease the efficiency of protein translation, yeast leader sequences are preferably used for expression of an overexpressed gene product or a chaperone protein in a yeast host cell. The sequences of many yeast leader sequences are known and are available to the skilled artisan, for example, by reference to Cigan et al. (Gene 59: 1-18 (1987)).

In addition to the promoter, the ribosomal-binding site and the position of the start codon, factors which can effect the level of expression obtained include the copy number of a replicable expression vector. The copy number of a vector is generally determined by the vector's origin of replication and any cis-acting control elements associated therewith. For example, an increase in copy number of a yeast episomal vector encoding a regulated centromere can be achieved by inducing transcription from a promoter which is closely juxtaposed to the centromere. Moreover, encoding the yeast FLP function in a yeast vector can also increase the copy number of the vector.

One skilled in the art can also readily design and make expression vectors which include the above-described sequences by combining DNA fragments from available vectors, by synthesizing nucleic acid molecules encoding such regulatory elements or by cloning and pacing new regulatory elements into the present vectors. Methods for making expression vectors are well-known. Overexpressed DNA methods are found in any of the myriad of standard laboratory manuals on genetic engineering.

The expression vectors of the present invention can be made by ligating human PDI coding regions in the proper orientation to the promoter and other sequence elements being used to control gene expression. After construction of the present expression vectors, such vectors are transformed into host cells where the overexpressed gene product and human PDI can be expressed. Methods for transforming yeast and other lower eukaryotic cells with expression vectors are well known and readily available to the skilled artisan. For example, expression vectors can be transformed into yeast cells by any of several procedures including lithium acetate, spheroplast, electroporation, and similar procedures.

In general, useful mutant strains of yeast include strains which have a genetic deficiency that can be used in combination with a yeast vector encoding a selectable marker. Many types of yeast strains are available from the Yeast Genetics Stock Center (Donner Laboratory, University of California, Berkeley, Calif. 94720), the American Type Culture Collection (12301 Parklawn Drive, Rockville, Md. 20852, hereinafter ATCC), the National Collection of Yeast Cultures (Food Research Institute, Colney Lane, Norwich NR4 7UA, UK) and the Centraalbureau voor Schimmelcultures (Yeast Division, Julianalaan 67a, 2628 BC Delft, Netherlands).

*Pichia pastoris* is useful for expression of glycoproteins because they can be economically cultured, give high yields, and when appropriately modified are capable of suitable glycosylation. *Pichia pastoris* particularly offers established genetics allowing for rapid transformations, tested protein localization strategies and facile gene knock-out techniques. Suitable vectors have expression control sequences, such as promoters, including 3-phosphoglycerate kinase or other glycolytic enzymes, and an origin of replication, termination sequences and the like as desired.

*Pichia pastoris is* useful for cell culture because they are able to grow to high cell densities and secrete large quantities of recombinant protein.

*Pichia pastoris* can be genetically modified so that they express glycoproteins in which the glycosylation pattern is human-like or humanized. Such can be achieved by eliminating selected endogenous glycosylation enzymes and/or supplying exogenous enzymes as described by Gerngross et al., US 20040018590. For example, a host cell can be selected or engineered to be depleted in 1,6-mannosyl transferase activities, which would otherwise add mannose residues onto the *N*-glycan on a glycoprotein.

In one embodiment, the host cell further includes an α1,2-mannosidase catalytic domain fused to a cellular targeting signal peptide not normally associated with the catalytic domain and selected to target the α1,2-mannosidase activity to the ER or Golgi apparatus of the host cell. Passage of a recombinant glycoprotein through the ER or Golgi apparatus of the host cell produces a recombinant glycoprotein comprising a Man₅GlcNAc₂ glycoform, for example, a recombinant glycoprotein composition comprising predominantly a Man₅GlcNAc₂ glycoform. For example, U.S. Patent No, 7,029,872 and U.S. Published Patent Application Nos. 2004/0018590 and 2005/0170452 disclose lower eukaryote host cells capable of producing a glycoprotein comprising a Man₅GlcNAc₂ glycoform.

In a further embodiment, the immediately preceding host cell further includes a GlcNAc transferase I (GnT I) catalytic domain fused to a cellular targeting signal peptide not normally associated with the catalytic domain and selected to target GlcNAc transferase I activity to the ER or Golgi apparatus of the host cell. Passage of the recombinant glycoprotein through the ER or Golgi apparatus of the host cell produces a recombinant glycoprotein comprising a GIcNAcMan₃GlcNAc₂ glycoform, for example a recombinant glycoprotein composition comprising predominantly a GlcNAcMan₃GlcNAc₂ glycoform. U.S. Patent No, 7,029,872 and U.S. Published Patent Application No. 2004/0230042 discloses lower eukaryote host cells that express mannosidase II enzymes and are capable of producing glycoproteins having predominantly a GlcNAc₂Man₃GlcNAc₂ glycoform. The glycoprotein produced in the above cells can be treated *in vitro* with a hexaminidase to produce a recombinant glycoprotein comprising a Man₃GlcNAc₂ glycoform.

In a further embodiment, the immediately preceding host cell further includes GlcNAc transferase D (GnT II) catalytic domain fused to a cellular targeting signal peptide not normally associated with the catalytic domain and selected to target GIcNAc transferase II activity to the ER or Golgi apparatus of the host cell. Passage of the recombinant glycoprotein through the ER or Golgi apparatus of the host cell produces a recombinant glycoprotein comprising a GlcNAc₂Man₃GlcNAc₂ glycoform, for example a recombinant glycoprotein composition comprising predominantly a GlcNAc₂Man₃GlcNAc₂ glycoform. U.S. Patent No, 7,029,872 and U.S. Published Patent Application Nos. 2004/0018590 and 2005/0170452 disclose lower eukaryote host cells capable of producing a glycoprotein comprising a GlcNAc₂Man₃GlcNAc₂ glycoform. The glycoprotein produced in the above cells can be treated *in vitro* with a hexaminidase to produce a recombinant glycoprotein comprising a Man₃GlcNAc₂ glycoform.

In a further embodiment, the immediately preceding host cell further includes a galactosyltransferase catalytic domain fused to a cellular targeting signal peptide not normally associated with the catalytic domain and selected to target galactosyltransferase activity to the ER or Golgi apparatus of the host cell. Passage of the recombinant glycoprotein through the ER or Golgi apparatus of the host cell produces a recombinant glycoprotein comprising a GalGlcNAc₂Man₃GlcNAc₂ or Gal₂GlcNAc₂Man₃GlcNAc₂ glycoform, or mixture thereof for example a recombinant glycoprotein composition comprising predominantly a GalGlcNAc₂Man₃GlcNAc₂ glycoform or Gal₂GlcNAc₂Man₃GleNAc₂ glycoform or mixture thereof. U.S. Patent No, 7,029,872 and U.S. Published Patent Application No. 2006/0040353 discloses lower eukaryote host cells capable of producing a glycoprotein comprising a Gal₂GlcNAc₂Man₃GlcNAc₂ glycoform. The glycoprotein produced in the above cells can be treated *in vitro* with a galactosidase to produce a recombinant glycoprotein comprising a GlcNAc₂Man₃GlcNAc₂ glycoform, for example a recombinant glycoprotein composition comprising predominantly a GlcNAc₂Man₃GlcNAc₂ glycoform.

In a further embodiment, the immediately preceding host cell further includes a sialyltransferase catalytic domain fused to a cellular targeting signal peptide not normally associated with the catalytic domain and selected to target sialytransferase activity to the ER or Golgi apparatus of the host cell. Passage of the recombinant glycoprotein through the ER or Golgi apparatus of the host cell produces a recombinant glycoprotein comprising predominantly a NANA₂Gal₂GlcNAc₂Man₃GlcNAc₂ glycoform or NANAGal₂GlcNAc₂Man₃GlcNAc₂ glycoform or mixture thereof. For lower eukaryote host cells such as yeast and filamentous fungi, it is useful that the host cell further include a means for providing CMP-sialic acid for transfer to the *N*-glycan. U.S. Published Patent Application No. 2005/0260729 discloses a method for genetically engineering lower eukaryotes to have a CMP-sialic acid synthesis pathway and U.S. Published Patent Application No. 2006/0286637 discloses a method for genetically engineering lower eukaryotes to produce sialylated glycoproteins. The glycoprotein produced in the above cells can be treated *in vitro* with a neuraminidase to produce a recombinant glycoprotein comprising predominantly a Gal₂GlcNAc₂Man₃GlcNAc₂ glycoform or GalGlcNAc₂Man₃GlcNAc2 glycoform or mixture thereof.

Any one of the preceding host cells can further include one or more GlcNAc transferase selected from the group consisting of GnT III, GnT IV, GnT V, GnT VI, and GnT IX to produce glycoproteins having bisected (GnT III) and/or multiantennary (GnT IV, V, VI, and IX) *N*-glycan structures such as disclosed in U.S. Published Patent Application Nos. 2004/074458 and 2007/0037248.

In further embodiments, the host cell that produces glycoproteins that have predominantly GlcNAcMan₅GlcNAc₂ *N-*glycans further includes a galactosyltransferase catalytic domain fused to a cellular targeting signal peptide not normally associated with the catalytic domain and selected to target Galactosyltransferase activity to the ER or Golgi apparatus of the host cell. Passage of the recombinant glycoprotein through the ER or Golgi apparatus of the host cell produces a recombinant glycoprotein comprising predominantly the GalGlcNAcMan₅GlcNAc₂ glycoform.

In a further embodiment, the immediately preceding host cell that produced glycoproteins that have predominantly the predominantly the GalGlcNAcMan₅GlcNAc₂ *N-*glycans further includes a sialyltransferase catalytic domain fused to a cellular targeting signal peptide not normally associated with the catalytic domain and selected to target sialytransferase activity to the ER or Golgi apparatus of the host cell. Passage of the recombinant glycoprotein through the ER or Golgi apparatus of the host cell produces a recombinant glycoprotein comprising a NANAGalGlcNAcMan₅GlcNAc₂ glycoform.

Various of the preceding host cells further include one or more sugar transporters such as UDP-GlcNAc transporters (for example, *Kluyveromyces lactis* and *Mus musculus* UDP-GlcNAc transporters), UDP-galactose transporters (for example, *Drosophila melanogaster* UDP-galactose transporter), and CMP-sialic acid transporter (for example, human sialic acid transporter). Because *Pichia pastoris* lacks the above transporters, it is preferable that *Pichia pastoris* host cells be genetically engineered to include the above transporters.

In further embodiments of the above host cells, the host cells are further genetically engineered to eliminate glycoproteins having α-mannosidase-resistant *N*-glycans by deleting or disrupting the β-mannosyltransferase gene (*BMT2*)(*See,* U.S. Published Patent Application No. 2006/0211085) and glycoproteins having phosphomannose residues by deleting or disrupting one or both of the phosphomannosyl transferase genes *PNO1* and *MNN4B (See* for example, U.S. Patent Nos. 7,198,921 and 7,259,007). In further still embodiments of the above host cells, the host cells are further genetically modified to eliminate *O*-glycosylation of the glycoprotein by deleting or disrupting one or more of the protein *O*-mannosyltransferase (Dol-P-Man:Protein (Ser/Thr) Mannosyl Transferase genes) (*PMTs*) (*See* U.S. Patent No. 5,714,377) or grown in the presence of i inhibitors such as Pmt-1, Pmti-2, and Pmti-3 as disclosed in Published International Application No. WO 2007061631, or both.

Thus, provided are host cells that have been genetically modified to produce glycoproteins wherein the predominant *N*-glycans thereon include but are not limited to Man₈GlcNAc₂, Man₇GlcNAc₂, Man₆GlcNAc₂, Man₅GleNAc₂, GlcNAcMan₅GlcNAc₂, GalGlcNAcMan₅GlcNAc₂, NANAGalGlcNAcMan₅GlcNAc₂, Man₃GlcNAc₂, GlcNAc(1-₄)Man₃GlcNAc₂, Gal₍₁₋₄₎GlcNAc₍₁₋₄₎Man₃GlcNAc₂, NANA₍₁₋₄₎Gal₍₁₋₄₎GlcNAc₍₁₋₄₎Man₃GlcNAc₂. Further included are host cells that produce glycoproteins that have particular mixtures of the aforementioned *N-*glycans thereon.

In the following examples, heterologous human proteins are expressed in host cells of the species *Pichia pastoris.* These examples demonstrate the invention with respect to specific embodiments of the invention, and are not to be construed as limiting in any manner. The skilled artisan, having read the disclosure and examples herein, will recognize that numerous variants, modifications and improvements to the methods and materials described that are possible without deviating from the practice of the present invention.

### EXAMPLE 1

This example shows that expression of heterologous human proteins in *Pichia pastoris* was enhanced by using host cells in which the gene encoding the endogenous PDI1 has been inactivated and replaced with an expression cassette encoding the human PDI. The example further shows that these host cells produced recombinant antibodies that had reduced O-glycosylation.

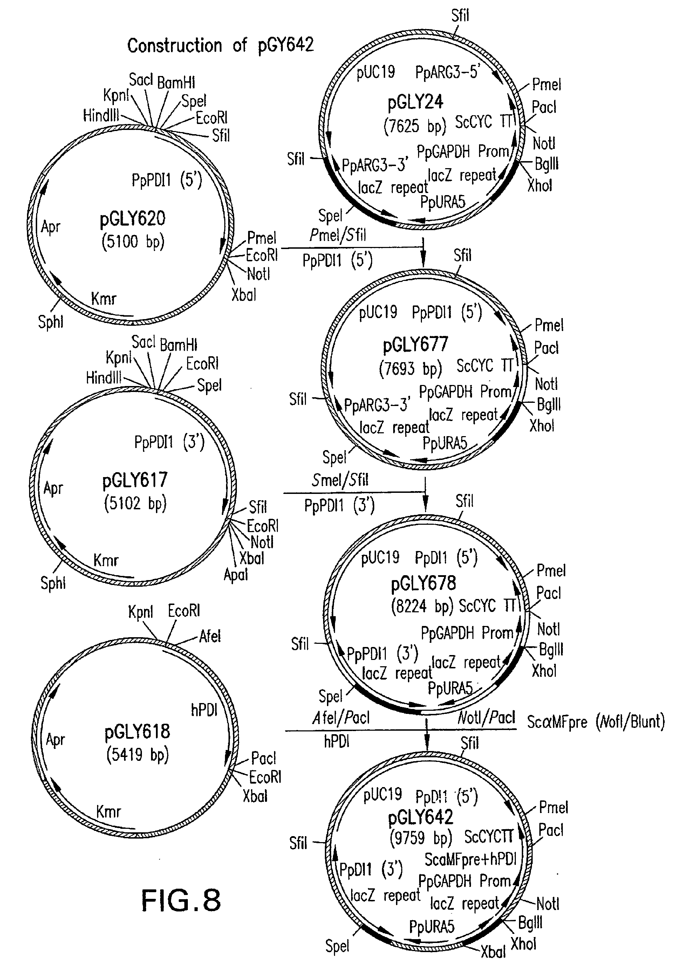

Construction of expression/integration plasmid vector pGLY642 comprising an expression cassette encoding the human PDI protein and nucleic acid molecules to target the plasmid vector to the *Pichia pastoris PDI1* locus for replacement of the gene encoding the *Pichia pastoris PDI1* with a nucleic acid molecule encoding the human PDI was as follows and is shown in Figure 8. cDNA encoding the human PDI was amplified by PCR using the primers hPDI/UP1: 5' AGCGCTGACGCCCCCGAGGAGGAGGACCAC 3' (SEQ ID NO: 1) and hPDI/LP-PacI: 5' CCTTAATTAATTACAGTTCATCATGCACAGCTTTCTGATCAT 3' (SEQ ID NO: 2), Pfu turbo DNA polymerase (Stratagene, La Jolla, CA), and a human liver cDNA (BD Bioscience, San Jose, CA). The PCR conditions were 1 cycle of 95°C for two minutes, 25 cycles of 95°C for 20 seconds, 58°C for 30 seconds, and 72°C for 1.5 minutes, and followed by one cycle of 72°C for 10 minutes. The resulting PCR product was cloned into plasmid vector pCR2.1 to make plasmid vector pGLY618. The nucleotide and amino acid sequences of the human PDI (SEQ ID NOs: 39 and 40, respectively) are shown in Table 11.

The nucleotide and amino acid sequences of the *Pichia pastoris* PDI1 (SEQ ID NOs:41 and 42, respectively) are shown in Table 11. Isolation of nucleic acid molecules comprising the *Pichia pastoris PDI1* 5' and 3' regions was performed by PCR amplification of the regions from *Pichia pastoris* genomic DNA. The 5' region was amplified using primers PB248: 5' ATGAATTCAGGCCATATCGGCCATTGTTTACTGTGCGCCCACAGTAG 3' (SEQ ID NO: 3); PB249: 5' ATGTTTAAACGTGAGGATTACTGGTGATGAAAGAC 3' (SEQ ID NO: 4). The 3' region was amplified using primers PB250: 5' AGACTAGTCTATTTG GAGACATTGACGGATCCAC 3' (SEQ ID NO: 5); PB251: 5' ATCTCGAGAGGCCAT GCAGGCCAACCACAAGATGAATCAAATTTTG-3' (SEQ ID NO: 6). *Pichia pastoris* strain NRRL-Y11430 genomic DNA was used for PCR amplification. The PCR conditions were one cycle of 95°C for two minutes, 25 cycles of 95°C for 30 seconds, 55°C for 30 seconds, and 72°C for 2.5 minutes, and followed by one cycle of 72°C for 10 minutes. The resulting PCR fragments, P_{P}PDI1 (5') and PpPDI1 (3'), were separately cloned into plasmid vector pCR2.1 to make plasmid vectors pGLY620 and pGLY617, respectively. To construct pGLY678, DNA fragments PpARG3-5' and PpARG-3' of integration plasmid vector pGLY24, which targets the plasmid vector to *Pichia pastoris ARG3* locus, were replaced with DNA fragments P_{P}PDI (5') and P_{P}PDI (3'), respectively, which targets the plasmid vector pGLY678 to the *PDI1 locus* and disrupts expression of the *PDI1* locus.

The nucleic acid molecule encoding the human PDI was then cloned into plasmid vector pGLY678 to produce plasmid vector pGLY642 in which the nucleic acid molecule encoding the human PDI was placed under the control of the *Pichia pastoris* GAPDH promoter (PpGAPDH). Expression/integration plasmid vector pGLY642 was constructed by ligating a nucleic acid molecule (SEQ ID NO: 27) encoding the *Saccharomyces cerevisiae* alpha mating factor pre-signal peptide (ScaMFpre-signal peptide (SEQ ID NO: 28) having a *Not*I restriction enzyme site at the 5' end and a blunt 3' end and the expression cassette comprising the nucleic acid molecule encoding the human PDI released from plasmid vector pGLY618 with *Afe*I and *Pac*I to produce a nucleic acid molecule having a blunt 5' end and a *Pac*I site at the 3' end into plasmid vector pGLY678 digested with *Not*I and *Pac*I*.* The resulting integration/expression plasmid vector pGLY642 comprises an expression cassette encoding a human PDI/ ScαMFpre-signal peptide fusion protein operably linked to the *Pichia pastoris* promoter and nucleic acid molecule sequences to target the plasmid vector to the *Pichia pastoris PDI1 locus* for disruption of the *PDI1 locus* and integration of the expression cassette into the *PDI1 locus.* Figure 8 illustrates the construction of plasmid vector pGLY642. The nucleotide and amino acid sequences of the ScαMFpre-signal peptide are shown in SEQ ID NOs: 27 and 28, respectively.

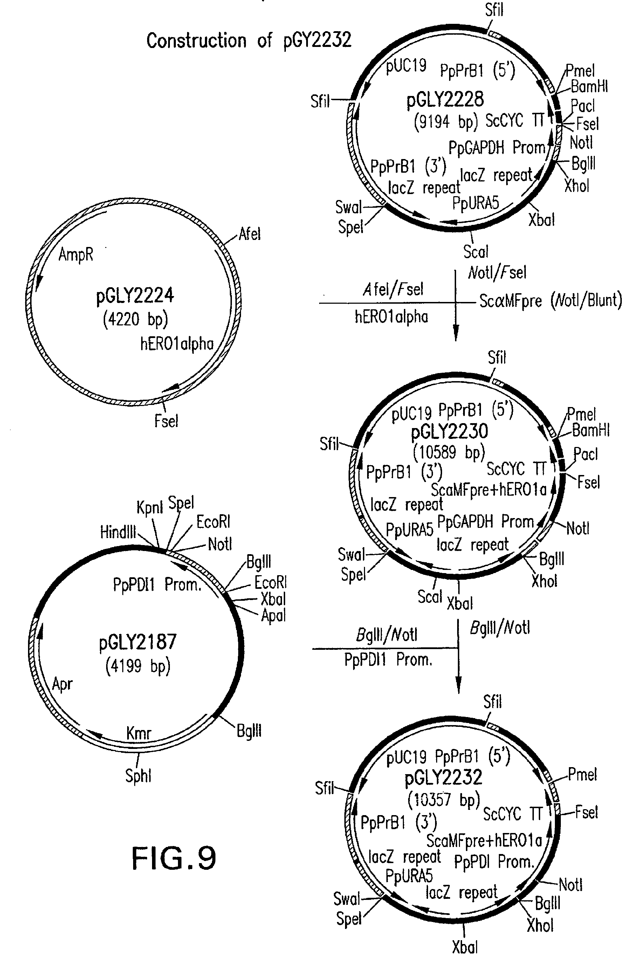

Construction of expression/integration vector pGLY2232 encoding the human ERO1α protein was as follows and is shown in Figure 9. A nucleic acid molecule encoding the human ERO1α protein was synthesized by GeneArt AG (Regensburg, Germany) and used to construct plasmid vector pGLY2224. The nucleotide and amino acid sequences of the human ERO1α protein (SEQ ID NOs: 43 and 44, respectively) are shown in Table 11. The nucleic acid molecule encoding the human ERO1α protein was released from the plasmid vector using restriction enzymes *Afe*I and *Fse*I and then ligated with a nucleic acid molecule encoding the ScαMPpre-signal peptide with *5' Not*I and 3' blunt ends as above into plasmid vector pGLY2228 digested with *Not*I and *Fse*I. Plasmid vector pGLY2228 also included nucleic acid molecules that included the 5' and 3' regions of the *Pichia pastoris PRB1* gene (PpPRB1-5' and PpPRB1-3' regions, respectively). The resulting plasmid vector, pGLY2230 was digested with *Bgl*II and *Not*I and then ligated with a nucleic acid molecule containing the *Pichia partoris PDI1* promoter (PpPDI promoter) which had been obtained from plasmid vector pGLY2187 digested with *Bg*lII and *Not*I. The nucleotide sequence of the PpPDI promoter is 5'-AACACGAACACTGTAAAT AGAATAAAAGAAAACTTGGATAGTAGAACTTCAATGTAGTGTTTCTATTGTCTTACG CGGCTCTTTAGATTGCAATCCCCAGAATGGAATCGTCCATCTTTCTCAACCCACTCA AAGATAATCTACCAGACATACCTACGCCCTCCATCCCAGCACCACGTCGCGATCACC CCTAAAACTTCAATAATTGAACACGTACTGATTTCCAAACCTTCTTCTTCTTCCTATC TATAAGA-3' (SEQ ID NO: 59). The resulting plasmid vector, pGLY2232, is an expression/integration vector that contains an expression cassette that encodes the human ERO1α fusion protein under control of the *Pichia pastoris PDI1* promoter and includes the 5' and 3' regions of the *Pichia pastoris PRB1* gene to target the plasmid vector to the *PRB1 locus* of genome for disruption of the *PRB1* locus and integration of the expression cassette into the *PRB1* locus. Figure 9 illustrates the construction of plasmid vector pGLY2232.

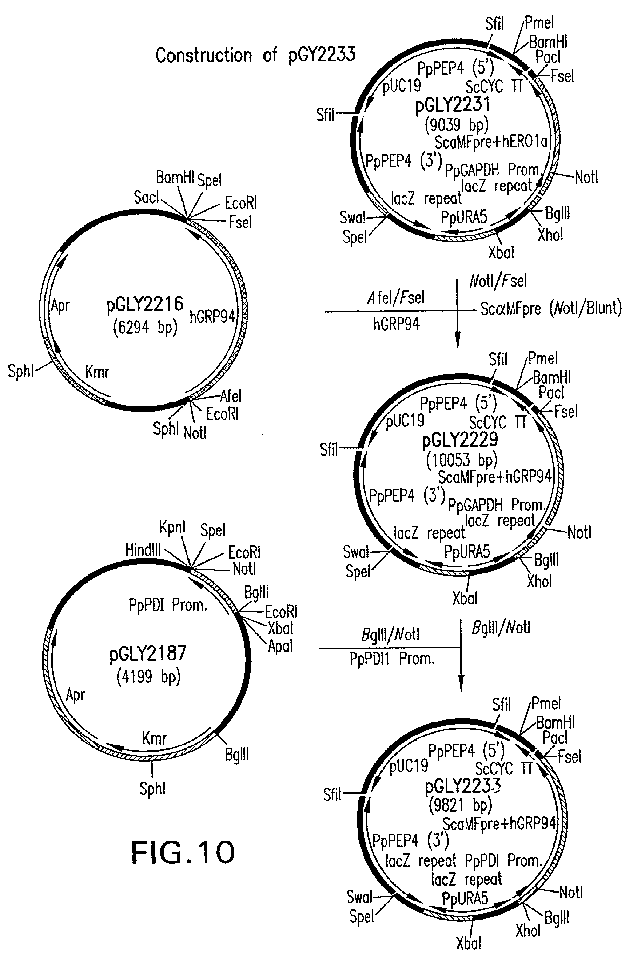

Construction of expression/integration vector pGLY2233 encoding the human GRP94 protein was as follows and is shown in Figure 10. The human GRP94 was PCR amplified from human liver cDNA (BD Bioscience) with the primers hGRP94/UP1: 5'-AGCGC TGACGATGAAGTTGATGTGGATGGTACAGTAG-3'; (SEQ ID NO: 15); and hGRP94/LP1: 5'-GGCCG GCCTT ACAAT TCATC ATGTT CAGCT GTAGA TTC 3'; (SEQ ID NO: 16). The PCR conditions were one cycle of 95°C for two minutes, 25 cycles of 95°C for 20 seconds, 55°C for 20 seconds, and 72°C for 2.5 minutes, and followed by one cycle of 72°C for 10 minutes. The PCR product was cloned into plasmid vector pCR2.1 to make plasmid vector pGLY2216. The nucleotide and amino acid sequences of the human GRP94 (SEQ ID NOs: 45 and 46, respectively) are shown in Table 11.

The nucleic acid molecule encoding the human GRP94 was released from plasmid vector pGLY2216 with *Afe*I and *Fse*I. The nucleic acid molecule was then ligated to a nucleic acid molecule encoding the ScαMPpre-signal peptide having *Not*I and blunt ends as above and plasmid vector pGLY2231 digested with *Not*I and *Fse*I carrying nucleic acid molecules comprising the *Pichia pastoris PEP4* 5' and 3' regions (PpPEP4-5' and PpPEP4-3' regions, respectively) to make plasmid vector pGLY2229. Plasmid vector pGLY2229 was digested with BgIII and NotI and a DNA fragment containing the PpPDI1 promoter was removed from plasmid vector pGLY2187 with BglII and NotI and the DNA fragment ligated into pGLY2229 to make plasmid vector pGLY2233. Plasmid vector pGLY2233 encodes the human GRP94 fusion protein under control of the *Pichia pastoris* PDI promoter and includes the 5' and 3' regions of the *Pichia pastoris PEP4* gene to target the plasmid vector to the *PEP4* locus of genome for disruption of the *PEP4* locus and integration of the expression cassette into the *PEP4* locus. Figure 10 illustrates the construction of plasmid vector pGLY2233.

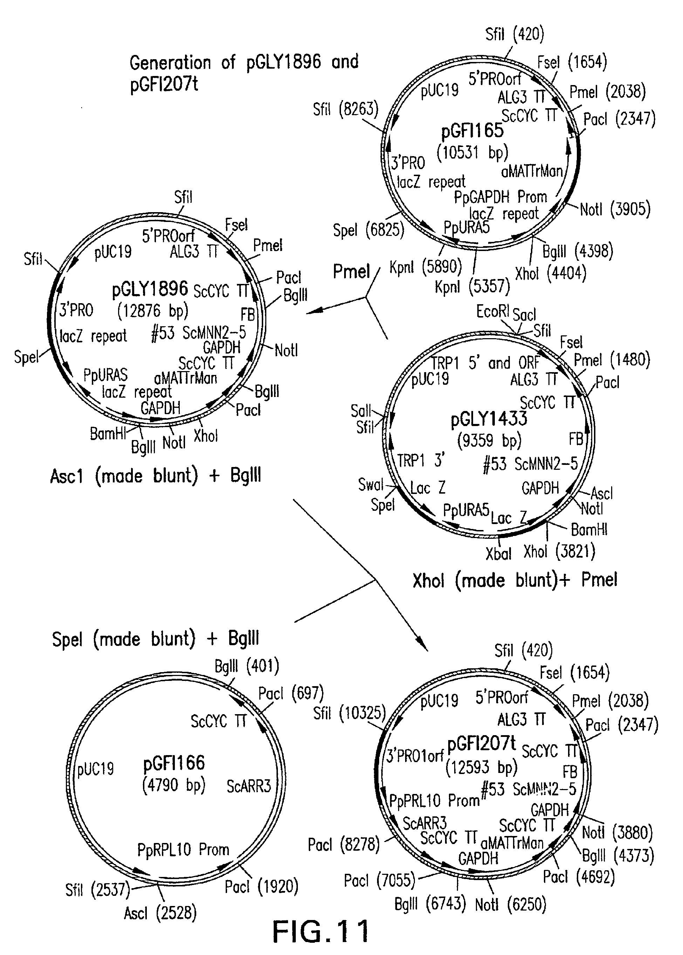

Construction of plasmid vectors pGLY1162, pGLY1896, and pGFI207t was as follows. All *Trichoderma reesei* α-1,2-mannosidase expression plasmid vectors were derived from pGFI165, which encodes the *T. reesei* α-1,2-mannosidase catalytic domain *(See* published International Application No. WO2007061631) fused to *S. cerevisiae* αMATpre signal peptide herein expression is under the control of the *Pichia pastoris* GAP promoter and wherein integration of the plasmid vectors is targeted to the *Pichia pastoris PROI* locus and selection is using the *Pichia pastoris URA5* gene. A map of plasmid vector pGFI165 is shown in Figure 11.

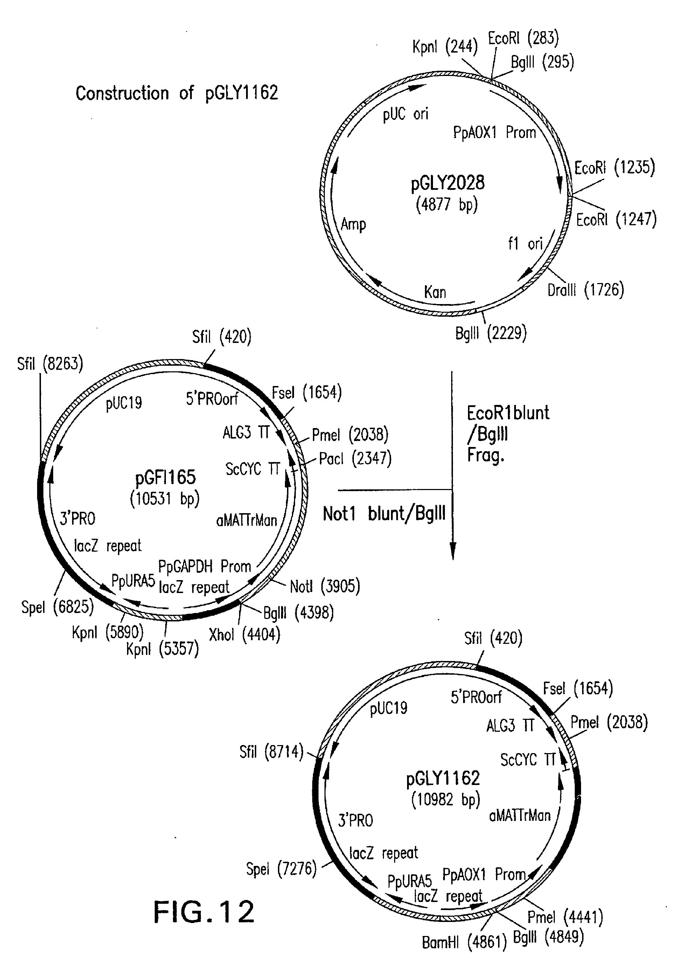

Plasmid vector pOLY1162 was made by replacing the GAP promoter in pGFI165 with the *Pichia pastoris* AOX1 (PpAOX1) promoter. This was accomplished by isolating the PpAOX1 promoter as an *Eco*RI (made blunt)-BglII fragment from pGLY2028, and inserting into pGFI165 that was digested with NotI (made blunt) and BglII. Integration of the plasmid vector is to the *Pichia pastoris PRO1* locus and selection is using the *Pichia pastoris URA5* gene. A map of plasmid vector pGLY1162 is shown in Figure 12.

Plasmid vector pGLY1896 contains an expression cassette encoding the mouse α-1,2-mannosidase catalytic domain fused to the *S. cerevisiae MNN2* membrane insertion leader peptide fusion protein (*See* Choi et al., Proc. Natl. Acad. Sci. USA 100: 5022 (2003)) inserted into plasmid vector pGFI165 (Figure 12). This was accomplished by isolating the OAPp-ScMNN2-mouse MNSI expression cassette from pGLY1433 digested wit*h Xho*I (and the ends made blunt) and *Pme*I, and inserting the fragment into pGFI165 that digested with *Pme*I. Integration of the plasmid vector is to the *Pichia pastoris PRO1* locus and selection is using the *Pichia pastoris URA5* gene. A map of plasmid vector pGLY1896 is shown in Figure 11.

Plasmid vector pGFI207t is similar to pGLY1896 except that the *URA5* selection marker was replaced with the *S. cerevisiae ARR3* (ScARR3) gene, which confers resistance to arsenite. This was accomplished by isolating the ScARR3 gene from pGFI166 digested with *Asc*I and the *Asc*I ends made blunt) and *Bgl*II, and inserting the fragment into pGLY1896 that digested with *Spe*I and the *Spe*I ends made blunt and *Bgl*II*.* Integration of the plasmid vector is to the *Pichia pastoris PRO1* locus and selection is using the *Saccharomyces cerevisiae ARR3* gene. A map of plasmid vector pGFI207t is shown in Figure 11.

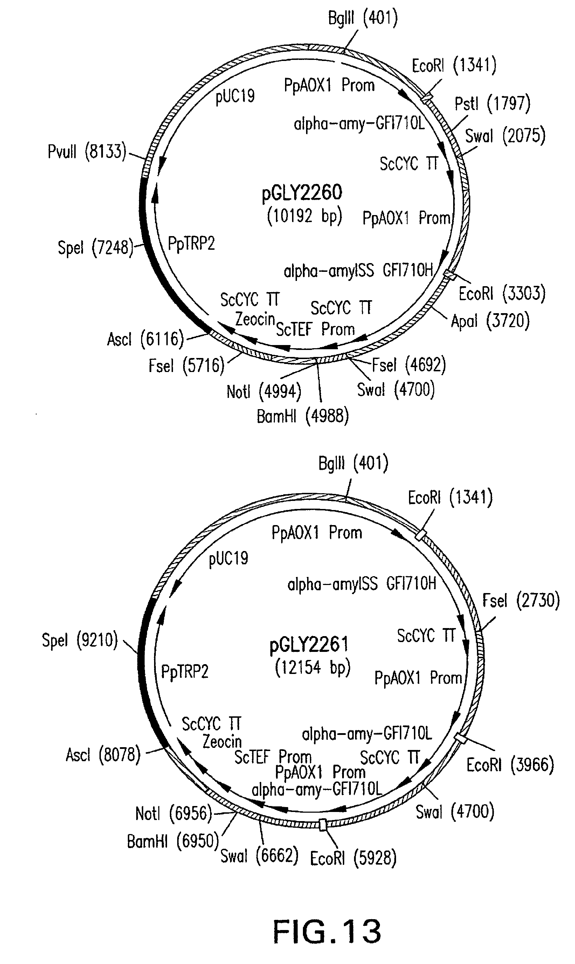

Construction of anti-DKK1 antibody expression/integration plasmid vectors pGLY2260 and pGLY2261 was as follows. Anti-DKK1 antibodies are antibodies that recognize Dickkopf protein 1, a ligand involved in the Wnt signaling pathway. To generate expression/integration plasmid vectors pGLY2260 and pGLY2261 encoding an anti-DKK1 antibody, codon-optimized nucleic acid molecules encoding heavy chain (HC; fusion protein containing VH + IgG₂m4) and light chain (LC; fusion protein containing VL + Lλ constant region) fusion proteins, each in frame with a nucleic acid molecule encoding an α-amylase (from *Aspergillus niger*) signal peptide were synthesized by GeneArt AG. The nucleotide and amino acid sequences for the α-amylase signal peptide are shown in SEQ ID NOs: 33 and 34. The nucleotide sequence of the HC is shown in SEQ ID NO: 51 and the amino acid sequence is shown in SEQ ID NO: 52. The nucleotide sequence of the LC is shown in SEQ ID NO: 53 and the amino acid sequence is shown in SEQ ID NO: 54. The IgG₂m4 isotype has been disclosed in U.S. Published Application No. 2007/0148167 and U.S. Published Application No. 2006/0228349. The nucleic acid molecules encoding the HC and LC fusion proteins were separately cloned using unique *5'-EcoR*I and *3'-Fse*I sites into expression plasmid vector pGLY1508 to form plasmid vectors pGLY1278 and pGLY1274, respectively. These plasmid vectors contained the Zeocin-resistance marker and *TRP2* integration sites and the *Pichia pastoris* AOX1 promoter operably linked to the nucleic acid molecules encoding the HC and LC fusion proteins. The LC fusion protein expression cassette was removed from pGLY1274 with *Bgl*II and *Bam*H1 and cloned into pGLY1278 digested with *Bgl*II to generate plasmid vector pGLY2260, which encodes the HC and LC fusion proteins and targets the expression cassettes to the *TRP2* locus for integration of the expression cassettes into the *TRP2* locus. The plasmid vector pGLY2261 contains an additional LC in plasmid vector pGLY2260. (Figure 13).

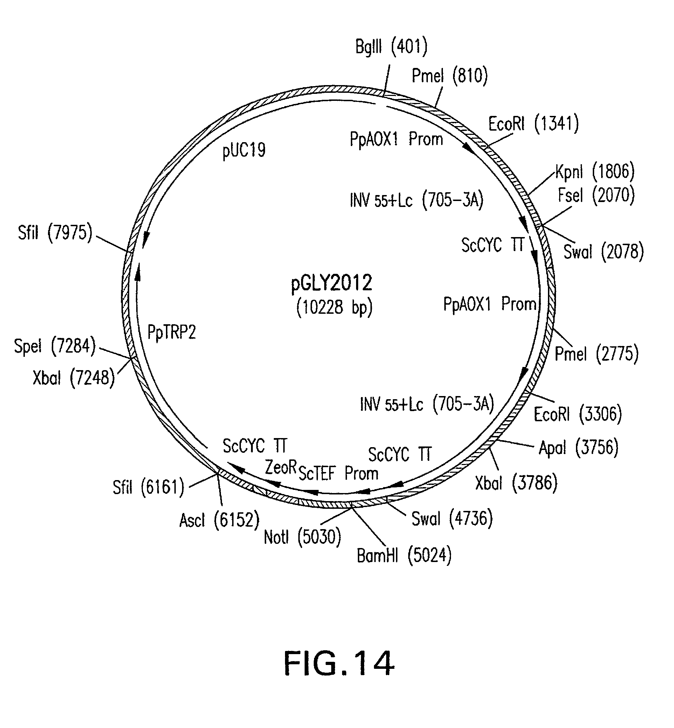

Construction of anti-ADDL antibody expression/integration plasmid vector pGLY2260 was as follows. Anti-ADDL antibodies are antibodies that recognize Aβ-derived diffusible ligands, see for example U.S. Published Application No. 20070081998. To generate expression/integration plasmid vector pGLY2012, codon-optimized nucleic acid molecules encoding heavy chain (HC; contained VH + IgG2m4) and light chain (LC; fusion protein containing VL + Lλ constant region) fusion proteins, each in frame with a nucleic acid molecule encoding *Saccharomyces cerevisiae* invertase signal peptide were synthesized by GeneArt AG. The nucleic acid molecules encoding the HC and LC fusion proteins were separately cloned using unique *5'-Eco*RI and *3'-Fse*l sites into expression/integration plasmid vectors pGLY1508 and pGLY1261 to form pGLY2011 and pGLY2010, respectively, which contained the Zeocin-resistance marker and *TRP2* integration sites and the *Pichia pastoris* AOX1 promoter operably linked to the nucleic acid molecules encoding the HC and LC fusion proteins. The HC expression cassette was removed from pGLY2011 with *Bgl*II and *Not*I and cloned into pGLY2010 digested with *Bam*HI and *Not*I to generate pGLY2012, which encodes the HC and LC fusion proteins and targets the expression cassettes to the *TRP2 locus* for integration of the expression cassettes into the *TRP2 locus* (Figure 14).

Yeast transformations with the above expression/integration vectors were as follows. *Pichia pastoris* strains were grown in 50 mL YPD media (yeast extract (1%), peptone (2%), dextrose (2%)) overnight to an OD of between about 0.2 to 6.0. After incubation on ice for 30 minutes, cells were pelleted by centrifugation at 2500-3000 rpm for 5minutes. Media was removed and the cells washed three times with ice cold sterile 1M sorbitol before resuspension in 0.5 ml ice cold sterile 1M sorbitol. Ten µL linearized DNA (5-20 µg) and 100 µL cell suspension was combined in an electroporation cuvette and incubated for 5 minutes on ice. Electroporation was in a Bio-Rad GenePulser Xcell following the preset *Pichia pastoris* protocol (2 kV, 25 µF, 200 Ω), immediately followed by the addition of 1 mL YPDS recovery media (YPD media plus 1 M sorbitol). The transformed cells were allowed to recover for four hours to overnight at room temperature (24°C) before plating the cells on selective media.

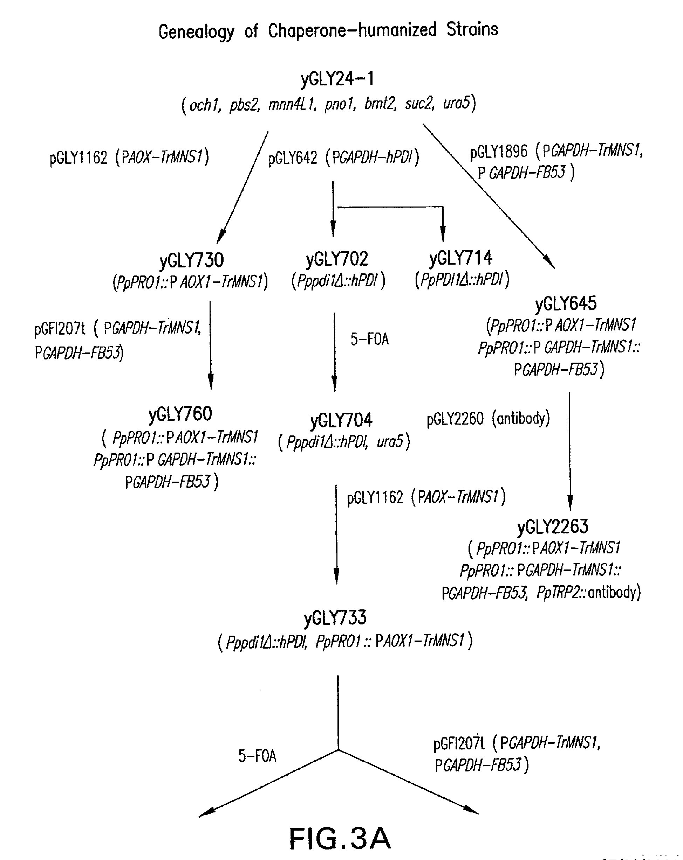

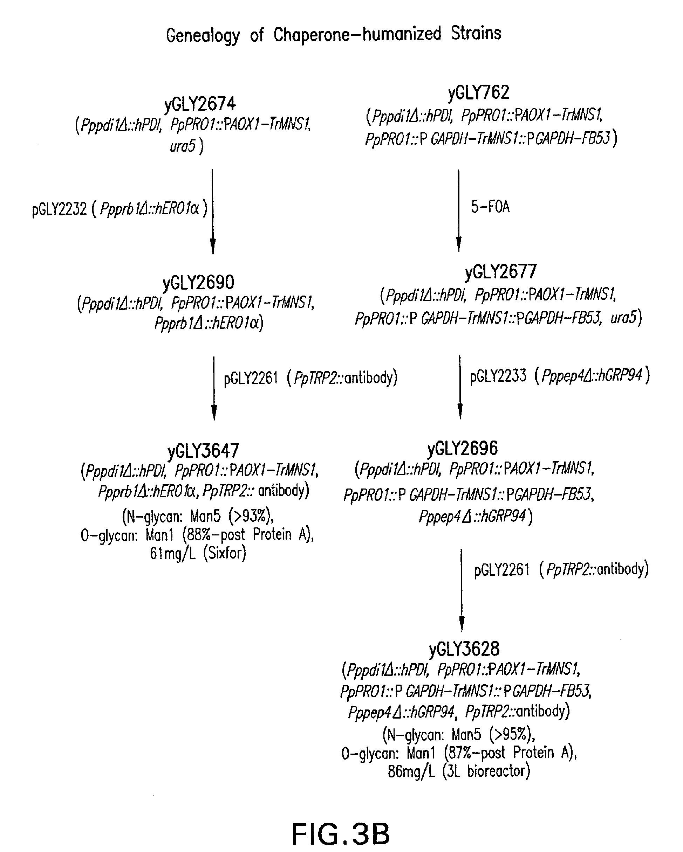

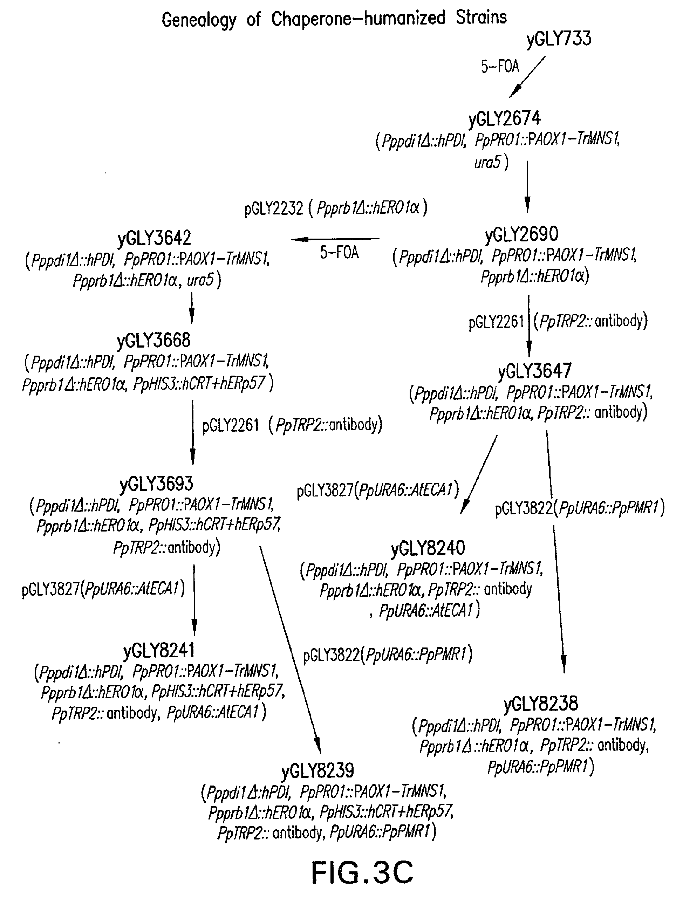

Generation of Cell Lines was as follows and is shown in Figure 3. The strain **yGLY24-1** (*ura5Δ::MET1 och1Δ::lacZ bmt2Δ::lacZlKlMNN2-2* / *mnn4L1Δ::lacZl MmSLC35A3 pno1Δmnn4Δ::lacZ met16Δ::lacZ*), was constructed using methods described earlier *(See* for example, Nett and Gerngross, Yeast 20:1279 (2003); Choi et al., Proc. NatI. Acad. Sci. USA 100:5022 (2003); Hamilton et al., Science 301:1244 (2003)). The *BMT2* gene has been disclosed in Mille et al., J. Biol. Chem. 283: 9724-9736 (2008) and U.S. Published Application No. 20060211085. The *PNO1* gene has been disclosed in U.S. Patent No. 7,198,921 and the *mnn4L1* gene (also referred to as *mnn4b*) has been disclosed in U.S. Patent No. 7,259,007. The *mnn4* refers to *mnn4L2* or *mnn4a.* In the genotype, K1MNN2-2 is the *Kluveromyces lactis* GlcNAc transporter and MmSLC35A3 is the *Mus musculus* GlcNAc transporter. The *URA5* deletion renders the yGLY24-1 strain auxotrophic for uracil *(See* U.S. Published application No. 2004/0229306) and was used to construct the humanized chaperone strains that follow. While the various expression cassettes were integrated into particular loci of the *Pichia pastoris* genome in the examples herein, it is understood that the operation of the invention is independent of the loci used for integration. Loci other than those disclosed herein can be used for integration of the expression cassettes. Suitable integration sites include those enumerated in U.S. Published application No. 20070072262 and include homologs to loci known for *Saccharomyces cerevisiae* and other yeast or fungi.

Control strain **yGLY645** (PpPDI1) was constructed. Strain yGLY645 expresses both a *Trichoderma Reesei* mannosidase1 (TrMNS1) and a mouse mannosidase IA (MuMNS1A), each constitutively expressed under the control of a PpGAPDH promoter, with the native *Pichia pastoris PDI1* locus intact. Strain yGLY645 was generated from strain yGLY24-1 by transforming yGLY24-1 with plasmid vector pGLY1896, which targeted the plasmid vector to the Proline 1 (*PRO1*) locus in the *Pichia* genome. Plasmid vector pGLY1896 contains expression cassettes encoding the *Trichoderma Reesei* mannosidase 1 (TrMNS1) and the mouse mannosidase IA (FB53, MuMNS1A), each constitutively expressed under the control of a PpGAPDH promoter.

Strains **yGLY702** and **yGLY704** were generated in order to test the effectiveness of the human PDI1 expressed in *Pichia partoris* cells in the absence of the endogenous *Pichia pastoris* PDI1 gene. Strains yGLY702 and yGLY704 (hPDI) were constructed as follows. Strain yGLY702 was generated by transforming yGLY24-1 with plasmid vector pGLY642 containing the expression cassette encoding the human PDI under control of the constitutive PpGAPDH promoter. Plasmid vector pGLY642 also contained an expression cassette encoding the *Pichia pastoris URA5,* which rendered strain yGLY702 prototrophic for uracil. The *URA5* expression cassette was removed by counterselecting yGLY702 on 5-FOA plates to produce strain yGLY704 in which, so that the *Pichia pastoris* PDI1 gene has been stably replaced by the human PDI gene and the strain is auxotrophic for uracil.

The replacement of the *Pichia pastoris PDI1* with the human PDI using plasmid vector pGLY642 was confirmed by colony PCR using the following primers specific to only the PpPDI1 ORF; PpPDI/UPi-1, 5'-GGTGAGGTTGAGGTCCCAAGTGACTATCAAGGTC-3'; (SEQ ID NO: 7); PpPDI/LPi-1, 5'-GACCTTGATAGTCACTTGGGACCTCAACCTCACC-3'; (SEQ ID NO: 8); PpPDI/UPi-2, 5' CGCCAATGATGAGGATGCCTCTTCAAAGGT TGTG-3'; (SEQ ID NO: 9); and PpPDI/LPi-2, 5'-CACAACCTTTGAAGAGGCATCCTCATCATT GGCG-3'; (SEQ ID NO: 10). Thus, the absence of PCR product indicates the knockout of PpPDI1. The PCR conditions were one cycle of 95°C for two minutes, 25 cycles of 95°C for 20 seconds, 58°C for 20 seconds, and 72°C for one minute, and followed by one cycle of 72°C for 10 minutes.

Additional PCR was used to confirm the double crossover of pGLY642 at the PpPDI1 locus using PCR primers; PpPDI-5'/UP, 5'-GGCGATTGCATTCGCGACTGTATC-3; (SEQ ID NO: 11); and, hPDI-3'/LP 5'-CCTAGAGAGCGGTGGCCAAGATG-3'; (SEQ ID NO: 12). PpPDI-5'/UP primes the upstream region of PpPDI1 that is absent in PpPDI1 (5') of pGY642 and hPDI-3'/LP primes human PDI ORF in pGLY642. The PCR conditions were one cycle of 95°C for two minutes, 25 cycles of 95°C for 20 seconds, 50°C for 30 seconds, and 72°C for 2.5 minutes, and followed by one cycle of 72°C for 10 minutes.

The integration efficiency of a plasmid vector as a knockout (i.e., a double crossover event) or as a 'roll-in' (i.e., a single integration of the plasmid vector into the genome, can be dependent upon a number of factors, including the number and length of homologous regions between vectors and the corresponding genes on host chromosomal DNA, selection markers, the role of the gene of interest, and the ability of the knocked-in gene to complement the endogenous function. The inventors found that in some instances pGLY642 was integrated as a double crossover, resulting in replacement of the endogenous PpPDI gene with human PpPDI, while in other cases, the pGLY642 plasmid vector was integrated as a single integration, resulting in presence of both the endogenous PpPDI1 gene and a human PpPDI gene. In order to distinguish between these events, the inventors utilized PCR primers of Sequence ID Nos. 11 through 14, described herein. If the PpPDI gene has been retained after integration of the pGLY642 plasmid vector, PpPDI-5'/UP and hPDI-3'/LP, directed to the internal PpPDI coding sequence, will result in an amplification product and a corresponding band. In the event of a knockout or double crossover, these primers will not result in any amplification product and no corresponding band will be visible.

The roll-in of pGLY642 was confirmed with the primers; PpPDI/UPi (SEQ ID NO: 7) and PpPDIILPi-1 (SEQ ID NO: 8) encoding PpPDI1, and hPDI/UP, 5'-GTGGCCACACCAGGGGGCATGGAAC-3'; (SEQ ID NO: 13); and hPDI-3'/LP, 5'-CCTAGAGAGCGGTGGCCAAG AG-3'; (SEQ ID NO: 14); encoding human PDI. The PCR conditions were one cycle of 95°C for two minutes, 25 cycles of 95°C for 20 seconds, 58°C for 20 seconds, and 72°C for one minute, and followed by 1 cycle of 72°C for 10 minutes for PpPDI1, and 1 cycle of 95°C for two minutes, 25 cycles of 95°C for 20 seconds, 50°C for 30 seconds, and 72°C for 2.5 minutes, and followed by one cycle of 72°C for 10 minutes for human PDI.

Strain **yGLY714** is a strain that contains both the *Pichia pastoris* PDI1 locus and expresses the human PDI and was a result of integration via a single crossover event Strain yGLY714 was generated from strain yGLY24-1 by integrating plasmid vector pGLY642, which comprises the human PDI gene under constitutive regulatory control of the *Pichia pastoris* GAPDH promoter, into the PpPDI 5'UTR region in yGLY24-1. Integration of this vector does not disrupt expression of the *Pichia pastoris PDII* locus. Thus, in yGLY714, the human PDI is constitutively expressed in the presence of the *Pichia pastoris* endogenous *PDII.*

Strain **yGLY733** was generated by transforming with plasmid vector pGLY1162, which comprises an expression cassette that encodes the *Trichoderma Reesei* mannosidase (TrMNS1) operably linked to the *Pichia pastoris* AOX1 promoter (PpAOXI-TrMNS1), into the *PRO1* locus of yGLY704. This strain has the gene encoding the *Pichia pastoris* PDI replaced with the expression cassette encoding the human *PDII,* has the PpAOX1-TrMNS1 expression cassette integrated into the *PRO1* locus, and is a *URA5* prototroph. The PpAOX1 promoter allows overexpression when the cells are grown in the presence of methanol.

Strain **yGLY762** was constructed by integrating expression cassettes encoding TrMNS1 and mouse mannosidase IA (MuMNS1A), each operably linked to the *Pichia pastoris* GAPDH promoter in plasmid vector pGFI207t into strain yGLY733 at the 5' *PRO1* locus UTR in *Pichia pastoris* genome. This strain has the gene encoding the *Pichia pastoris* PDI1 replaced with the expression cassette encoding the human PDI, has the PpGAPDH-TrMNS and PpGAPDH-MuMNS1A expression cassettes integrated into the *PRO1* locus, and is a *URA5* prototroph.

Strain **yGLY730** is a control strain for strain yGLY733. Strain yGLY730 was generated by transforming pGLY1162, which comprises an expression cassette that encodes the *Trichoderma Reesei* mannosidase (TrMNS1) operably linked to the *Pichia pastoris* AOX1 promoter (PpAOX1-TrMNS1), into the *PRO1* locus of yGLY24-1. This strain has the *Pichia pastoris* PDI1, has the PpAOX1-TrMNS1 expression cassette integrated into the *PRO1* locus, and is a *URA5* prototroph.

Control Strain **yGLY760** was constructed by integrating expression cassettes encoding TrMNS1 and mouse mannosidase IA (MuMNS1A), each operably linked to the *Pichia pastoris* GAPDH promoter in plasmid vector pGFI207t into control strain yGLY730 at the 5' *PRO1* locus UTR in *Pichia pastoris* genome. This strain has the gene encoding the *Pichia pastoris* PDI1, has the PpGAPDH-TrMNS1 and PpGAPDH-MuMNS1A expression cassettes integrated into the *PRO1* locus, and is a *URA5* prototroph.

Strain **yGLY2263** was generated by transforming strain yGLY645 with integration/expression plasmid pGLY2260, which targets an expression cassette encoding the anti-DKK1 antibody to the *TXP2* tocus.

Strain **yGLY2674** was generated by counterselecting yGLY733 on 5-FOA plates. This strain has the gene encoding the *Pichia pastoris* PDI1 replaced with the expression cassette encoding the human PDI, has the PpAOX1-TrMNS1 expression cassette integrated into the *PRO1* locus, and is a *URA5* auxotroph.

Strain **yGLY2677** was generated by counterselecting yGLY762 on 5-FOA plates. This strain has the gene encoding the *Pichia pastoris* PDI1 replaced with the expression cassette encoding the human PDI, has the PpAOX1-TrMNS1 expression cassette integrated into the *PRO1* locus, has the PpGAPH-TrMNSI and PpGAPDH-MuMNS1A expression cassettes integrated into the *PRO1* locus, and is a *URA5* auxotroph.

Strains **yGLY2690** was generated by integrating plasmid vector pGLY2232, which encodes the human ERO1α protein, into the *PRB1* locus. This strain has the gene encoding the *Pichia pastoris* PDI1 replaced with the expression cassette encoding the human PDI, has the PpAOX1-TrMNS1 expression cassette integrated into the *PRO1* locus, the human ERO1α expression cassette integrated into the PRB1 locus, and is a *URA5* prototroph.

Strains **yGLY2696** was generated by integrating plasmid vector pGLY2233, which encodes the human GRP94 protein, into the PEP4 locus. This strain has the gene encoding the *Pichia pastoris* PDI1 replaced with the expression cassette encoding the human PDI, has the PpAOX1-TrMNS1 expression cassette integrated into the *PRO1* locus, has the PpGAPDH-TrMNS1 and PpGAPDH-MuMNS1A expression cassettes integrated into the *PRO1* locus, has the human GRP94 integrated into the *PEP4* locus, and is a *URA5* prototroph.

Strain **yGLY3628** was generated by transforming strain yGLY2696 with integration/expression plasmid pGLY2261, which targets an expression cassette encoding the anti-DKK1 antibody to the *TRP2* locus.

Strain **yGLY3647** was generated by transforming strain yGLY2690 with integration/expression plasmid pGLY2261, which targets an expression cassette encoding the anti-DKK1 antibody to the *TRP2* locus.

The yield of protein produced in a strain, which expresses the human PDI protein in place of the *Pichia pastoris* PDI1 protein, was compared to the yield of the same protein produced in a strain, which expresses both the human and *Pichia pastoris* PDI proteins, and a strain, which expresses only the *Pichia pastoris* PDI1 protein. Strain yGLY733, which expresses the human PDI protein in place of the *Pichia pastoris* PDI1 protein, strain yGLY714, which expresses both the human and *Pichia pastoris* PDI1 proteins, and strain yGLY730, which expresses only the *Pichia pastoris* PDI1 protein were evaluated to determine the effect of replacing the *Pichia pastoris* PDI1 protein with the human PDI protein on antibody titers produced by the strains. All three yeast strains were transformed with plasmid vector pGLY2261, which encodes the anti-DKK1 antibody.

Titer improvement for culture growth was determined from deep-well plate screening in accordance with the NIH ImageJ software protocol, as described in Rasband, ImageJ, U. S. National Institutes of Health, Bethesda, Maryland, USA, 1997-2007; and Abramoff, et al., Biophotonics International,11: 36-42 (2004). Briefly, antibody screening in 96 deep-well plates was performed essentially as follows. Transformants were inoculated to 600 µL BMGY and grown at 24°C at 840 rpm for two days in a Micro-Plate Shaker. The resulting 50 µL seed culture was transferred to two 96-well plates containing 600 µL fresh BMGY per well and incubated for two days at the same culture condition as above. The two expansion plates were combined to one prior to centrifugation for 5 minutes at 1000 rpm, the cell pellets were induced in 600 µL BMMY per well for two days and then the centrifuged 400 µL clear supernatant was purified using protein A beads. The purified proteins were subjected to SDS-PAGE electrophoresis and the density of protein bands were analyzed using NIH ImageJ software.

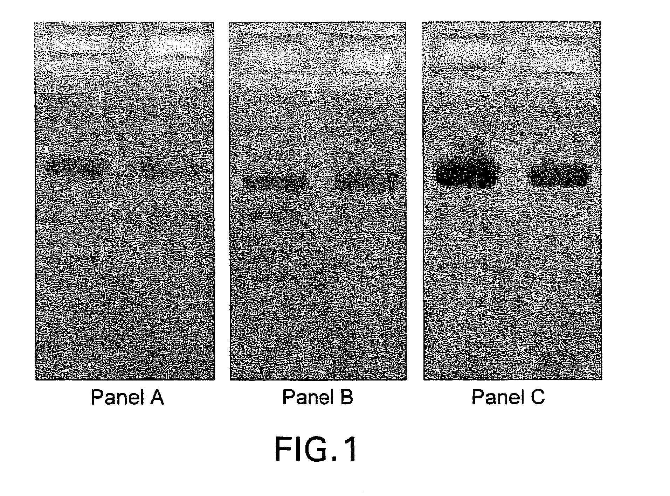

Representative results are shown in Figure 1. Figure 1 (Panel B) shows that while yGLY714, which expresses both *Pichia pasroris* PDI1 and human PDI, improved yield two-fold over the control (yGLY730) (Panel A), a five-fold increase in yield was achieved with strain yGLY733, which expresses only the human PDI (Panel C). The results are also presented in Table 1.

| Table 1 | | | |
|---|---|---|---|
| Replacement of PpPDI1 | | | |
| | yGLY730 (control) | yGLY714 (Both Pichia and human PDI) | yGLY733 (human PDI) |
| *Pichia pastoris* PDI1 | Wild-type | Wild-type | Knockout |
| Human PDI | None | Overexpression | Overexpression |
| Titer improvement | Control | 2-fold | 5-fold |

Strains yGLY730 and yGLY733 were transformed with plasmid vector pGLY2012 which encodes the anti-ADDL antibody. The transformed strains were evaluated by 96 deep well screening as described above and antibody was produced in 500 mL SixFors and 3L fermentors using the following procedures. Bioreactor Screenings (SIXFORS) were done in 0.5 L vessels (Sixfors multi-fermentation system, ATR Biotech, Laurel, MD) under the following conditions: pH at 6.5, 24°C, 0.3 SLPM, and an initial stirrer speed of 550 rpm with an initial working volume of 350 mL (330 mL BMGY medium and 20mL inoculum). IRIS multi-fermenter software (ATR Biotech, Laurel, MD) was used to linearly increase the stirrer speed from 550 rpm to 1200 rpm over 10 hours, one hour after inoculation. Seed cultures (200 mL of BMGY in a 1 L baffled flask) were inoculated directly from agar plates. The seed flasks were incubated for 72 hours at 24°C to reach optical densities (OD₆₀₀) between 95 and 100. The fermenters were inoculated with 200 mL stationary phase flask cultures that were concentrated to 20 mL by centrifugation. The batch phase ended on completion of the initial charge glycerol (18-24h) fermentation and were followed by a second batch phase that was initiated by the addition of 17 mL of glycerol feed solution (50% [w/w] glycerol, 5 mg/L Biotin, 12.5 ml/L PTM1 salts(65 g/L FeSO₄.7H₂O, 20 g/L ZnCl₂, 9 g/L H₂SO₄, 6 g/L CuSO₄.5H₂O, 5 g/L H₂SO₄, 3 g/L MnSO₄-7H₂O, 500 mg/L CoCl₂·6H₂O, 200 mg/L NaMoO₄·2H₂O, 200 mg/L biotin, 80 mg/L Nal, 20 mg/L H₃BO₄)). Upon completion of the second batch phase, as signaled by a spike in dissolved oxygen, the induction phase was initiated by feeding a methanol feed solution (100% MeOH 5 mg/L biotin, 12.5 mL/L PTM1) at 0.6 g/h for 32-40 hours. The cultivation is harvested by centrifugation.

Bioreactor cultivations (3L) were done in 3L (Applikon, Foster City, CA) and 15L (Applikon, Foster City, CA ) glass bioreactors and a 40L (Applikon, Foster City, CA) stainless steel, steam in place bioreactor. Seed cultures were prepared by inoculating BMGY media directly with frozen stock vials at a 1% volumetric ratio. Seed flasks were incubated at 24°C for 48 hours to obtain an optical density (OD₆₀₀) of 20±5 to ensure that cells are growing exponentially upon transfer. The cultivation medium contained 40 g glycerol, 18.2 g sorbitol, 2.3 g K₂HPO₄, 11.9 g KH₂PO₄, 10 g yeast extract (BD, Franklin Lakes, NJ), 20 g peptone (BD, Franklin Lakes, NJ), 4 x 10-3 g biotin and 13.4 g Yeast Nitrogen Base (BD, Franklin Lakes, NJ) per liter. The bioreactor was inoculated with a 10% volumetric ratio of seed to initial media. Cultivations were done in fed-batch mode under the following conditions: temperature set at 24±0.5°C, pH controlled at to 6.5±0.1 with NH₄OH, dissolved oxygen was maintained at 1.7±0.1 mg/L by cascading agitation rate on the addition of O₂. The airflow rate was maintained at 0.7 vvm. After depletion of the initial charge glycerol (40 g/L), a 50% glycerol solution containing 12.5 mL/L of PTM1 salts was fed exponentially at 50% of the minimum growth rate for eight hours until 250 g/L of wet cell weight was reached. Induction was initiated after a 30 minute starvation phase when methanol was fed exponentially to maintain a specific growth rate of 0.01 h⁻¹. When an oxygen uptake rate of 150 mM/L/h was reached the methanol feed rate was kept constant to avoid oxygen limitation. The results are shown in Table 2, which shows about a three-fold increase in antibody titer.

The antibodies were also analyzed to determine whether replacing the *Pichia pastoris* PDI1 gene with an expression cassette encoding the human PDI would have an effect on *O*-glycosylation of the antibodies. In general, *O*-glycosylation of antibodies intended for use in humans is undesirable.

*O*-glycan determination was performed using a Dionex-HPLC (HPAEC-PAD) as follows. To measure *O*-glycosylation reduction, protein was purified from the growth medium using protein A chromatography (Li et al. Nat. Biotechnol. 24(2):210-5 (2006)) and the *O*-glycans released from and separated from protein by alkaline elimination (beta -elimination) (Harvey, Mass Spectrometry Reviews 18: 349- 451 (1999)). This process also reduces the newly formed reducing terminus of the released *O*-glycan (either oligomannose or mannose) to mannitol. The mannitol group thus serves as a unique indicator of each *O*- glycan. 0.5 nmole or more of protein, contained within a volume of 100 µL PBS buffer, was required for beta elimination. The sample was treated with 25 µL alkaline borohydride reagent and incubated at 50°C for 16 hours. About 20 uL arabitol internal standard was added, followed by 10 µL glacial acetic acid. The sample was then centrifuged through a Millipore filter containing both SEPABEADS and AG 50W-X8 resin and washed with water. The samples, including wash, were transferred to plastic autosampler vials and evaporated to dryness in a centrifugal evaporator. 150 µL 1% AcOH/MeOH was added to the samples and the samples evaporated to dryness in a centrifugal evaporator. This last step was repeated five more times. 200 µL of water was added and 100 µL of the sample was analyzed by high pH anion-exchange chromatography coupled with pulsed electrochemical detection-Dionex HPLC (HPAEC-PAD). Average *O*-glycan occupancy was determined based upon the amount of mannitol recovered.

As shown in Table 2, *O*-glycosylation was reduced in strains in which the *Pichia pastoris* PDI1 was replaced with an expression cassette encoding the human PDI. In strain yGLY733, *O*-glycan occupancy (number of *O*-glycosylation sites *O*-glycosylated) was reduced and for those sites occupied, the percent of *O*-glycans consisting of only one mannose was increased. These results suggest that replacing the *Pichia pastoris* PDI1 with an expression cassette encoding the human PDI will enable the production of antibodies in *Pichia pastoris* with reduced *O*-glycosylation.

| Table 2 | | |
|---|---|---|
| Anti-ADDL antibody: *O*-Glycan & Titer | | |
| | yGLY730 | yGLY733 |
| *Pichia* PDI1 | Wild-type | Knockout |
| [Human PDI | None | Overexpressed |
| *O*-glycan Occupancy (H2L2) | 7.4 | 42 |
| *O*-glycan % (Man1/Man2) | 75.5/24.5 | 82.5/17.5 |
| | 12.5mg/L (SixFors) | 38.3mg/L (SixFors) |
| Titer | | 93mg/L (3L) |

The above three strams (yGLY730, yGLY714, and yGLY733) produce glycoproteins that have *Pichia pastoris N*-glycosylation patterns. GS 2.0 strains are *Pichia pastoris* strains that have been genetically engineered to produce glycoproteins having predominantly Man₅GlcNAc₂ *N*-glycans. The following experiment was performed with GS 2.0 strains that produce glycoproteins that have predominantly Man₅GlcNAc₂ *N*-glycans to determine the effect of replacing the *Pichia pastoris* PDI1 protein with the human PDI protein on antibody titers produced by these strains. Strains yGLY2690 and yGLY2696 are GFI 2.0 strains that produce glycoproteins that have predominantly Man₅GlcNAc₂ *N*-glycans and have the *Pichia pastoris* PDI1 gene replaced with the expression cassette encoding the human PDI protein *(See* Figure 3). These two strains were transformed with plasmid vector pGLY2261, which encodes the anti-DKK1 antibody, to produce strains yGLY3647 and yGLY3628 (*See* Figure 3) and the strains evaluated by 96 deep well screening as described above. Antibody was produced in 500 ml SixFors and 3L fermentors using the parameters described above to determine the effect of replacing the *Pichia pastoris* PDI1 protein with the human PDI protein on antibody titers produced by the strains. The results are shown in Table 3. Strain yGLY2263 is a control in which plasmid vector pGLY2260 was transformed into strain yGLY645, which produces glycoproteins having predominantly Man₅GlcNAc₂ *N*-glycans and expresses only the endogenous PDI1 gene.

Table 3 shows that replacing the gene encoding the *Pichia pastoris* PDI1 with an expression cassette encoding the human PDI in yeast genetically engineered to produce glycoproteins that have predominantly MansGlcNAc₂ *N*-glycans effects an improvement in the titers of antibodies produced by the yeast. Table 3 also shows that *O*-glycosylation occupancy was still reduced in these strains genetically engineered to produce glycoproteins having predominantly Man₅GlcNAc₂ *N*-glycans. Additionally, Table 3 shows an increase in the amount of *N*-glycosylation in the strains with the endogenous PDI1 replaced with the human PDI.

| Table 3 | | | |
|---|---|---|---|
| Anti-DKK1 antibody: Titer, *N*-glycan & *O*-glycan | | | |
| GS2.0 Strain | yGLY2263 (control) | yGLY3647 | yGLY3628 |
| *Pichia pastoris* PDI1 | Wild-type | Knockout | Knockout |
| Human PDI | None | Overexpressed | Overexpressed |
| Human ERO1α | None | Expressed | None |
| Human GRP94 | None | None | Expressed |
| *Pichia pastoris* PRB1 | Intact | Knockout | Intact |
| *Pichia pastoris* PEP4 | Intact | Intact | Knockout |
| *N*-plvcan (Man5) | 83.7% | 93.4% | 95.4% |
| -*O*-glycan. (Occupancy: H2L2) | 23.7 | 9.2 | 10.0 |
| *O*-glycan (Man1/Man2) | 55/40 | 88/12 | 87/13 |
| Titer | 27mg/L (SixFors) | 61mg/L (SixFors) | 86mg/L (3L) |

### EXAMPLE 2

A benefit of the strains shown in Tables 2 and 3 is that making yeast strains that have replaced the endogenous *PDI1* gene with an expression cassette that encodes a heterologous PDI not only effects an increase in protein yield but also effects a decrease in both the number of attached *O*-glycans (occupancy) and a decrease in undesired Man₂ *O*-glycan structures. Recombinant proteins produced in yeast often display aberrant *O*-glycosylation structures relative to compositions of the same glycoprotein produced from mammalian cell culture, reflecting the significant differences between the glycosylation machinery of mammalian and yeast cells. These aberrant structures may be immunogenic in humans.

The inventors noted that host cells of *Pichia pastoris* carrying the human PDI gene in place of the endogenous *Pichia pastoris PDI1* gene were strain more resistant to *PMT* protein inhibitors (*See* published International Application No. WO2007061631), suggesting that these strains might be better suited to tolerate deletions of various *PMT* genes. This is because in prior attempts to make *ΔPMT* knockouts *in ΔOCHI*/*ΔPNO1*/*ΔPBS2* strains of *Pichia pastoris, ΔPMT1* knockouts and *ΔPMT2* knockouts could not be obtained; presumably because they are lethal in this genetic background (unpublished results). *ΔPMT4* knockouts could be obtained, but they typically exhibited only weak growth and poor protein expression compared to parental strains (*See* Figures 6 and 7). While *ΔPMT5* and *ΔPMT6* knockouts could be obtained, the deletions exhibited little or no effect on cell growth or protein expression compared to parental strains, suggesting that these *PMT* genes were not effective in reduction of *O*-glycosylation.

*PMT* knockout yeast strains were created in the appropriate *Pichia pastoris* strains following the procedure outlined for *Saccharomyces cerevisiae* in Gentzsch and Tanner, EMBO J. 15: 25752-5759 (1996), as described further in Published International Application No. WO 2007061631. The nucleic acid molecules encoding the *Pichia pastoris PMT1* and *PMT4* are shown in SEQ ID NOs: 47 and 49. The amino acid sequences of the *Pichia pastoris PMT*1 and *PMT4* are shown in SEQ ID NOs: 48 and 50. The primers and DNA templates used for making the *PMT* deletions using the PCR overlap method are listed below.

To make a *PMT1* knockout, the following procedure was followed. Three PCR reactions were set up. PCR reaction A comprised primers PMT1-KO1: 5'-TGAACCCATCT GTAAATAGAATGC-3' (SEQ ID NO: 17) and PMT1-KO2: 5'-GTGTCACCTAAATCGTA TGTGCCCATTTACTGGA AGCTGCTAACC-3' (SEQ ID NO: 18) and *Pichia pastoris* NRRL-Y11430 genomic DNA as the template. PCR reaction B comprised primers PMT1-KO3: 5'-CTCCCTATAGTGAGTCGTATTCATCATTGTACTTT GGTATATTGG-3' (SEQ ID NO: 19) and PMT1-KO4: 5'-TATTTGTACCTGCGTCCTGTTTGC-3' (SEQ ID NO: 20) and Pichia *pastoris* NRRL-Y11430 genomic DNA as the template. PCR reaction C comprised primers PR29: 5'-CACATACGATTTAGGTGACAC-3' (SEQ ID NO: 21) and PR32: 5'-AATAC GACTCACTATAGGGAG-3' (SEQ ID NO: 22) and the template was plasmid vector pAG25 (Goldstein and McCusker, Yeast 15: 1541 (1999)). The conditions for all three PCR reactions were one cycle of 98°C for two minutes, 25 cycles of 98°C for 10 seconds, 54°C for 30 seconds, and 72°C for four minutes, and followed by one cycle of 72°C for 10 minutes.

Then in a second PCR reaction, primers PMT1-KO1 + PMT1-KO4 from above were mixed with the PCR-generated fragments from PCR reactions A, B, and C above. The PCR conditions were one cycle of 98°C for two minutes, 30 cycles of 98°C for 10 seconds, 56°C for 10 seconds, and 72°C for four minutes, and followed by one cycle of 72°C for 10 minutes.

The fragment generated in the second PCR reaction was gel-purified and used to transform appropriate strains in which the *Pichia pastoris PDI1* gene has been replaced with an expression cassette encoding the human PDI1 protein. Selection of transformants was on rich media plates (YPD) containing 100 µg/mL nourseothricin.

To make a *PMT4* knockout, the following procedure was followed. Three PCR reactions were set up. PCR reaction A comprised primers PMT4-KO1: 5'-TGCTCTCCGCGTGCAATAGAAACT -3' (SEQ ID NO: 23) and PMT4-KO2: 5'-CTCCCTATAGTGAGTCGTATTCACAGTGTACCATCT TTCATCTCC -3' (SEQ ID NO: 24) and *Pichia pastoris* NRRL-Y11430 genomic DNA as the template. PCR reaction B comprised primers PMT4-KO3: 5'-GTGTCACCTAAATCGTATGTGAACCTAACTCTAA TTCTTCAAA GC -3' (SEQ ID NO: 25) and PMT4-KO4: 5'-ACTAGGGTATATAATTCCCAAGGT -3' (SEQ ID NO: 26) and *Pichia pastoris* NRRL-Y11430 genomic DNA as the template. PCR reaction C comprised primers PR29: 5'-CACATACGATTTAGGTGACAC-3' (SEQ ID NO: 21) and PR32: 5'-AATACGACTCACTATAGGGAG-3' (SEQ ID NO: 22) and plasmid vector pAG25 as the template.

The conditions for all three PCR reactions were one cycle of 98°C for two minutes, 25 cycles of 98°C for 10 seconds, 54°C for 30 seconds, and 72°C for four minutes, and followed by one cycle of 72°C for 10 minutes.

Then in a second PCR reaction, primers PMT4-KO1 + PMT4-K04 from above were mixed with the PCR-generated fragments from PCR reactions A, B, and C above. The PCR conditions were one cycle of 98°C for two minutes, 30 cycles of 98°C for 10 seconds, 56°C for 10 seconds, and 72°C for four minutes, and followed by one cycle of 72°C for 10 minutes.

The fragment generated in the second PCR reaction was gel-purified and used to transform appropriate strains in which the *Pichia pastoris PDI1* gene has been replaced with an expression cassette encoding the human PDI protein. Selection of transformants was on rich media plates (YPD) containing 100 µg/mL nourseothricin.

To test the ability of the strains to produce antibodies with reduced *O-*glycosylation, expression vectors encoding an anti-Her2 antibody and an anti-CD20 antibody were constructed.

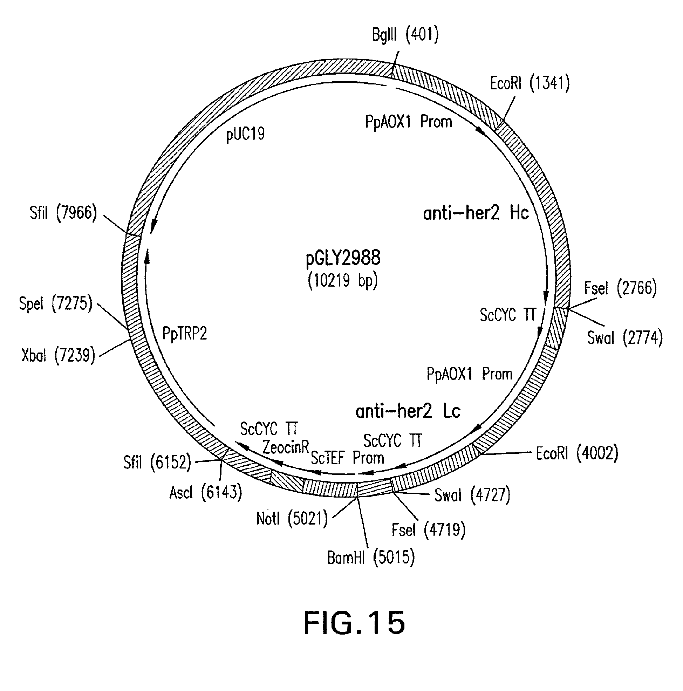

Expression/integration plasmid vector pGLY2988 contains expression cassettes encoding the heavy and light chains of an anti-Her2 antibody. Anti-Her2 heavy (HC) and light (LC) chains fused at the N-terminus to α-MAT pre signal peptide were synthesized by GeneArt AG. Each was synthesized with unique 5' *Eco*RI and 3' *Fse*1 sites. The nucleotide and amino acid sequences of the anti-Her2 HC are shown in SEQ ID Nos: 29 and 30, respectively. The nucleotide and amino acid sequences of the anti-Her2 LC are shown in SEQ ID Nos: 31 and 32, respectively. Both nucleic acid molecule fragments encoding the HC and LC fusion proteins were separately subcloned using 5' *Eco*R1 and 3' *Fse*1 unique sites into an expression plasmid vector pGLY2198 (contains the *Pichia pastoris TRP2* targeting nucleic acid molecule and the Zeocin-resistance marker) to form plasmid vector pGLY2987 and pGLY2338, respectively. The LC expression cassette encoding the LC fusion protein under the control of the *Pichia pastoris* AOX1 promoter and *Saccharomyces cerevisiae* Cyc terminator was removed from plasmid vector pGLY2338 by digesting with *Bam*HI and *Not*I and then cloning the DNA fragment into plasmid vector pGLY2987 digested with *Bam*H1 and *Not*1, thus generating the final expression plasmid vector pGLY2988 (Figure 15).

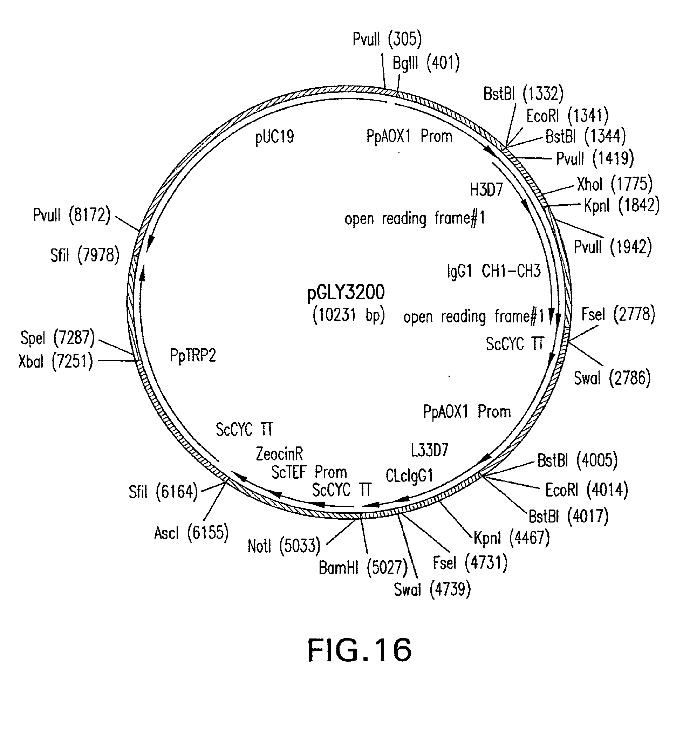

Expression/integration plasmid vector pGLY3200 (map is identical to pGLY2988 except LC and HC are anti-CD20 with α-amylase signal sequences). Anti-CD20 sequences were from GenMab sequence 2C6 except Light chain (LC) framework sequences matched those from VKappa 3 germline. Heavy (HC) and Light (LC) variable sequences fused at the N-terminus to the α-amylase (from *Aspergillus niger)* signal peptide were synthesized by GeneArt AG. Each was synthesized with unique 5' *Eco*R1 and 3' *Kpn*I sites which allowed for the direct cloning of variable regions into expression vectors containing the IgGI and V kappa constant regions. The nucleotide and amino acid sequences of the anti-CD20 HC are shown in SEQ ID Nos: 37 and 38, respectively. The nucleotide and amino acid sequences of the anti-CD20 LC are shown in SEQ ID Nos: 35 and 36, respectively. Both HC and LC fusion proteins were subcloned into IgG1 plasmid vector pGLY3184 and V Kappa plasmid vector pGLY2600, respectively, (each plasmid vector contains the *Pichia pastoris TRP2* targeting nucleic acid molecule and Zeocin-resistance marker) to form plasmid vectors pGLY3192 and pGLY3196, respectively. The LC expression cassette encoding the LC fusion protein under the control of the *Pichia pastorir* AOX1 promoter and *Saccharomyces cerevisiae* Cyc terminator was removed from plasmid vector pGLY3196 by digesting with *Bam*HI and *Not*I and then cloning the DNA fragment into plasmid vector pGLY3192 digested with *Bam*HI and *Not*I*,* thus generating the final expression plasmid vector pGLY3200 (Figure 16).

Transformation of appropriate strains disclosed herein with the above anti-Her2 or anti-CD20 antibody expression/integration plasmid vectors was performed essentially as follows. Appropriate *Pichia pastoris* strains were grown in 50 mL YPD media (yeast extract (1%), peptone (2%), dextrose (2%)) overnight to an OD of between about 0.2 to 6. After incubation on ice for 30 minutes, cells were pelleted by centrifugation at 2500-3000 rpm for 5minutes. Media was removed and the cells washed three times with ice cold sterile 1M sorbitol before resuspension in 0.5 ml ice cold sterile 1M sorbitol. Ten µL linearized DNA (5-20 ug) and 100 µL cell suspension was combined in an electroporation cuvette and incubated for 5 minutes on ice. Electroporation was in a Bio-Rad GenePulser Xcell following the preset *Pichia pastoris* protocol (2 kV, 25 µF, 200 Ω), immediately followed by the addition of 1 mL YPDS recovery media (YPD media plus 1 M sorbitol). The transformed cells were allowed to recover for four hours to overnight at room temperature (24°C) before plating the cells on selective media.

Cell Growth conditions of the transformed strains for antibody production was generally as follows. Protein expression for the transformed yeast strains was carried out at in shake flasks at 24°C with buffered glycerol-complex medium (BMGY) consisting of 1% yeast extract, 2% peptone, 100 mM potassium phosphate buffer pH 6.0, 1.34% yeast nitrogen base, 4 x 10-5 % biotin, and 1% glycerol. The induction medium for protein expression was buffered methanol-complex medium (BMMY) consisting of 1% methanol instead of glycerol in BMGY. Pmt inhibitor (Pmti-3) in methanol was added to the growth medium to a final concentration of 0.2 µM, 2 µM, or 20µM at the time the induction medium was added. Cells were harvested and centrifuged at 2,000 rpm for five minutes.

SixFors Fermenter Screening Protocol followed the parameters shown in Table 4.

| Table 4 | | |
|---|---|---|
| SixFors Fermenter Parameters | | |
| Parameter | Set-point | Actuated Element |
| pH | 6.5 ± 0.1 | 30% NH₄OH |
| Temperature | 24 ± 0.1 | Cooling Water & Heating Blanket |
| Dissolved 02 | n/a | Initial impeller speed of 550 rpm is ramped to 1200 rpm over first 10 hr, then fixed at 1200 rpm for remainder of run |

At time of about 18 hours post-inoculation, SixFors vessels containing 350 mL media A (See Table 6 below) plus 4% glycerol were inoculated with strain of interest. A small dose (0.3 mL of 0.2 mg/mL in 100% methanol) of Pmti-3 (5-[[3-(1-Phenyl-2-hydroxy)ethoxy)-4-(2- phenylethoxy)]phenyl]methylene]-4-oxo-2-thioxo-3-thiazolidineacetic Acid) (*See* Published International Application No. WO 2007061631) was added with inoculum. At time about 20 hour, a bolus of 17 mL 50% glycerol solution (Glycerol Fed-Batch Feed, *See* Table 7 below) plus a larger dose (0.3 mL of 4 mg/mL) of Pmti-3 was added per vessel. At about 26 hours, when the glycerol was consumed, as indicated by a positive spike in the dissolved oxygen (DO) concentration, a methanol feed (See Table 8 below) was initiated at 0.7 mL/hr continuously. At the same time, another dose of Pmti-3 (0.3 mL of 4 mg/mL stock) was added per vessel. At time about 48 hours, another dose (0.3 mL of 4 mg/mL) of Pmti-3 was added per vessel. Cultures were harvested and processed at time about 60 hours post-inoculation.

| Table 5 | | |
|---|---|---|
| Composition of Media A | | |
| Martone L-1 | 20 | g/L |
| Yeast Extract | 10 | g/L |
| KH₂PO4 | 11.9 | g/L |
| K₂HPO₄ | 2.3 | g/L |
| Sorbitol | 18.2 | g/L |
| Glycerol | 40 | g/L |
| Antifoam Sigma 204 | 8 | drops/L |
| 10X YNB w/Ammonium Sulfate w/o Amino Acids (134 g/L) | 100 | mL/L |
| 250X Biotin (0.4 g/L) | 10 | mL/L |
| 500X Chloramphenicol (50 g/L) | 2 | mL/L |
| 500X Kanamycin (50 g/L) | 2 | mL/L |

| Table 6 | | |
|---|---|---|
| Glycerol Fed-Batch Feed | | |
| Glycerol | 50 | % m/m |
| PTM1 Salts (see Table IV-E below) | 12.5 | mL/L |
| 250X Biotin (0.4 g/L) | 12.5 | mL/L |

| Table 7 | | |
|---|---|---|
| Methanol Feed | | |
| Methanol | 100 | % m/m |
| PTM1 Salts | 12.5 | mL/L |
| 250X Biotin (0.4 g/L) | 12.5 | mL/L |

| Table 8 | | |
|---|---|---|
| PTM1 Salts | | |
| CuSO4-5H2O | 6 | g/L |
| NaI | 80 | mg/L |
| MnSO4-7H2O | 3 | g/L |
| NaMoO4-2H2O | 200 | mg/L |
| H3BO3 | 20 | mg/L |
| CoCl2-6H2O | 500 | mg/L |
| ZnC12 | 20 | g/L |
| FeSO4-7H2O | 65 | g/L |
| Biotin | 200 | mg/L |
| H2SO4 (98%) | 5 | mL/L |

*O*-glycan determination was performed using a Dionex-HPLC (HPAEC-PAD) as follows. To measure *O*-glycosylation reduction, protein was purified from the growth medium using protein A chromatography (Li et al. Nat. Biotechnol. 24(2):210-5 (2006)) and the *O*-glycans released from and separated from protein by alkaline elimination (beta -elimination) (Harvey, Mass Spectrometry Reviews 18: 349- 451 (1999)). This process also reduces the newly formed reducing terminus of the released *O*-glycan (either oligomannose or mannose) to mannitol. The mannitol group thus serves as a unique indicator of each *O*- glycan. 0.5 nmole or more of protein, contained within a volume of 100 µL PBS buffer, was required for beta elimination. The sample was treated with 25 µL alkaline borohydride reagent and incubated at 50°C for 16 hours. About 20 uL arabitol internal standard was added, followed by 10 µL glacial acetic acid. The sample was then centrifuged through a Millipore filter containing both SEPABEADS and AG 50W-X8 resin and washed with water. The samples, including wash, were transferred to plastic autosampler vials and evaporated to dryness in a centrifugal evaporator. 150 µL 1% AcOH/MeOH was added to the samples and the samples evaporated to dryness in a centrifugal evaporator. This last step was repeated five more times. 200 µL of water was added and 100 µL of the sample was analyzed by high pH anion-exchange chromatography coupled with pulsed electrochemical detection-Dionex HPLC (HPAEC-PAD). Average *O*-glycan occupancy was determined based upon the amount of mannitol recovered.

Figures 4-7 show that the *Pichia pastoris* strains in which the endogenous *PDI1* is replaced with a heterologous PDI from the same species as the recombinant protein to be produced in the strain and in which native *PMT1* or *PMT4* genes have been deleted are capable of producing recombinant human antibody at higher titers and with reduced *O*-glycosylation compared to production of the antibodies in strains that contain the endogenous *PDI1* and do not have deletions of the *PMT1* or *PMT4* genes.

Figures 4A and 4B shows representative results from shakeflask (A) and 0.5L bioreactor (B) expression studies in which human anti-Her2 antibody was produced in *Pichia pastoris* strains in which the human PDI gene (hPDI) replaced the endogenous *PDI1* and strains in which the human PDI replaced the endogenous *PDI1* and the *PMT1* gene disrupted (hPDI + Δpmt1). Antibodies were recovered and resolved by polyacrylamide gel electrophoresis on non-reducing and reducing polyacrylamide gels. Under non-reducing conditions, the antibodies remained intact whereas under reducing conditions, the antibodies were resolved into HCs and LCs. Lanes 1-2 shows antibodies produced from two clones produced from transformation of strain yGLY2696 with plasmid vector pGLY2988 encoding the anti-Her2 antibody and lanes 3-6 shows the antibodies produced from four clones produced from transformation of strain yGLY2696 in which the *PMT1* gene was deleted and with plasmid vector pGLY2988 encoding the anti-Her2 antibody. The Figures showed that the *PMT1* deletion improved antibody yield.

Figure 5 shows representative results from a shakeflask expression study in which human anti-DKK1 antibody was produced in *Pichia pastoris* strains in which the human PDI gene (hPDI) replaced the endogenous *PDI1* and strains in which the human PDI replaced the endogenous *PDI1* and the *PMT1* gene is disrupted (hPDI + Δpmt1). Antibodies were recovered and resolved by polyacrylamide gel electrophoresis on non-reducing and reducing polyacrylamide gels. Under non-reducing conditions, the antibodies remained intact whereas under reducing conditions, the antibodies were resolved into HCs and LCs. Lanes 1 and 3 shows antibodies produced from two clones produced from transformation of strains yGLY2696 and yGLY2690 with plasmid vector pGLY2260 encoding the anti-DKK1 antibody and lanes 2 and 4 shows the antibodies produced from two clones produced from transformation of strains yGLY2696 and yGLY2690 in which the *PMT1* gene was deleted with plasmid vector pGLY2260 encoding the anti-DKK1 antibody. The figure shows that the *PMT1* deletion improved antibody yield.

Figure 6 shows results from a 0.5 L bioreactor expression study where human anti-Her2 antibody is produced in *Pichia pastoris* strains in which the human PDI replaced the endogenous *PDI1* and the *PMT4* gene is disrupted (HPDI + Δpmt4), and strains that express only the endogenous *PDI1* but in which the *PMT4* gene is disrupted (PpPDI + Δpmt4). Antibodies were recovered and resolved by polyacrylamide gel electrophoresis on non-reducing polyacrylamide gels. Lanes 1 and 2 shows antibodies produced from two clones from transformation of strain yGLY24-1 with plasmid vector pGLY2988 encoding the anti-Her2 antibody and lanes 3-5 show anti-Her2 antibodies produced from three clones produced from transformation of strain yGLY2690 in which the *PMT4* gene was deleted. The figure shows that the *PMT4* deletion improved antibody yield but in order to have that improvement in yield, the cell must also have the endogenous *PDI1* gene replaced with an expression cassette encoding the human PDI.

Figure 7 shows results from a shakeflask expression study where human anti-CD20 antibody is produced in *Pichia pastoris* strains in which the human PDI replaced the endogenous *PDI1* and the *PMT4* gene disrupted (hPDI + Δpmt4) and strains that express only the endogenous PDI1 but in which the *PMT4* gene is disrupted (PpPDI + Δpmt4). Antibodies were recovered and resolved by polyacrylamide gel electrophoresis on non-reducing and reducing polyacrylamide gels. Lane 1 shows antibodies produced from strain yGLY24-1 transformed with plasmid vector pGLY3200 encoding the anti-CD20 antibody; lanes 2-7 show anti-CD20 antibodies produced from six clones produced from transformation of strain yGLY2690 in which the *PMT4* gene was deleted. The figure shows that the *PMT4* deletion improved antibody yield but in order to have that improvement in yield, the cell must also have the endogenous PDII gene replaced with an expression cassette encoding the human PDI.

### EXAMPLE 3

This example describes a chimeric BiP gene, in which the human ATPase domain is replaced by the ATPase domain of *Pichia pastoris* KAR2, fused to the human BiP peptide binding domain, under the control of the KAR2, or other ER-specific promoter from *Pichia pastoris.* The nucleotide and amino acid sequences of the human BiP are shown in Table 11 as SEQ ID NOs: 55 and 56, respectively. The nucleotide and amino acid sequences of the chimeric BiP are shown in Table 11 as SEQ ID NOs: 57 and 58, respectively. Further improvements in yield may be obtained by combining the replacement of the endogenous PDI1 gene, as described above, with the use of chimeric BiP and human ERdj3 (SEQ D NOs: 76 and 77, respectively).

### EXAMPLE 4

This example demonstrates that occupancy of O-glycans in proteins produced in the above strains expressing the human PDI in place of the *Pichia pastoris* PDI1 can be significantly reduced when either the *Pichia pastoris* Golgi Ca²⁺ ATPase (PpPMRI) or the *Arabidopsis thaliana* ER Ca²⁺ ATPase (AtECA1) is overexpressed in the strains. In this example, the effect is illustrated using glycoengineered *Pichia pastoris* strains that produce antibodies having predominantly Man₅GlcNAc₂ N-glycans.

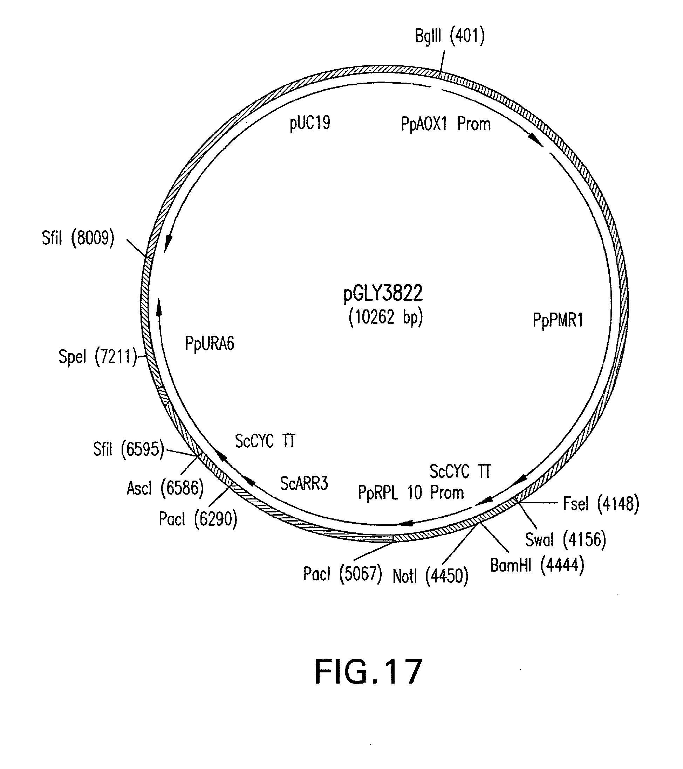

An expression cassette encoding the PpPMR1 gene was constructed as follows. The open reading frame of *P. pastoris* Golgi Ca²⁺ ATPase (PpPMR1) was PCR amplified from *P. pastoris* NRRL-Y11430 genomic DNA using the primers (PpPMR1/UP: 5'-GAATTCAT GACAGCTAATGAAAATCCTTTTGAGAATGAG-3' (SEQ ID NO: 64) and PpPMR1/LP: 5'-GGCCGGCCTCAAACAGCCATGCTGTATCCATTGTATG -3' (SEQ ID NO: 65). The PCR conditions were one cycle of 95°C for two minutes; five cycles of 95°C for 10 seconds, 52°C for 20 seconds, and 72°C for 3 minutes; 20 cycles of 95°C for 10 seconds, 55°C for 20 seconds, and 72°C for 3 minutes; followed by 1 cycle of 72°C for 10 minutes. The resulting PCR product was cloned into pCR2.1 and designated pGLY3811. PpPMR1 was removed from pGLY3811 by digesting with plasmid with *Pst*I and *Fse*I) and the *Pst*I end had been made blunt with T4 DNA polymerase prior to digestion with *Fse*I. The DNA fragment encoding the PpPMR1 was cloned into pGFI30t digested with *EcoRI* with the ends made blunt with T4 DNA polymerse and *Fse*I to generate pGLY3822 in which the PpPMR1 is operably linked to the AOX1 promoter. Plasmid pGLY3822 targets the *Pichio pastoris URA6* locus. Plasmid pGLY3822 is shown in Figure 17. The DNA sequence of PpPMR1 is set forth in SEQ ID NO: 60 and the amino acid sequence of the PpPMR1 is shown in SEQ ID NO: 61.

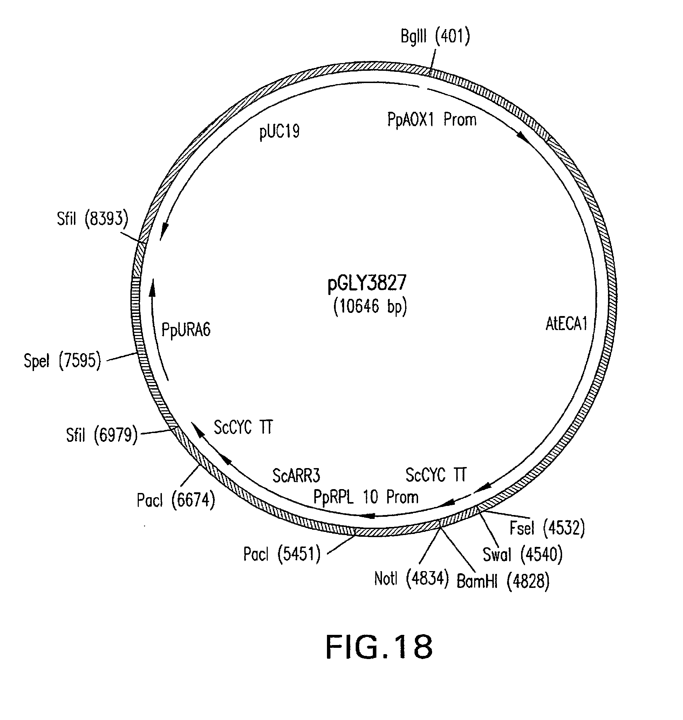

An expression cassette encoding the *Aabidopsis thaliana* ER Ca²⁺ ATPase (AtECA1) was constructed as follows. A DNA encoding AtECA1 was synthesized from GeneArt AG (Regensburg, Germany) and cloned to make pGLY3306. The synthesized AtECA1 was removed from pGLY3306 by digesting with *Mly*I and *Fse*I and cloning the DNA fragment encoding the AtECA1 into pGFI30t digested with EcoRI with the ends made blunt with T4 DNA polymerase and *Fse*I to generate integration/expression plasmid pGLY3827. Plasmid pGLY3827 targets the *Pichia pastoris URA6* locus. Plasmid pGLY3827 is shown in Figure 18. The DNA sequence of the AtECA1 was codon-optimized for expression in *Pichia pastoris* and is shown in SEQ ID NO: 62. The encoded AtECA1 has the amino acid sequence set forth in SEQ ID NO: 63.

Integration/expression plasmid pGLY3822 (contains expression cassette encoding PpPMR1) or pGLY3827 (contains expression cassette encoding AtECA1) was linearized with *Spel* and transformed into *Pichia pastoris* strain **yGLY3647** or **yGLY3693** at the *URA6* locus. The genomic integration of pGLY3822 or pGLY3827 at *URA6* locus was confirmed by colony PCR (cPCR) using primers, 5'AOX1 (5'-GCGACTGGTTCCAATTGACAAGCTT-3' (SEQ ID NO: 66) and PpPMR1/cLP (5'-GGTTGCTCTCGTCGATACTCAAGTGGGAAG-3' (SEQ ID NO: 67) for confirming PpPMR1 integration into the URA6 locus, and 5'AOX1 and AtECA1/cLP (5'-GTCGGCTGGAACCTTATCACCAACTCTCAG-3' (SEQ ID NO: 68) for confirming integration of AtECA1 into the *URA6* locus. The PCR conditions were one cycle of 95°C for 2 minutes, 25 cycles of 95°C for 10 seconds, 55°C for 20 seconds, and 72°C for one minute; followed by one cycle of 72°C for 10 minutes.

Strain **yGLY8238** was generated by transforming strain yGLY3647 with integration/expression plasmid pGLY3833 encoding the PpPMR1 and targeting the *URA6* locus. In strain yGLY3647, the *Pichio pastoris* PDI1 chaperone gene has been replaced with the human PDI gene as described in Example 1 and shown in Figures 3A and 3B.

Strain **yGLY8240** was generated by transforming strain yGLY3647 with plasmid pGLY3827 encoding the AtECA1 and targeting the *URA6* locus. The geneology of the strains is shown in Figures 3A and 3B.

The strains were evaluated for the effect the addition of PpPMR1 or AtECA1 to the humanized chaperone strains might have on reducing *O*-glycosylation of the antibodies produced by the strains. As shown in Table 9 the addition of either PpPMR1 or AtECA1 into strain yGLY3647 effected a significant reduction in *O*-glycosylation occupancy compared to strain yGLY3647 expressing the human PDI in place of the *Pichia pastoris* PDI1 or strain yGLY2263 expressing only the endogenous PDI1 but capable of making antibodies with a Man₅GlcNAc₂ glycoform as strain yGLY3647. The results also suggest that yeast strains that express its endogenous PDI1 and not the human PDI and overexpress a Ca2⁺ ATPase will produce glycoproteins with reduced O-glycan occupancy.

| Table 9 | | | | |
|---|---|---|---|---|
| Strain | yGLY2263 | yGLY3647 | yGLY3647 + Ca²⁺ ATPase | |
| | | | yGLY8240 | yGLY8238 |
| | | | AtECA1 | PpPMR1 |
| *O*-glycan occupancy (H2+L2: anti-DKK1) | 23.7 | 9.2 | 5.54 | 6.28 |
| *O*-glycan occupancy was determined by Mannitol assay. | | | | |

### EXAMPLE 5

A DNA fragment encoding the human calreticulin (hCRT) without its native signal sequence was PCR amplified from a human liver cDNA library (BD Biosciences, San Jose, CA) using primers hCRT-BstZ17I-HA/UP: 5'-GTATACCCATACGACGTCCCAGACTA CGCTGAGCCCGCCGTCTACTTCAAGGAGC-3' (SEQ ID NO: 73) and hCRT-PacI/LP: 5'-TTAATTAACTACAGCTCGTCATGGGCCTGGCCGGGGACATCTTCC-3' (SEQ ID NO: 74). The PCR conditions were one cycle of 98°C for two min; 30 cycles of 98°C for 10 seconds, 55°C for 30 seconds, and 72°C for two minutes, and followed by one cycle of 72°C for 10 minutes. The resulting PCR product was cloned into pCR2.1 Topo vector to make pGLY1224. The DNA encoding the hCRT further included modifications such that the encoded truncated hCRT has an HA tag at its N-terminus and HDEL at its C-teminus. The DNA encoding the hCRT was released from pGLY1224 by digestion with *Bst*Z17I and *Pac*I and the DNA fragment cloned into an expression vector pGLY579, which had been digested with *Not*I and *Pac*I, along with a DNA fragment encoding the *S. cerevisiae* alpha-mating factor pre signal sequence having *Not*I and *Pac*I compatible ends to create pGLY1230. This plasmid is an integration/expression plasmid that encodes the hCRT with the *S. cerevisiae* alpha-mating factor pre signal sequence and HA tag at the N-terminus and an HDEL sequence at its C-terminus operably linked to the Pichia pastoris GAPDH promoter and targeting the HIS3 locus of *Pichia pastoris.*

A DNA fragment encoding the human ERp57 (hERp57) was synthesized by GeneArt AG having *Not*I and *Pac*I compatible ends. The DNA fragment was then cloned into pGLY129 digested with *Not*I and *Pac*I to produce pGLY1231. This plasmid encodes the hERp57 operably linked to the Pichia pastoris PMA1 promoter.

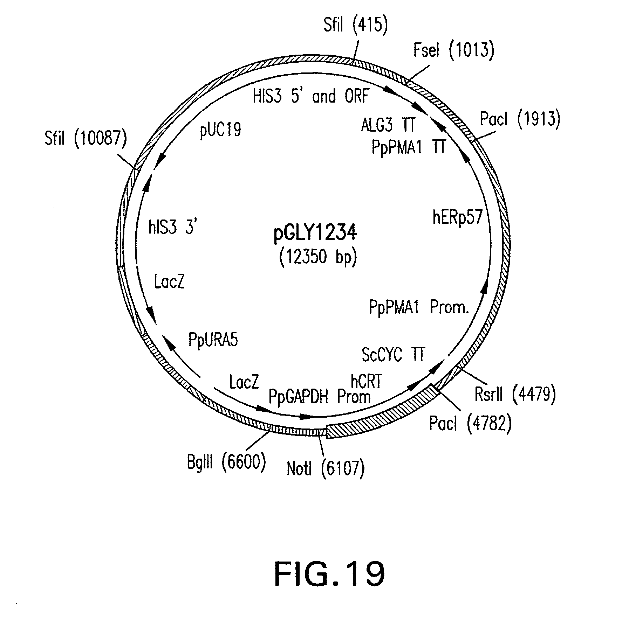

Plasmid pGLY1231 was digested with *Swa*I and the DNA fragment encoding the hERp57 was cloned into plasmid pGLY1230 digested with *Pme*I. Thus, integration/expression plasmid pGLY1234 encodes both the hCRT and hERp57. Plasmid pGLY1234 is shown in Figure 19.

Strain **yGLY3642** was generated by counterselecting strain yGLY2690 in the presence of 5'FOA, a *URA5* auxotroph.

Strain **yGLY3668** was generated by transforming yGLY3642 with integration/expression plasmid pGLY1234 encoding the hCRT and hERp57 and which targets the *HIS3* locus.

Strain **yGLY3693** was generated by transforming strain yGLY3668 with integration/expression plasmid pGLY2261, which targets an expression cassette encoding the anti-DKK1 antibody to the *TRP2* locus.

Strain **yGLY8239** was generated by transforming strain yGLY3693 with integration/expression plasmid pGLY3833 encoding the PpPMR1 and targeting the *URA6* locus.

Strain **yGLY8241** was generated by transforming strain yGLY3693 with integration/expression plasmid pGLY3827 encoding the AtECA1 and targeting the *URA6* locus.

The geneology of the strains described in this example are shown in Figures 3A and 3B.

The above strains were evaluated to see whether the addition of hCRT and hERp57 to the humanized chaperone strains expressing PpPMR1 or AtECA1 of the previous example might effect a further reduction in *O*-glycan occupancy of the antibodies produced. As shown in Table 10, in strain yGLY3693 expressing hCRT and hERp57 alone, there was about a 2-fold decrease in *O*-glycan occupancy, which was further decreased up to a 4-fold in strains that further expressed PpPMR1 or AtECA1. The results also suggest that yeast strains that express its endogenous PDI1 and not the human PDI and overexpress a Ca²⁺ ATPase will produce glycoproteins with reduced *O*-glycan occupancy.

| Table 10 | | | | |
|---|---|---|---|---|
| Strain | yGLY2263 | yGLY3693 | yGLY3693 + Ca²⁺ ATPase | |
| | | | yGLY8241 | yGLY8239 |
| | | | AtECA1 | PpPMR1 |
| *O*-glycan occupancy (H2+L2: anti-DKK1) | 23.7 | 10.43 | 5.59 | 7.86 |
| *O*-glycan occupancy was determined by Mannitol assay. | | | | |

| Table 11 | | |
|---|---|---|
| BRIEF DESCRIPTION OF THE SEQUENCES | | |
| SEQ ID NO: | Description | Sequence |
| 1 | PCR primer hPDI/UP1 | AGCGCTGACGCCCCCGAGGAGGAGGACCAC |
| 2 | PCR primer hPDI/LP-PacI | CCTTAATTAATTACAGTTCATCATGCACAGCTTTCTGATCAT |
| 3 | PCR primer PB248 | |
| 4 | PCR primer PB249 | ATGTTTA AACGTGAGGATTACTGGTGATGAAAGAC |
| 5 | PCR primer PB250 | AGACTAGTCTATTTGGAG ACATTGACGGATCCAC |
| 6 | PCR primer PB251 | |
| 7 | PCR primer PpPDI/UPi-1 | GGTGAGGTTGAGGTCCCAAGTGACTATCAAGGTC |
| 8 | PCR primer PpPDI/LPi-1 | GACCTTGATAGTCACTTGGGACCTCAACCTCACC |
| 9 | PCR primer PpPDI/UPi-2 | CGCCAATGATGAGGATGCCTCTTCAAAGGTTGTG |
| 10 | PCR primer PpPDI/LPi-2 | CACAACCTTTGAAGAGGCATCCTCATCATTGGCG |
| 11 | PCR primer PpPDI-5'/UP | GGCGATTGCATTCGCGAC TGTATC |
| 12 | PCR primer hPDI-3'/LP | CCTAGAGAGCGGTGG CCAAGATG |
| 13 | PCR primer hPDI/UP | GTGGCCACACCAGGGGGC ATGGAAC |
| 14 | PCR primer hPDI-3'/LP | CCTAGAGAGCGGTGG CCAAGATG |
| 15 | PCR primer hGRP94/UP1 | AGCGCTGACGATGAAGTTGATGTGGATGGTACA GTAG |
| 16 | PCR primer hGRP94/LP1 | GGCCGGCCTTACAATTCATCATG TTCAGCTGTAGATTC |
| 17 | PCR primer PMT1-KO1 | TGAACCCATCTGTAAATAGAATGC |
| 18 | PCR primer PMT1-KO2 | |
| 19 | PCR primer PMT1-KO3 | |
| 20 | PCR primer PMT1-KO4 | TATTTGTACCTGCGTCCTGTTTGC |
| 21 | PCR primer PR29 | CACATACGATTTAGGTGACAC |
| 22 | PCR primer PR32 | AATACGACTCACTATAGGGAG |
| 23 | PCR primer PMT4-KO1 | TGCTCTCCGCGTGCAATAGAAACT |
| 24 | PCR primer PMT4-KO2 | |
| 25 | PCR primer PMT4-KO3 | |
| 26 | PCR primer PMT4-KO4 | ACTAGGGTATATAATTCCCAAGGT |
| 27 | *Saccharomyces cerevisiae* mating factor pre-signal peptide (DNA) | |
| 28 | *Saccharomyces cerevisiae* mating factor pre-signal peptide (protein) | MRFPSIFTAVLFAASSALA |
| 29 | Anti-Her2 Heavy chain (VH + IgG1 constant | |
| | region) (DNA) | |
| 30 | Anti-Her2 Heavy chain (VH + IgG1 constant region) (protein) | |
| | | |
| 31 | Anti-Her2 light chain (VL + Kappa constant region) (DNA) | |
| 32 | Anti-Her2 light chain (VL + Kappa constant region) | |
| 33 | Alpha amylase signal peptide (from *Aspergillus niger α-*amylase) (DNA) | |
| 34 | Alpha amylase signal peptide (from *Aspergillusniger* α-amylase) | MVAWWSLFLY GLQVAAPALA |
| 35 | Anti-CD20 Light chain Variable Region (DNA) | |
| 36 | Anti-CD20 Light chain Variable Region | |
| 37 | Anti-CD20 Heavy chain Variable Region (DNA) | |
| 38 | Anti-CD20 Heavy chain Variable Region | |
| 39 | human PDI Gene (DNA) | |
| | | |
| 40 | human PDI Gene (protein) | |
| | | |
| 41 | *Pichia pastoris* PDI1 Gene (DNA) | |
| | | |
| 42 | *Pichia pastoris* PDI1 Gene (protein) | |
| 43 | human ERO1α Gene (DNA) | |
| | | |
| 44 | human ERO1α Gene (protein) | |
| 45 | human GRP94 Gene (DNA) | |
| | | |
| | | |
| 46 | human GRP94 Gene (protein) | |
| 47 | PpPMT1 gene (DNA) CDS 3016-5385 | |
| | | |
| | | |
| | | |
| | | |
| | | |
| | | |
| 48 | PpPMT1 gene (protein) | |
| 49 | PpPMT4 (DNA) | |
| | CDS 3168-5394 | |
| | | |
| | | |
| | | |
| | | |
| | | |
| | | |
| 50 | PpPMT4 (protein) | |
| 51 | anti-DKK1 Heavy chain (VH+ IgG2m4) (α-amylase encoding sequences underlined) (DNA) | |
| | | |
| 52 | anti-DKK1 Heavy chain (VH + IgG2m4) (protein) | |
| 53 | anti-DKK1 Light chain (VL + lambda constant regions)(α-amylase encoding | |
| | sequences underlined) (DNA) | |
| 54 | anti-DKK1 Light chain (VL + lambda constant regions) (protein) | |
| 55 | Human BiP (DNA) | |
| | | |
| 56 | Human BiP (protein) ATPase domain underlined | |
| | | |
| 57 | Chimeric BiP (DNA) | |
| | | |
| 58 | Chimeric BiP (protein) ATPase domain underlined | |
| 59 | PpPDI1 promoter | |
| 60 | PpPMR1 | |
| | | |
| | | |
| 61 | PpPMR1 | |
| | | |
| 62 | *Arabidopsis Thaliana* AtECA1 (codon optimized for *Pichia pastoris*) | |
| | | |
| | | |
| 63 | AtECA1 | |
| 64 | ppPMR1/UP | GAATTCATGACAGCTAATGAAAATCCTTTTGAGAATGAG |
| 65 | PpPMR1/LP | GGCCGGCCTCAAACAGCCATGCTGTATCCATTGTATG |
| 66 | 5'AOX1 | GCGACTGGTTCCAATTGACAAGCTT |
| 67 | PpPMR1/cLP | GGTTGCTCTCGTCGATACTCAAGTGGGAAG |
| 68 | AtECA1/cLP | GTCGGCTGGAACCTTATCACCAACTCTCAG |
| 69 | Human calreticulin (hCRT)-DNA | |
| 70 | Human calreticulin | |
| | (hCRT)-protein | |
| 71 | Human ERp57 (DNA) | |
| | | |
| 72 | Human ERp57 (protein) | |
| 73 | hCRT-BstZ17I-HA/UP | |
| 74 | hCRT-PacI/LP | |
| 75 | Synthetic peptide that binds CRT | KLGFFKR |
| 76 | hERdj3 (DNA) | |
| | | |
| 77 | hERdj3 (protein) | |

While the present invention is described herein with reference to illustrated embodiments, it should be understood that the invention is not limited hereto. Those having ordinary skill in the art and access to the teachings herein will recognize additional modifications and embodiments within the scope thereof. Therefore, the present invention is limited only by the claims attached herein.

## Claims

1. A *Pichia pastoris* host cell comprising a deletion or disruption of an endogenous gene encoding a Protein Disulphide Isomerase (PDI), a nucleic acid molecule encoding a human PDI integrated into the genome of the host cell, and a nucleic acid molecule encoding a recombinant human protein, wherein the human PDI is overexpressed.

2. The host cell of Claim 1, wherein the function of at least one endogenous gene encoding a protein *O*-mannosyltransferase (*PMT*) protein has been reduced, disrupted, or deleted.

3. The host cell of Claim 1 or 2, wherein the host cell further includes a nucleic acid molecule encoding an endogenous or heterologous Ca²⁺ ATPase.

4. The host cell of Claim 1, 2 or 3, wherein the host cell further includes a nucleic acid molecule encoding an ERp57 protein and a nucleic acid molecule encoding a calreticulin protein.

5. The host cell of Claim 1, wherein the host cell further includes a nucleic acid molecule encoding a human ERO1α protein.

6. The host cell of any preceding Claim, wherein the recombinant protein is selected from the group consisting of enzymes, cytokines, growth factors, hormones, vaccines and antibodies.

7. The host cell of any preceding Claim, wherein the host cell has been genetically modified to express glycoproteins wherein the predominant *N*-glycans thereon are selected from Man₈GlcNAC₂, Man₇GlcNAc₂, Man₆GlcNAc₂, Man₅GlcNAC₂, GlcNAcMan₅GlcNAc₂, GalGlcNAcMan₅GlcNAc₂, NANAGalGlcNAcMan₅GlcNAc₂, Man₃GlcNAc₂, GlcNAc₍₁₋₄₎Man₃GlcNAc₂, Gal₍₁₋₄₎GlcNAc₍₁₋₄₎Man₃GlcNAc₂, NANA₍₁₋₄₎Gal₍₁₋₄₎GlcNAc₍₁₋₄₎Man₃GlcNAc₂ and mixtures thereof.

8. A method for producing a recombinant human protein comprising:
(a) providing a *Pichia pastoris* host cell comprising a deletion or disruption of an endogenous gene encoding a Protein Disulphide Isomerase (PDI) and a nucleic acid molecule encoding a human PDI integrated into the genome of the host cell, wherein human PDI is overexpressed;
(b) introducing a nucleic acid molecule into the host cell encoding the recombinant human protein; and
(c) growing the host cell under conditions suitable for producing the recombinant human protein.

9. A method for producing a recombinant human protein having reduced *O-*glycosylation comprising:
(a) providing a *Pichia pastoris* host cell comprising a deletion or disruption of an endogenous gene encoding a Protein Disulphide Isomerase (PDI) and a nucleic acid molecule encoding a human PDI integrated into the genome of the host cell, wherein the human PDI is overexpressed;
(b) introducing a nucleic acid molecule into the host cell encoding the recombinant human protein; and
(c) growing the host cell under conditions suitable for producing the recombinant human protein.

10. The method of Claim 8 or 9, wherein the function of at least one endogenous gene encoding a protein *O*-mannosyltransferase (*PMT*) protein has been reduced, disrupted, or deleted.

11. The method of Claim 8, 9 or 10, wherein the host cell further includes a nucleic acid molecule encoding an endogenous or heterologous Ca²⁺ ATPase.

12. The method of Claim 8, 9, 10 or 11, wherein the host cell further includes a nucleic acid molecule encoding an ERp57 protein and a nucleic acid molecule encoding a calreticulin protein.

13. The method of Claim 8, wherein the host cell further includes a nucleic acid molecule encoding a human EROlα protein.

14. The method of any one of Claims 8 to 12, wherein the recombinant protein is selected from the group consisting of enzymes, cytokines, growth factors, hormones, vaccines and antibodies.

15. The method of any one of Claims 8 to 12, wherein the host cell has been genetically modified to express glycoproteins wherein the predominant *N*-glycans thereon are selected from Man₈GlcNAc₂, Man₇GlcNAc₂, Man₆GlcNAc₂, Man₅GlcNAc₂, GlcNAcMan₅GlcNAc₂, GalGlcNAcMan₅GlcNAc₂, NANAGalGlcNAcMan₅GlcNAC₂, Man₃GlcNAc₂, GlcNAc₍₁₋₄₎Man₃GlcNAc₂, Gal₍₁₋₄₎GlcNAc₍₁₋₄₎Man₃GlcNAc₂, NANA₍₁₋₄₎Gal₍₁₋₄₎GlcNAc₍₁₋₄₎Man₃GlcNAc₂ and mixtures thereof.

## Patentansprüche

1. Eine *Pichia-pastoris*-Wirtszelle, umfassend eine Deletion oder Disruption eines endogenen Gens, das eine Protein-Disulfid-Isomerase (PDI) kodiert, ein Nukleinsäuremolekül, das eine menschliche PDI kodiert, integriert in das Genom der Wirtszelle, und ein Nukleinsäuremolekül, das ein rekombinantes menschliches Protein kodiert, wobei die menschliche PDI überexprimiert ist.

2. Die Wirtszelle nach Anspruch 1, wobei die Funktion von wenigstens einem endogenen Gen, das ein Protein-*O*-Mannosyltransferase(*PMT*)-Protein kodiert, reduziert, disruptiert oder deletiert worden ist.

3. Die Wirtszelle nach Anspruch 1 oder 2, wobei die Wirtszelle ferner ein Nukleinsäuremolekül enthält, das eine endogene oder heterologe Ca²⁺-ATPase kodiert.

4. Die Wirtszelle nach Anspruch 1, 2 oder 3, wobei die Wirtszelle ferner ein Nukleinsäuremolekül, das ein ERp57-Protein kodiert, und ein Nukleinsäuremolekül, das ein Calreticulin-Protein kodiert, enthält.

5. Die Wirtszelle nach Anspruch 1, wobei die Wirtszelle ferner ein Nukleinsäuremolekül enthält, das ein menschliches ERO1α-Protein kodiert.

6. Die Wirtszelle nach einem vorhergehenden Anspruch, wobei das rekombinante Protein ausgewählt ist aus der Gruppe, bestehend aus Enzymen, Zytokinen, Wachstumsfaktoren, Hormonen, Vakzinen und Antikörpern.

7. Die Wirtszelle nach einem vorhergehenden Anspruch, wobei die Wirtszelle genetisch modifiziert worden ist, um Glykoproteine zu exprimieren, wobei die vorherrschenden *N*-Glykane daran ausgewählt sind aus Man₈GlcNAc₂, Man₇GlcNAc₂, Man₆GlcNAc₂, Man₅GlcNAc₂, GlcNAcMan₅GlcNAc₂, GalGlcNAcMan₅GlcNAc₂, NANAGalGlcNAcMan₅GlcNAc₂, Man₃GlcNAc₂, GlcNAc₍₁₋₄₎Man₃GlcNAc₂, Gal₍₁₋₄₎GlcNAc₍₁₋₄₎Man₃GlcNAc₂, NANA₍₁₋₄₎Gal₍₁₋₄₎GlcNAc₍₁₋₄₎Man₃GlcNAc₂ und Mischungen davon.

8. Ein Verfahren zur Erzeugung eines rekombinanten menschlichen Proteins, umfassend:
(a) Bereitstellen einer *Pichia-pastoris*-Wirtszelle, die eine Deletion oder Disruption eines endogenen Gens, das eine Protein-Disulfid-Isomerase (PDI) kodiert, und ein Nukleinsäuremolekül, das eine menschliche PDI kodiert, integriert in das Genom der Wirtszelle, umfasst, wobei die menschliche PDI überexprimiert ist,
(b) Einbringen eines Nukleinsäuremoleküls, welches das rekombinante menschliche Protein kodiert, in die Wirtszelle und
(c) Kultivieren der Wirtszelle unter Bedingungen, die sich für die Erzeugung des rekombinanten menschlichen Proteins eignen.

9. Ein Verfahren zur Erzeugung eines rekombinanten menschlichen Proteins mit verringerter *O*-Glykosylierung, umfassend:
(a) Bereitstellen einer *Pichia-pastoris*-Wirtszelle, die eine Deletion oder Disruption eines endogenen Gens, das eine Protein-Disulfid-Isomerase (PDI) kodiert, und ein Nukleinsäuremolekül, das eine menschliche PDI kodiert, integriert in das Genom der Wirtszelle, umfasst, wobei die menschliche PDI überexprimiert ist,
(b) Einbringen eines Nukleinsäuremoleküls, welches das rekombinante menschliche Protein kodiert, in die Wirtszelle und
(c) Kultivieren der Wirtszelle unter Bedingungen, die sich für die Erzeugung des rekombinanten menschlichen Proteins eignen.

10. Das Verfahren nach Anspruch 8 oder 9, wobei die Funktion von wenigstens einem endogenen Gen, das ein Protein-*O*-Mannosyltransferase(*PMT*)-Protein kodiert, reduziert, disruptiert oder deletiert worden ist.

11. Das Verfahren nach Anspruch 8, 9 oder 10, wobei die Wirtszelle ferner ein Nukleinsäuremolekül enthält, das eine endogene oder heterologe Ca²⁺-ATPase kodiert.

12. Das Verfahren nach Anspruch 8, 9, 10 oder 11, wobei die Wirtszelle ferner ein Nukleinsäuremolekül, das ein ERp57-Protein kodiert, und ein Nukleinsäuremolekül, das ein Calreticulin-Protein kodiert, enthält.

13. Das Verfahren nach Anspruch 8, wobei die Wirtszelle ferner ein Nukleinsäuremolekül enthält, das ein menschliches ERO1α-Protein kodiert.

14. Das Verfahren nach einem der Ansprüche 8 bis 12, wobei das rekombinante Protein ausgewählt ist aus der Gruppe, bestehend aus Enzymen, Zytokinen, Wachstumsfaktoren, Hormonen, Vakzinen und Antikörpern.

15. Das Verfahren nach einem der Ansprüche 8 bis 12, wobei die Wirtszelle genetisch modifiziert worden ist, um Glykoproteine zu exprimieren, wobei die vorherrschenden *N*-Glykane daran ausgewählt sind aus Man₈GlcNAc₂, Man₇GlcNAc₂, Man₆GlcNAc₂, Man₅GlcNAc₂, GlcNAcMan₅GlcNAc₂, GalGlcNAcMan₅GlcNAc₂, NANAGalGlcNAcMan₅GlcNAc₂, Man₃GlcNAc₂, GlcNAc₍₁₋₄₎Man₃GlcNAc₂, Gal₍₁₋₄₎GlcNAc₍₁₋₄₎Man₃GlcNAc₂, NANA₍₁₋₄₎Gal₍₁₋₄₎GlcNAc₍₁₋₄₎Man₃GlcNAc₂ und Mischungen davon.

## Revendications

1. Cellule hôte de *Pichia pastoris* comprenant une délétion ou une perturbation d'un gène endogène codant une protéine disulfure isomérase (PDI), une molécule d'acide nucléique codant une PDI humaine intégrée dans le génome de la cellule hôte et une molécule d'acide nucléique codant une protéine recombinante humaine, dans laquelle la PDI humaine est surexprimée.

2. Cellule hôte selon la revendication 1, dans laquelle la fonction d'au moins un gène endogène codant une protéine, protéine *O*-mannosyltransférase (PMT), a été réduite, perturbée ou supprimée.

3. Cellule hôte selon la revendication 1 ou 2, où la cellule hôte comprend en outre une molécule d'acide nucléique codant une Ca²⁺ATPase endogène ou hétérologue.

4. Cellule hôte selon la revendication 1, 2 ou 3, où la cellule hôte comprend en outre une molécule d'acide nucléique codant une protéine ERp57 et une molécule d'acide nucléique codant une protéine calréticuline.

5. Cellule hôte selon la revendication 1, où la cellule hôte comprend en outre une molécule d'acide nucléique codant une protéine ERO1α humaine.

6. Cellule hôte selon l'une quelconque des revendications précédentes, dans laquelle la protéine recombinante est sélectionnée parmi le groupe consistant en enzymes, cytokines, facteurs de croissance, hormones, vaccins et anticorps.

7. Cellule hôte selon l'une quelconque des revendications précédentes, où la cellule hôte a été génétiquement modifiée pour exprimer des glycoprotéines sur lesquelles les *N-*glycanes prédominants sont sélectionnés parmi Man₈GlcNAc₂, Man₇GlcNAc₂, Man₆GlcNAc₂, Man₅GlcNAc₂, GlcNAcMan₅GlcNAc₂, GalGlcNAcMan₅GlcNAc₂, NANAGalGlcNAcMan₅GlcNAc₂, Man₃GlcNAc₂, GlcNAc₍₁₋₄₎Man₃GlcNAC₂, Gal₍₁₋₄)GlcNAc₍₁₋₄₎Man₃GlcNAc₂, NANA₍₁₋₄₎Gal₍₁₋₄₎GlcNAc₍₁₋₄₎Man₃GlcNAc₂ et des mélanges de ceux-ci.

8. Procédé de production d'une protéine recombinante humaine, comprenant :
(a) fournir une cellule hôte de *Pichia pastoris* comprenant une délétion ou une perturbation d'un gène endogène codant une protéine disulfure isomérase (PDI) et une molécule d'acide nucléique codant une PDI humaine intégrée dans le génome de la cellule hôte où la PDI humaine est surexprimée;
(b) introduire une molécule d'acide nucléique dans la cellule hôte codant la protéine recombinante humaine; et
(c) cultiver la cellule hôte dans des conditions qui conviennent à la production de la protéine recombinante humaine.

9. Procédé de production d'une protéine recombinante humaine ayant une *O-*glycosylation réduite, comprenant:
(a) fournir une cellule hôte de *Pichia pastoris* comprenant une délétion ou une perturbation d'un gène endogène codant une protéine disulfure isomérase (PDI) et une molécule d'acide nucléique codant une PDI humaine intégrée dans le génome de la cellule hôte où la PDI humaine est surexprimée;
(b) introduire une molécule d'acide nucléique dans la cellule hôte codant la protéine recombinante humaine; et
(c) cultiver la cellule hôte dans des conditions qui conviennent à la production de la protéine recombinante humaine.

10. Procédé selon la revendication 8 ou 9, dans lequel la fonction d'au moins un gène endogène codant une protéine, protéine *O*-mannosyltransférase (PMT), a été réduite, perturbée ou supprimée.

11. Procédé selon la revendication 8, 9 ou 10, dans lequel la cellule hôte comprend en outre une molécule d'acide nucléique codant une Ca²⁺ATPase endogène ou hétérologue.

12. Procédé selon la revendication 8, 9, 10 ou 11, dans lequel la cellule hôte comprend en outre une molécule d'acide nucléique codant une protéine ERp57 et une molécule d'acide nucléique codant une protéine calréticuline.

13. Procédé selon la revendication 8, dans lequel la cellule hôte comprend en outre une molécule d'acide nucléique codant une protéine ERO1α humaine.

14. Procédé selon l'une quelconque des revendications 8 à 12, dans lequel la protéine recombinante est sélectionnée parmi le groupe consistant en enzymes, cytokines, facteurs de croissance, hormones, vaccins et anticorps.

15. Procédé selon l'une quelconque des revendications 8 à 12, dans lequel la cellule hôte a été génétiquement modifiée pour exprimer des glycoprotéines sur lesquelles les *N-*glycanes prédominants sont sélectionnés parmi Man₈GlcNAc₂, Man₇GlcNAc₂, Man₆GlcNAc₂, Man₅GlcNAc₂, GlcNAcMan₅GlcNAc₂, GalGlcNAcMan₅GlcNAc₂, NANAGalGlcNAcMan₅GlcNAc₂, Man₃GlcNAc₂, GlcNAc₍₁₋₄₎Man₃GlcNAc₂, Gal₍₁₋₄₎GlcNAc₍₁₋₄₎Man₃GlcNAc₂, NANA₍₁₋₄₎Gal₍₁₋₄₎GlcNAc₍₁₋₄₎Man₃GlcNAc₂ et des mélanges de ceux-ci.
